# EUROPEAN PATENT APPLICATION

(11) **EP 4 711 380 A1**
(43) Date of publication of application: **18.03.2026**
(21) Application number: 24382990.0
(22) Date of filing: 16.09.2024
(51) Int. Cl.: C07K 14/47, A61P 35/00

(54) **LIGAND-DEPENDENT COREPRESSOR, (LCOR), MUTANTS AND FRAGMENTS THEREOF, AND USES FOR CANCER THERAPY**

(71) Applicant: Fundació Institut Hospital del Mar d'Investigacions Mèdiques, 08003 Barcelona (ES)
(72) Inventor: CELIÀ TERRASA, Antoni, 08003 Barcelona (ES); SERRA MIR, Gabriel, 08003 Barcelona (ES); PALOMEQUE ALARCON, José Ángel, 08003 Barcelona (ES); BLASCO BENITO, Sandra, 08003 Barcelona (ES)
(74) Representative: Hoffmann Eitle

(57) **Abstract**

The present invention provides a novel and improved cancer therapy based on an isolated or synthesized polynucleotide sequence coding for a mutant of a Ligand-dependent corepressor (LCOR), or a fragment thereof, capable of inducing expression of the antigen processing/presentation machinery (APM) genes, wherein the mutant or fragment of a Ligand-dependent corepressor (LCOR) is characterized by comprising a functional HTH domain, and wherein said mutant or fragment of a Ligand-dependent corepressor (LCOR) is further characterized by comprising an absent or inactivated Nuclear Receptor Binding Domain.

## Description

### TECHNICAL FIELD OF THE INVENTION

The present invention refers to the immunotherapy field, more particularly to cancer immunotherapy or therapies using the action of the immune system to eliminate cancer cells.

### BACKGROUND OF THE INVENTION

Immune-checkpoint blockade (ICB) immunotherapy unleashes antitumor immune responses and has emerged as one of the most effective therapies in oncology. Nevertheless, most patients do not respond or acquire resistance to ICB, and this strategy is not active against all cancer types. The underlying tumor-intrinsic mechanisms of ICB resistance are thus still under intense investigation. Among these, the interferon sensitivity within the tumor phenotypic heterogeneity might help to explain how patients with similar tumor types still show varying sensitivity to ICB. Other immunotherapies, such as adoptive cell transfer (ACT) has demonstrated impressive results in oncology and shows great promise for the future of immune-oncology, including for solid tumors. ACTs includes tumor-infiltrating lymphocyte (TIL) therapies, engineered T cells and NK cells, and chimeric antigen receptor T-cell (CAR T-cell) therapies.

Interferon signaling plays a central role in tumor immunosurveillance and immunotherapy response, in part by modulation of antigen presentation machinery (APM) in tumor cells. APM includes major histocompatibility complex (MHC) class I genes (HLAs), aiding molecules (β2M), transporters (TAP complex and tapasin) and immunoproteasome genes (PSMBs) among others, which are the cellular effectors of antigen presentation allowing its recognition by the immune system. Multiple studies and clinical reports have identified genetic alterations in antigen presentation components and the IFN pathway as major mechanisms of ICB resistance. Epigenetic factors and transactivators, such as EZH2, NF-κB and NLRCS, can also modulate MHC-I and other APM genes affecting tumor immunogenicity. Therefore, a better understanding of APM regulation in different tumor cell populations and phenotypes can help to elucidate the mechanisms underlying immunotherapy resistance.

Stem cell phenotypes, such as embryonic and slow-cycling adult stem cells, downregulate MHCs, favoring immune surveillance evasion. Cancer Stem Cells (CSCs) resemble their normal counterparts in many aspects and may co-opt similar immune-evasive phenotypes relevant in ICB resistance. CSCs actively interplay with immune-secreted cytokines, including IFNs, and some reports have shown low expression of MHCs and Transporter 1 (TAP1) in CSCs, suggesting a role in immune surveillance evasion. CSCs also express CD274 (PD-L1), a mechanism that contributes to immunosuppression but not to anti-PD-L1 ICB escape. Epithelial-to-mesenchymal transition, often linked to stemness, also promotes immunosuppressive features. In the context of immunotherapy resistance, skin squamous cell carcinoma (SCC) tumor-initiating cells (TICs) have been linked to adoptive cell transfer resistance by upregulation of CD80; and head and neck SCC CSCs have ICB resistance by downregulating the secretion of T cell-recruiting chemokines. However, the connection between stemness and APM pathway dysregulation leading to ICB escape, and the molecular mechanisms controlling it, was not established.

In the mammary gland, normal mammary stem cells (MaSCs) and CSCs are usually governed by differentiating cell fate determinants and stem cell transcription factors. LCOR is a differentiation factor sensitizing these to IFN, which drives intrinsic tumor cell differentiation and reduced tumor growth. However, how LCOR intersects the IFN response has never been explored and may be critical to understanding cellular immunity. Therefore, we were interested in exploring their mechanistic connections with immunity and ICB resistance in Triple Negative Breast Cancer (TNBC). This is clinically relevant, because there is an urgent need to improve the efficacy of ICB in breast cancer, approved in TNBC with only limited clinical benefit to date. Here, we show that breast LCORlow CSCs shut down both antigen processing and presentation, contributing to immune-checkpoint therapy resistance in TNBC. LCOR activates transcription of the APM independently of IFN, rendering CSCs visible and vulnerable to immune attack during ICB. Our results demonstrate the relevance of phenotypic heterogeneity and LCOR biology in APM regulation as an excellent therapeutic partner of ICB therapy.

### BRIEF DESCRIPTION OF THE FIGURES

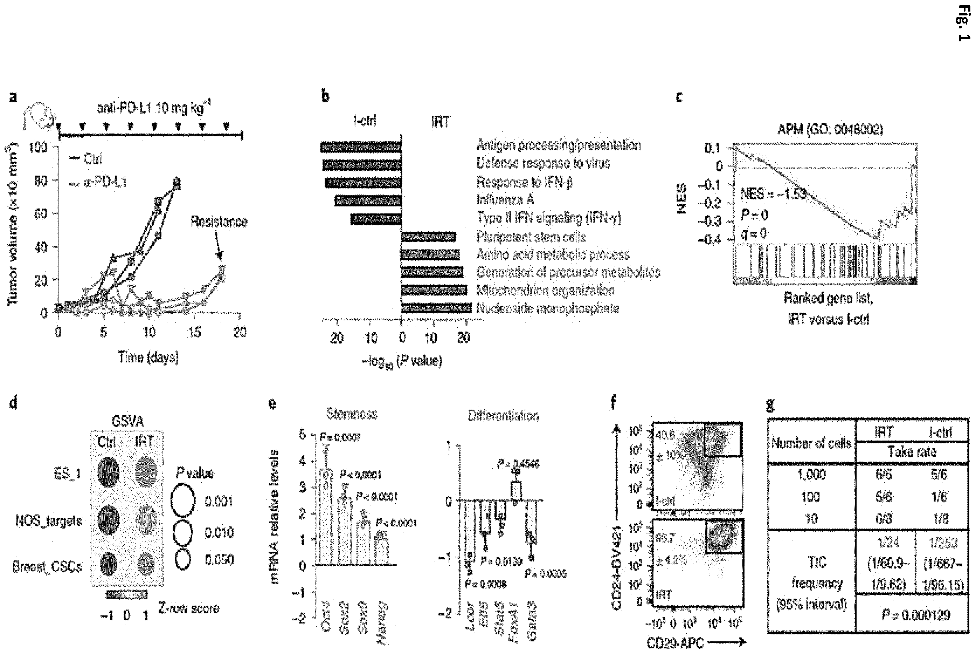

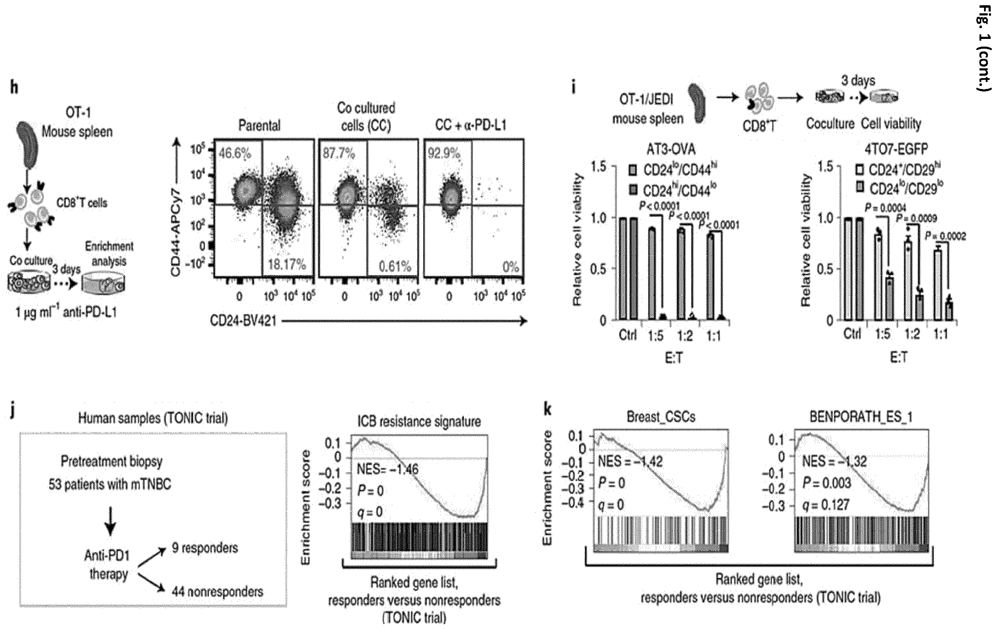

**FIG.1****.** a, Growth curves for 4TO7 tumors in Balb/c mice treated with anti-PD-L1 ICB. Dose and regime are indicated. n = 3 tumors per condition. b, GO analysis of upregulated (red) and downregulated (blue) pathways by RNA-seq in 4TO7 anti-PD-L1 IRT and control cells (I-Ctrl). P value computed by Fisher exact test. c, GSEA of APM (GO: 0048002) in IRT versus control cells. Family-wise error rate (FWER) P value and FDR q value are provided. d, GSVA of previously described stemness signatures: embryonic stem cells (ES_1), NANOG-OCT4-SOX2 targets (NOS_Targets) and breast_CSCs (LIU breast ER- CSCs). Circle size represents significance and colors the mean z-row score. b-d, Two independent biological replicates. e, RT-qPCR of stem cell and differentiation genes in IRT versus I-Ctrl tumor cells. Log2 fold change of IRT versus control cells. Data represent mean ± s.e.m. f, Flow cytometry analysis of CD24hi/CD29hi population in IRT and control cells. Data represent mean ± s.e.m. e,f, n = 3 individual biological replicates. g, MFP injection and LDA of IRT 4TO7 cells in NSG mice. Table represents serial dilution injections with the take rate; n indicates the number of MFP injections. TIC frequency calculated by ELDA software is shown in red. P value obtained by Pearson's chi-squared two-tailed test. h, CTL assay representation and CD24lo/CD44hi CSC flow cytometry analysis. Data represent percentage of viable cells in a representative experiment. Anti-PD-L1 treatment at 1 µg ml-1. i, OT-I/JEDI CTL assay with CSCs and non-CSCs isolated from AT3-OVA and 4TO7-EGFP cells. E:T denotes effector to target ratio. h,i, Data represent mean ± s.e.m. n = 3 independent biological replicates. j,k, GSEA of transcriptomic ranked list for responder versus nonresponder patients from the TONIC trial40, with our IRS (j) and ES_1 (k) and LIU breast ER- CSC signatures. e,i, Exact P value by two-tailed Student's t-test; d, Wilcoxon signed-rank test. f,h,i, Cells were gated from P3.

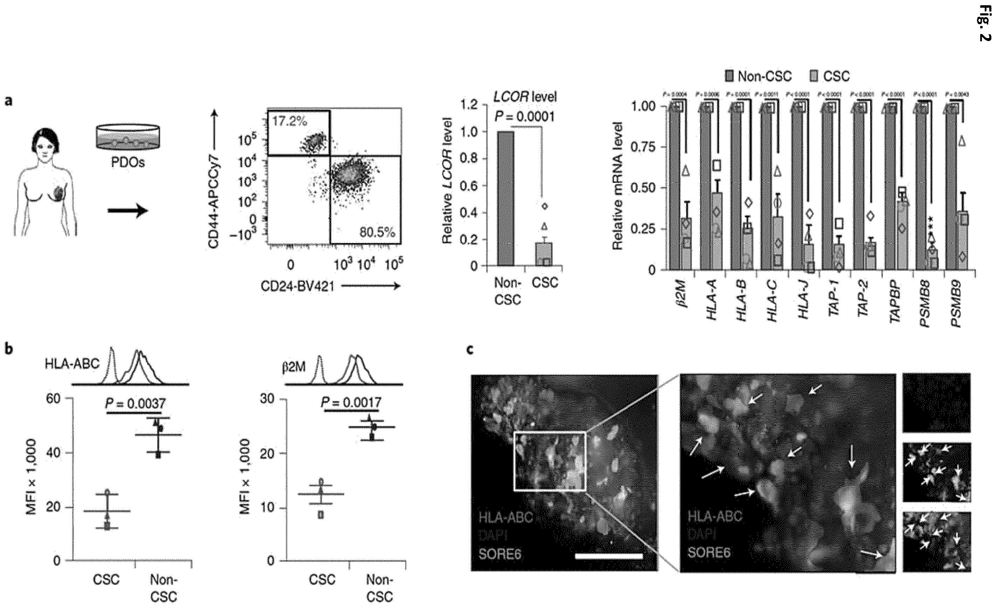

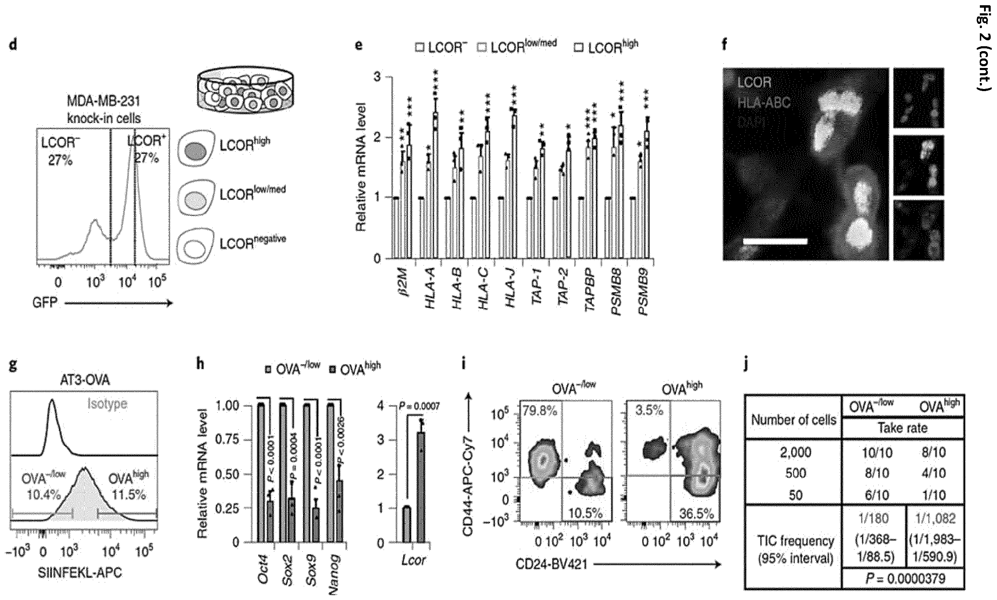

**FIG.2** a, PDOs from four patients with TNBC and isolation of CD24lo/CD44hi (CSC) and CD24hi/CD44lo (non-CSC). Right: RT-qPCR analysis of LCOR and APM genes. n = 4 independent biological replicates; data represent mean ± s.e.m. b, Flow cytometry analysis of pan-HLAs and β2M in CSC and non-CSC cells from PDOs. n = 3 independent biological replicates; data represent relative mean fluorescent intensity (MFI; versus isotype control) ± s.e.m. c, Representative LSFM imaging of a PDO with the CSC SORE6-GFP reporter (green), pan-HLA-ABC (purple) and DAPI (blue). Panels on the right show single channels. n = 3 independent organoids. d, LCOR-GFP knock-in flow cytometry analysis in MDA-MB-231 cells. e, RT-qPCR analysis for APM genes of LCOR knock-in MDA-MB-231 cells isolated by LCOR-, LCORIow/med and LCORhigh. d,e, n = 3 individual biological replicates; data represent mean ± s.e.m. f, IF of pan-HLA-ABC in LCOR knock-in MDA-MB-231 cells: LCOR-GFP (green), HLA-ABC (purple) and DAPI (blue). n = 3 independent biological replicates; image of one representative experiment. g,h, Flow cytometry isolation (g) and RT-qPCR analysis (h) of OVA-/low and OVAhigh (SIINFEKL OVA peptide) from AT3 cells with ectopic OVA overexpression (AT3-OVA). g,h, n = 3 individual biological replicates for; data represent mean ± s.e.m. i, Flow cytometry analysis of OVA-/low and OVAhigh cell distribution within CD24lo/CD44hi CSC markers of AT3 cells. j, Orthotopic MFP injection and LDA of OVA-/low and OVAhigh AT3-OVA cells in immunodeficient NSG mice. Table represents serial dilution injections with the corresponding take rate, and n indicates the number of MFP injections for each dilution. TIC frequency calculated by ELDA software is shown in red. P value was obtained by Pearson's chi-squared two-tailed test. c,f, Scale bars, 100 and 40 µm, respectively. a,b,h, Exact P values determined by two-tailed Student's t-test; e, *P < 0.05, **P < 0.01, ***P < 0.005 by one-way ANOVA with Bonferroni post hoc test. a,b,e,g, Cells were gated from P3.

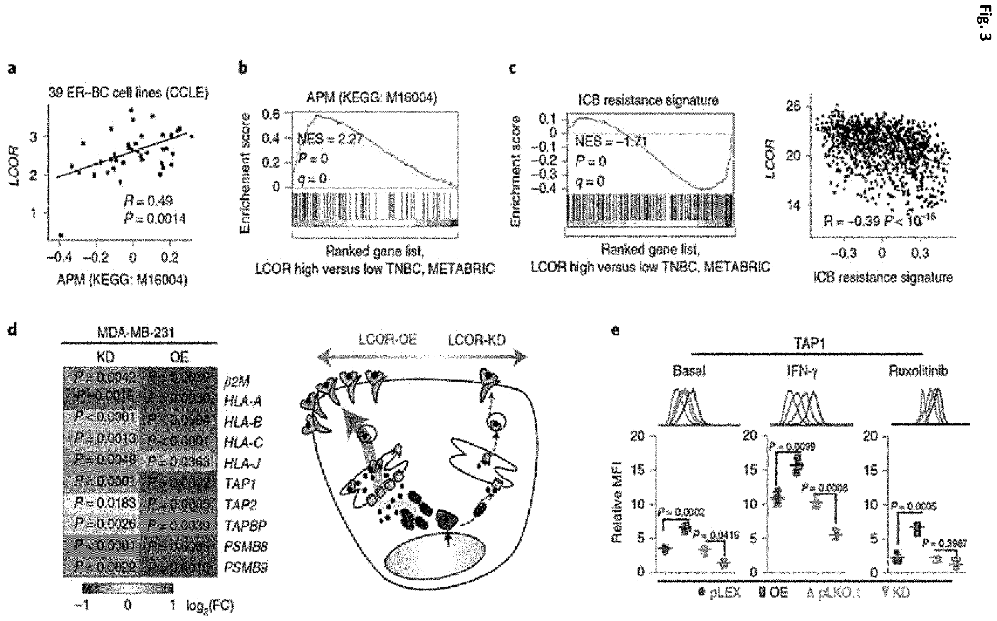

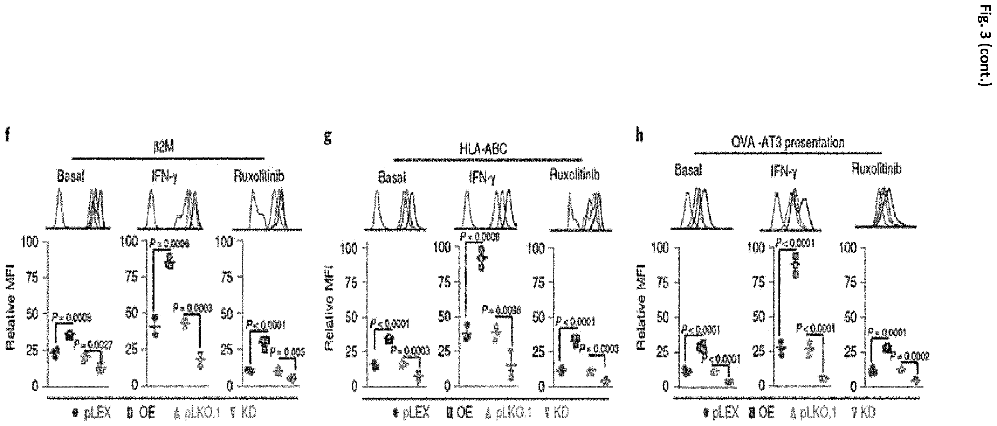

**FIG.3** a, Correlation of LCOR mRNA with APM (KEGG: M16004) signature in 39 ER-negative breast cancer cell lines from CCLE. P value calculated by Rho correlation. b, GSEA of the METABRIC dataset TNBC stratified as LCOR-high versus -low patients based on the median and interrogated with the APM (KEGG: M16004) signature. FWER P value and FDR q value are provided. c, Left: GSEA of our ICB resistance signature in the TNBC METABRIC dataset stratified by LCOR median. Right: correlation of GSVA score of ICB resistance signature with LCOR in the METABRIC TNBC dataset. Blue line represents lineal regression analysis by Rho correlation and P value. d, Heat map of RT-qPCR analysis of APM genes in LCOR-OE and LCOR-KD transduced MDA-MB-231 cell lines relative to their respective controls. n = 3 individual biological replicates; data represent the mean. e-g, Flow cytometry analysis of TAP1 (e), β2M (f) and pan-HLA (g) levels under basal conditions, IFN-y treatment treatment at 10 ng ml-1 and ruxolitinib (1 µM) in MDA-MB-231 LCOR-OE and LCOR-KD cells with respect to their controls and isotype negative control. n = 3 independent biological replicates; data represent MFI relative to the isotype ± s.e.m. h, Flow cytometry analysis of SIINFEKL OVA peptide presented by H2-K1b in AT3-OVA cells at the indicated conditions (IFN-y treatment at 10 ng ml-1 and ruxolitinib at 1 µM). n = 3 independent biological replicates; data represent MFI relative to the isotype ± s.e.m. d-h, Exact P values calculated by two-tailed Student's t-test. e-h, Cells were gated from P3.

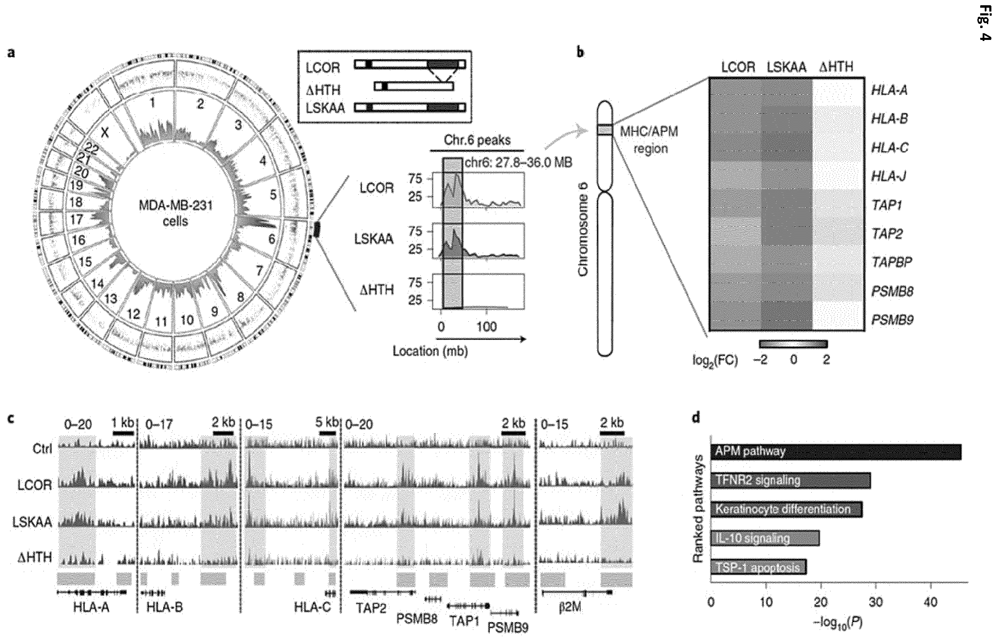

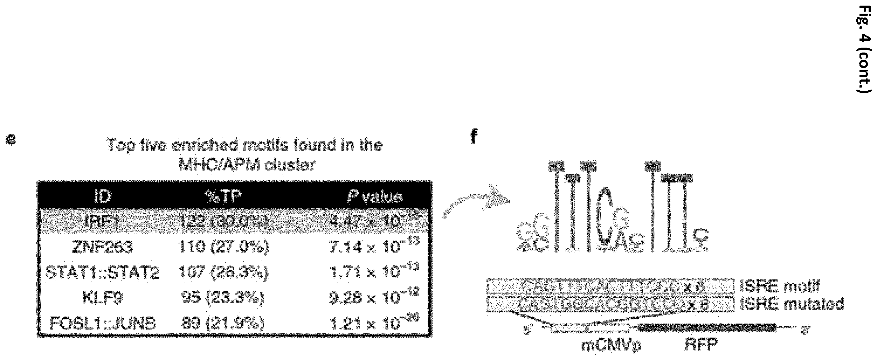

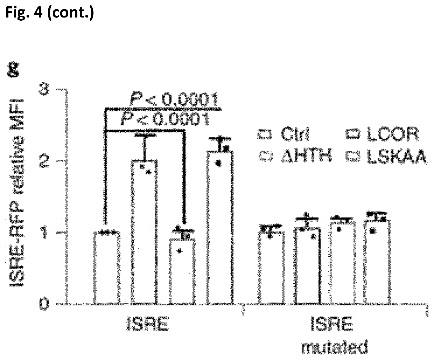

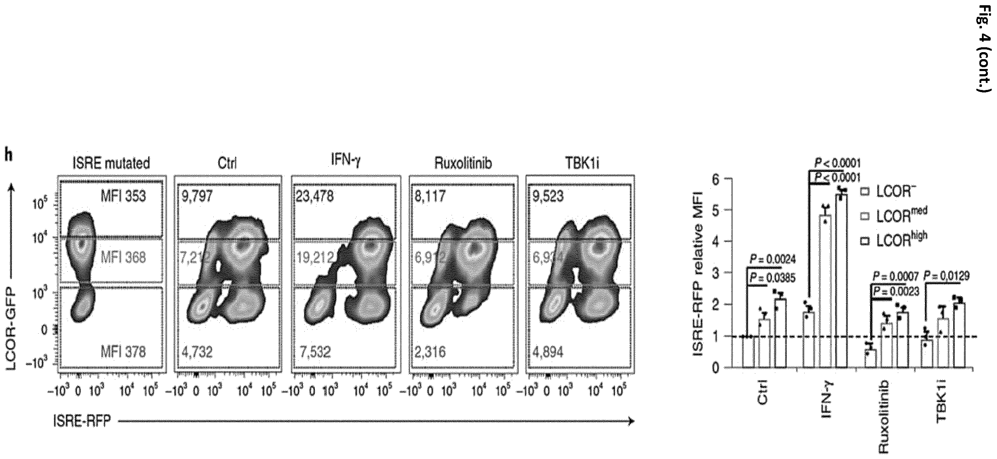

**FIG.4** a, ChlP-seq analysis of pLEX-HA, pLEX-LCOR-HA, pLEX-LSKAA-HA and pLEX-ΔHTH-HA (helix-turn-helix) in MDA-MB-231 cells. LSKAA is a LCOR mutant with two amino acid change in the nuclear receptor domain disrupting this domain for a proper interaction with nuclear receptors. Genome-wide distribution of LCOR-HA peaks and in chr6 of indicated conditions. Two independent biological replicates. b, Heat map RT-qPCR analysis of APM genes located in the MHC cluster of chr6 in MDA-MB-231 cells, represented by log2 fold change (log2(FC). n = 3 individual biological replicates; data represent the mean. c, ChlP-seq LCOR peak occupancy in APM genes in this specific region of chr6 and β2M on chr15. Blue horizontal shading shows either GREs defined by H3K27ac or H3K4me3 defined by UCSC (https://genome.ucsc.edu/). Occupancy and scale are indicated. Data show a replicate from a. d, Pathway analysis of genes with LCOR ChlP-seq peaks using BioCarta-2016. Data represent -log10(P); P value computed from Fisher's exact test. e, ChlP-seq HOMER analysis and top five enriched binding motifs of LCOR peaks in the MHC cluster region. P value of enrichment and percentage of motif sequence labeled and classified as positive among peaks (%TP). P value computed from Fisher's exact test. f, Scheme of the interferon-stimulated regulatory element (ISRE) red fluorescent protein (RFP)-reporter containing six tandem ISRE motifs and minimal CMV promoter (mCMVp). g, Flow cytometry analysis normalized to control condition using ISRE and !SRE-mutated reporters at the indicated conditions in MDA-MB-231 cells. n = 3 individual biological replicates; data represent MFI relative to isotype control mean ± s.e.m. h, Flow cytometry analysis of ISRE reporter in MDA-MB-231 LCOR-GFP knock-in under control (ctrl) conditions and after 24 h treatment with IFN-y (10 ng ml-1), ruxolitinib (0.5 µM) or BX-795 (TBK1i, 0.5 nM). Left: density plots and MFI of mCherry in LCOR-, LCORmed/low and LCORhigh. Right: MFI quantification. n = 3 individual biological replicates; data represent relative MFI mean ± s.e.m. g,h, Exact P values calculated by one-way ANOVA and Bonferroni post hoc analysis; cells were gated from P3.

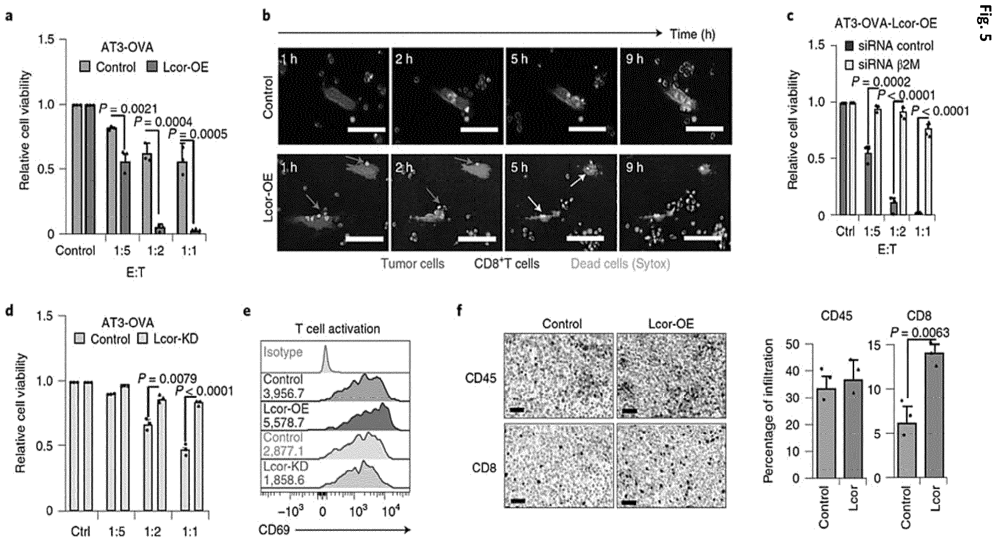

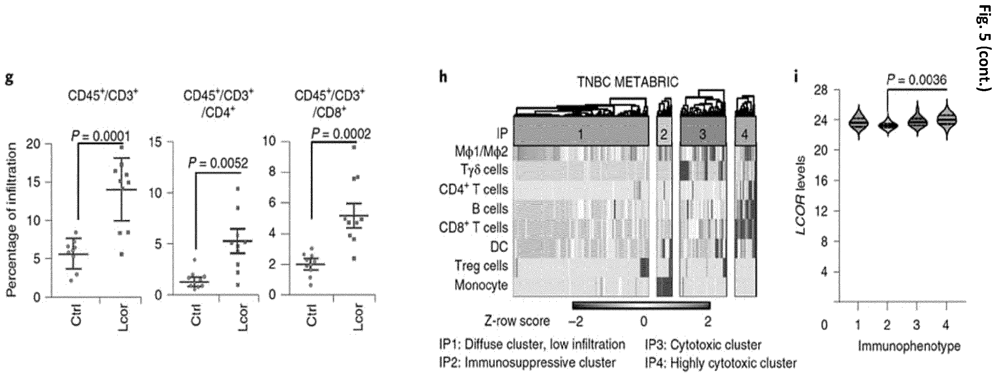

**FIG.5** a-e, Cytotoxic Lymphocyte (CTL) assays of AT3-OVA cells after 72 h of coculture with OT-1 CD8+ T cells at the indicated conditions, and effector (E) and tumor (T) cell ratios. a, Lcor-OE versus control vector. b, Time-lapse confocal imaging of a, ratio 1:1. Tumor cells are labeled red, CD8+ T cells blue and cell dead tracker (SYTOX) green (white shading when mixed with tumor cells). Blue arrows indicate tumor-immune interactions, and white arrows killed tumor cells. c, Lcor-OE siRNA β2m versus Lcor-OE siRNA control. d, Lcor-KD versus control vector. e, Flow cytometry analysis of CD69 expression in CD8+ T cells after CTL. a-e, n = 3 independent biological replicates; a,c,d, data represent mean ± s.e.m.; e, relative MFI of a representative replicate. f, IHC and quantification analysis of percentage of tumor-infiltrating immune cells (CD45+) and CD8+ T cells (CD8+) in control versus Lcor-OE 4TO7 tumors. n = 3 individual biological replicates; data represent mean ± s.e.m. g, Flow cytometry analysis of percentage of tumor-infiltrating lymphoid cells (CD45+CD3+), CD8+ and CD4+ T cells in control or Lcor-OE 4TO7 tumors. n = 10 individual biological replicates; data represent mean ± s.e.m. h,i, Computational immunophenotype clustering by deconvolution of TNBC METABRIC dataset using xCell50. h, Heat map representing clusters of patients based on four IPs as indicated. i, LCOR levels in each immunophenotype. b,f, Scale bars, 20 µm. a,c,d,g,h, Exact P values by two-tailed Student's t-test; i, one-way ANOVA with Bonferroni post hoc analysis.

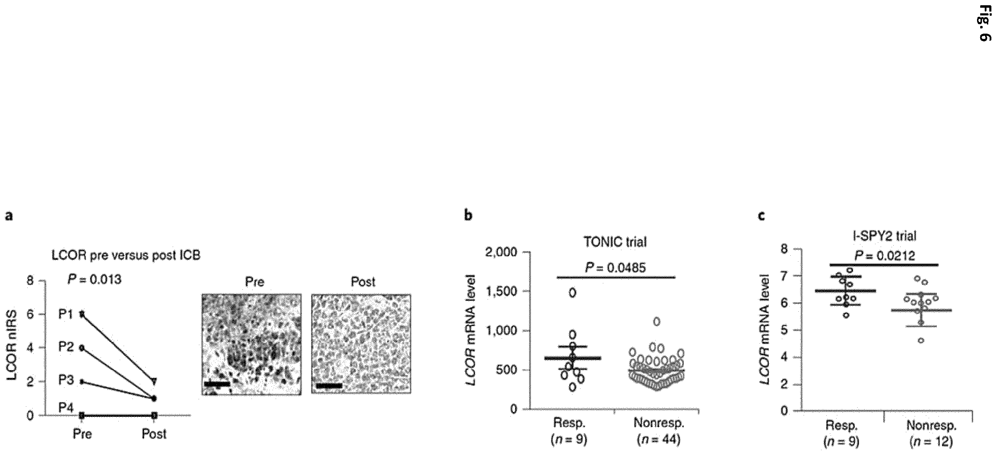

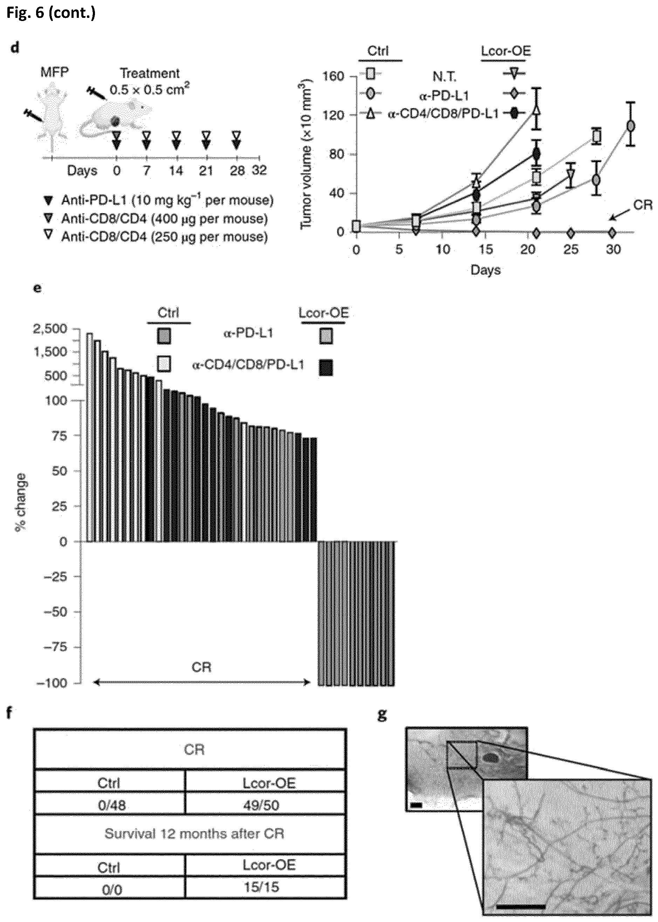

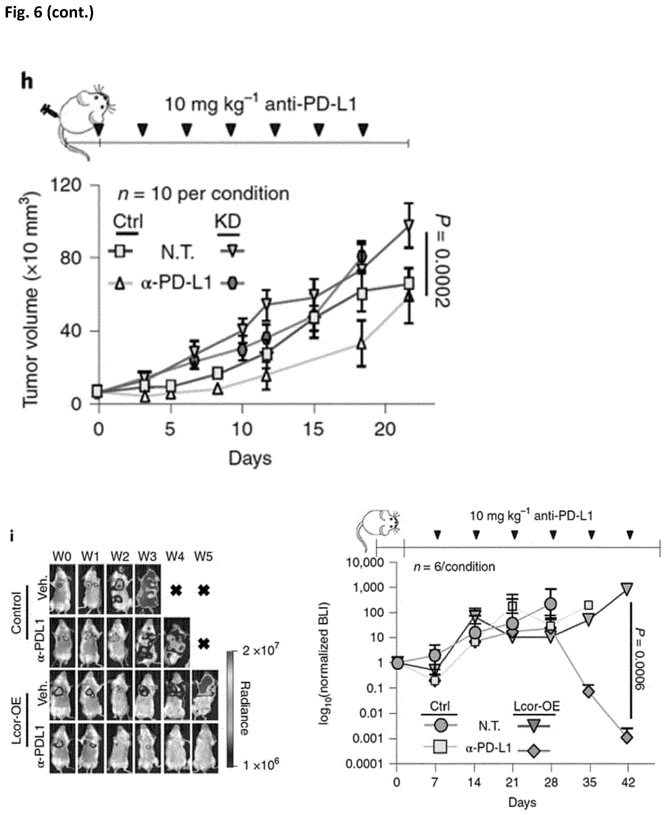

**FIG.6** a, Immunohystochemistry (IHC) analysis of LCOR in samples from patients pre and post treatment for TNBC: P1 and P2 were treated with anti-PD-L1 atezolizumab + abraxane and carboplatin while P3 and P4 were treated with anti-PD1 nivolumab + SYK/FTL3 inhibitor (TAK-659). Left: nIRS of LCOR in pre- versus post-treated samples. Right: representative images of pre- and post-treated samples with LCOR nIRS = 6 and nIRS = 2, respectively. P value by Wilcoxon signed-rank test. b,c, Analysis of LCOR mRNA levels in all preinduced responders (CR + PR) versus nonresponders (PD + SD) patients in the TONIC trial (b) and in the I-SPY2 trial (c), comparing responders (CR + PR) and patients with progression disease (SD + PD). n is indicated separately for each condition; data represent mean ± s.e.m. in both. d, Growth curves of orthotopic 4TO7 transplanted tumors and individualized treatment starting at the reach point of tumor size 0.5 × 0.5 cm2, with the indicated conditions. Treatments were administered intraperitoneally at the indicated dose regimes weekly. n = 10 mammary glands for each condition; data represent mean ± s.e.m. e, Waterfall plot representing change of percentage in tumor volume from the first day of treatment to the respective endpoint. n = 10 mammary glands for each condition, f, Take rate of complete response (CR) to treatment and tumor-free mouse survival 12 months after treatment withdrawal. Compilation of five independent experiments (n = 10 per experiment) treated with anti-PD-L1 (10 mg kg-1) every 3-7 days. g, Carmine staining of a representative cured mammary gland. n = 3 cured mammary glands. h, Growth curve of orthotopic 4TO7 control versus Lcor-KD tumors at the indicated dose regime every 3 days. n = 10 mammary gland tumors; data represent mean ± s.e.m. i, Preclinical lung metastasis assay by TV injection of 4TO7 cells. Once metastases were established, anti-PD-L1 treatment was commenced at the indicated dose regimen. n = 6 mice per condition; data represent mean ± s.e.m. in log10 scale. Veh., vehicle. b,c, Exact P values by one-tailed Student's t-test; h,i, one-way ANOVA. N.T., nontreated d,h,i.

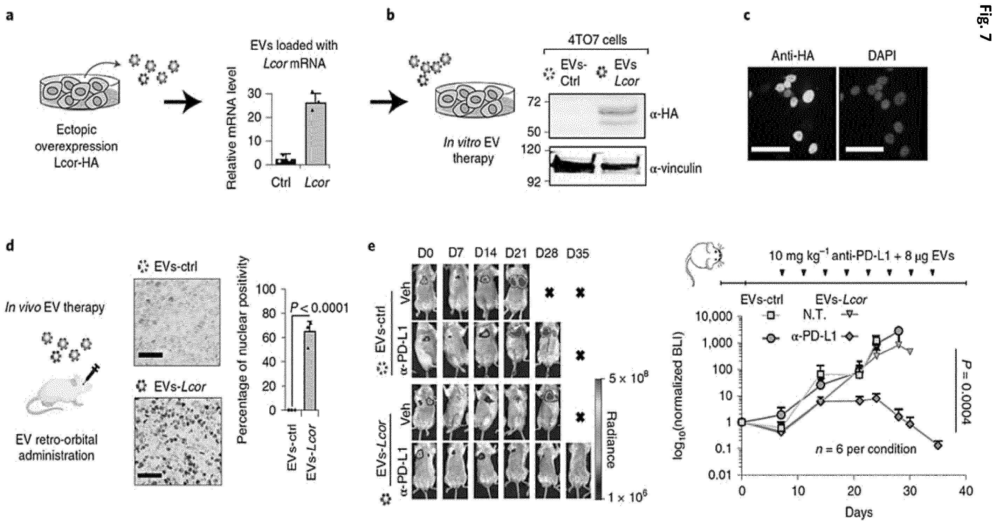

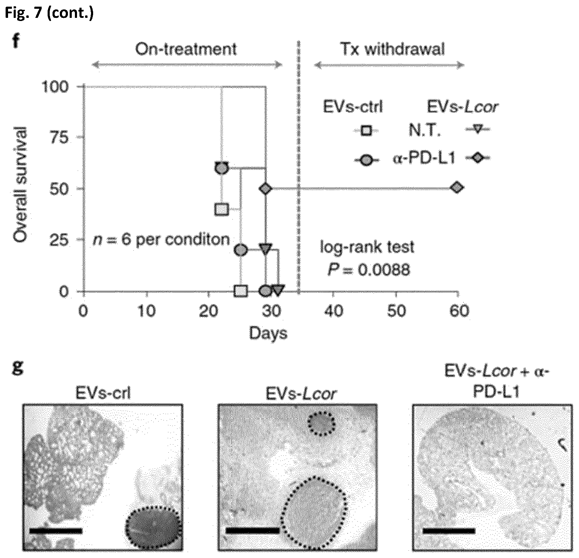

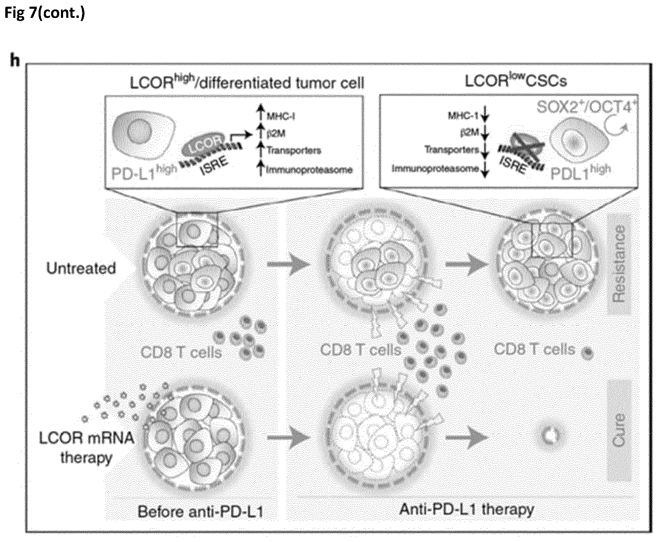

**FIG.7** a, Lcor mRNA levels in EVs from HEK293T cells with ectopic overexpression of Lcor-HA (pLEX-Lcor-HA) versus control (pLEX-HA). Data represent mean ± s.e.m. of three technical replicates. Representative experiment of five independent experiments listed in Source data. b, In vitro treatment with control and Lcor-HA-EVs over 3 days and anti-HA immunoblot in 4TO7 cells. Representative immunoblot of two biological replicates. c, Lcor-HA protein expression and localization in recipient cells. Representative image of three independent biological replicates. d, EV delivery, uptake and protein translation in vivo: retro-orbital administration of EVs and metastatic tumor tissue IHC with anti-HA and quantification of nuclear positivity. n = 3 independent biological replicates; data represent mean ± s.e.m. e, Preclinical lung metastasis assay by TV injection of 50,000 4TO7 cells. After 8 days, metastases were established; EV + anti-PD-L1 treatment was started at the indicated dose regime and repeated every 3 days. n = 6 mice per condition; data represent mean ± s.e.m. in log10 scale. Representative BLI images (left) and growth curves (right) of the indicated conditions. f, Mouse survival Kaplan-Meier plot of the lung metastasis assay in e. Tx, treatment. P value calculated by log-rank test. g, Representative images of established lung metastases treated with control EVs + anti-PD-L1 (left), Lcor EVs (middle) and Lcor EVs + anti-PD-L1 (right). h, Schematic model of the main thrust of this study: (1) LCORlow CSCs led to immunoediting escape and resistance to ICB therapy in TNBC; (2) LCOR increased the expression of APM genes through ISREs, independently of IFN; (3) LCOR-mediated immunogenicity determined response to ICB, CSC elimination and overcoming resistance in TNBC; and (4) LCOR mRNA therapy led to increased APM in CSC and avoided their escape to ICB, resulting in complete pathological response and cure. c,d, Scale bars, 60 µm; g, 300 µm. d, Exact P values by two-tailed Student's t-test; e, one-way ANOVA; f, logrank test.

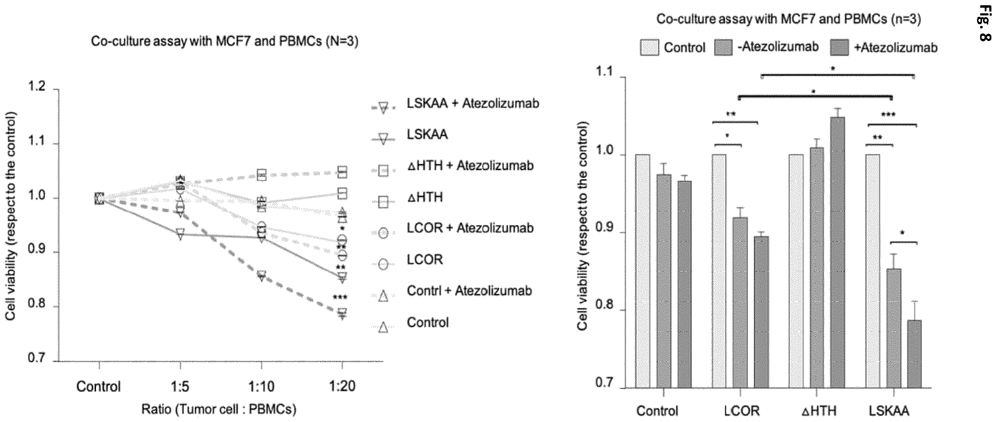

**FIG.8** Cytotoxic T Lymphocyte (CTL) coculture assay of human MCF7, estrogen-positive breast cancer cells, with human peripheral blood mononuclear cells (PBMCs) during 5 days with/without anti-PD-L1 (Atezolizumab). n = 3 individual biological replicates; data represents mean ± SEM. MCF7 cells were transduced to ectopically overexpress the indicated forms of LCOR, including: LCOR (LCOR wild-type); LSKAA (LCOR with two amino acid change in the nuclear receptor domain); and delta-HTH (ΔHTH; LCOR without HTH domain).

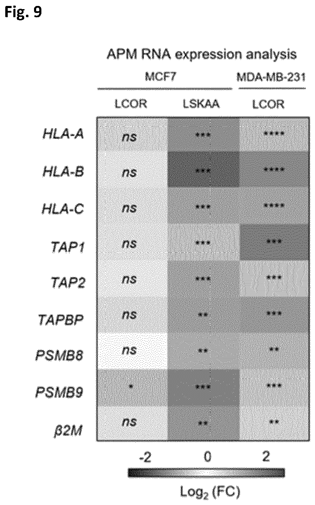

**FIG.9** Heat map RT-qPCR analysis of APM genes located in the MHC cluster of chr6 in MCF7 (ER+ breast cancer cells), and MDA-MB-231 (ER-negative breast cancer cells) represented by log2 fold change log2(FC). n = 3 individual biological replicates; data represent the mean.

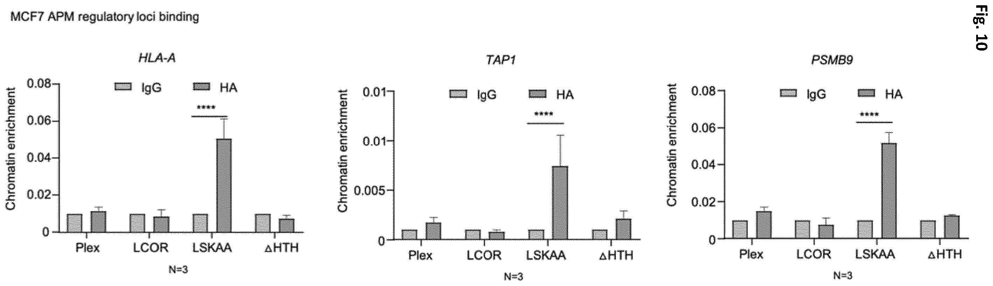

**FIG.10** Anti-HA ChIP-qPCR analysis in MCF7 cells with ectopic expression of LCOR forms fused with HA. Conditions are LCOR (LCOR wild-type); LSKAA (LCOR with two amino acid change in the nuclear receptor domain); and delta-HTH (ΔHTH; LCOR without HTH domain). n = 3 independent biological replicates; data represents mean ± SEM

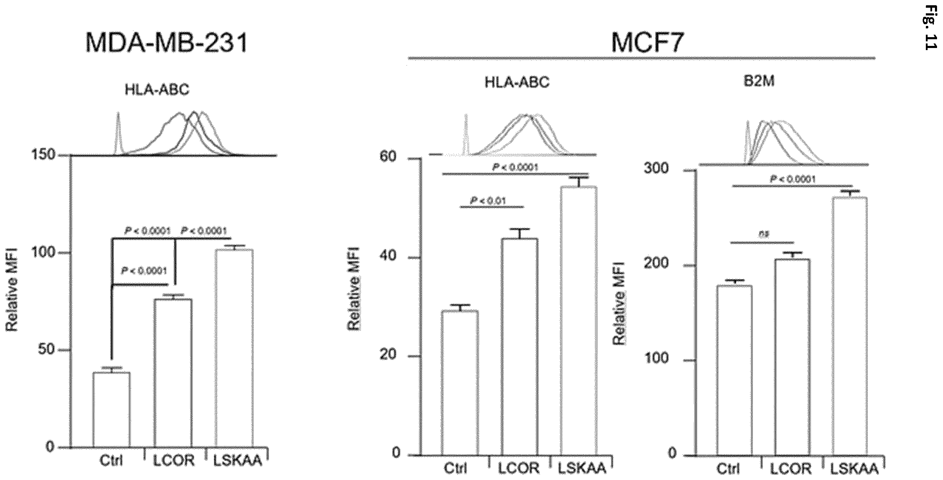

**FIG.11** Flow cytometry analysis of anti-Pan-HLA-ABC in MCF7 cells with ectopic expression of LCOR and LSKAA. n = 3 independent biological replicates; data represents relative mean fluorescence intensity (MFI) ± SEM.

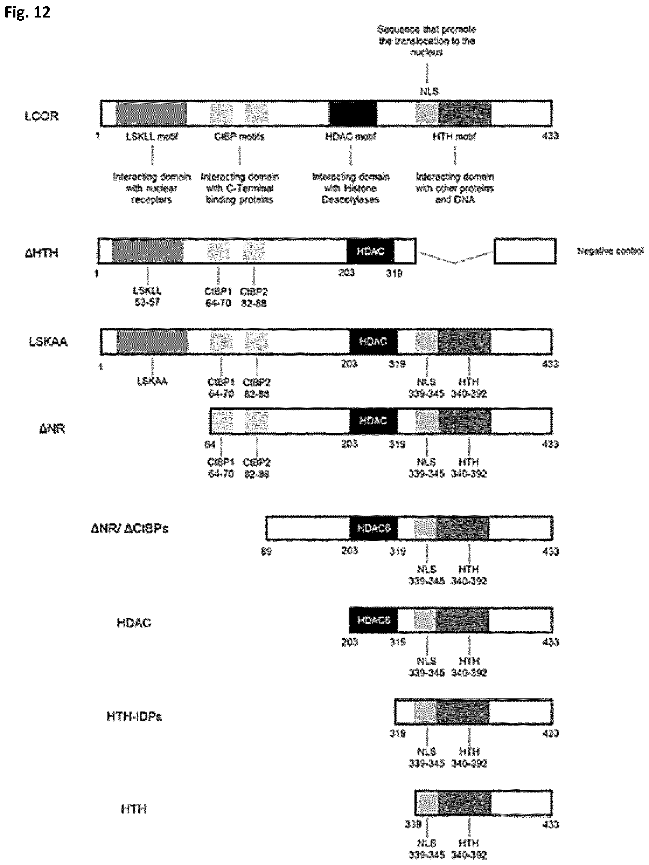

**FIG.12** Delineation of LCOR fragments. Schematic representation of LCOR protein structure and domains, including the different LCOR fragments generated with progressive deletion of protein domains as indicated. Delta symbol denotes the deletion of the indicated domain. These fragments are designed for protein ectopic expression using pLEX-MCS plasmid backbone.

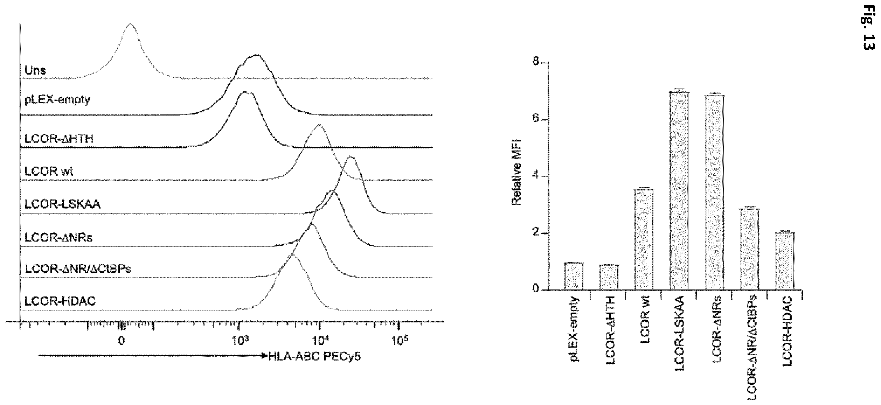

**FIG.13** Functional test of LCOR fragments in MHC-I regulation. (A) Flow cytometry analysis of MDA-MB-231 cells transduced to overexpress the indicated fragments compared to control conditions (pLEX; empty vector). Anti-pan-HLA-ABC-PECyS measures pan-HLA-ABC expression at the cell surface of these cells. (B) Bar plots of relative mean fluorescence intensitiy (MFI) from the flow cytometry analysis of the indicated LCOR fragments.

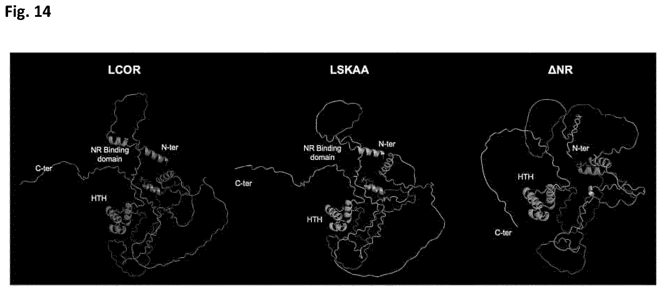

**FIG.14****.** Predicted structure of LCOR wt, LCOR-LSKAA, and LCOR-dNR.

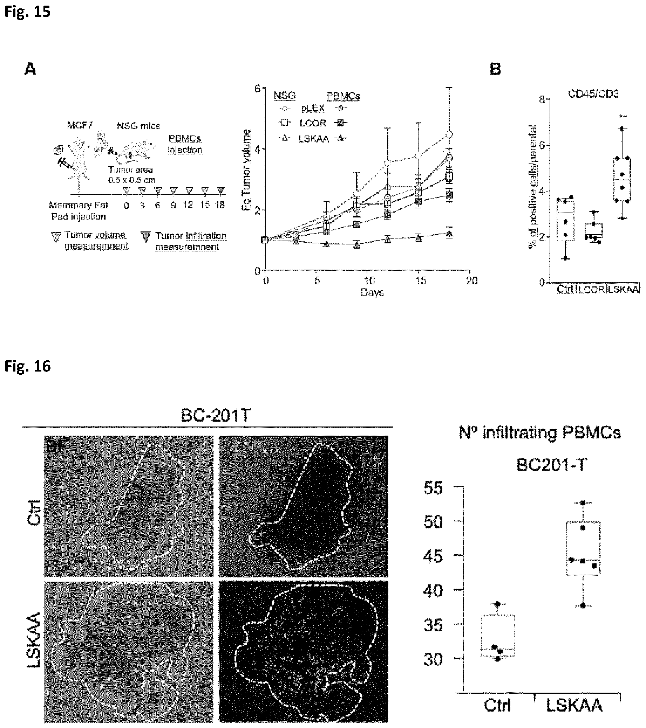

**FIG.15****.** A) ER+ breast MCF7 cancer cells implanted into immunodeficient mice (NSG) and cell immune humanization with human peripheral blood mononuclear cells (PBMCs). Plot shows tumor growth in vivo at the indicated conditions. B) Tumor infiltrating lymphocytes (TILs) at the indicated conditions.
**FIG.16****.** Coculture of human peripheral blood mononuclear cells (PBMCs) and tumoroids from an ER+BC tumor sample (patient BC-201T) transduced with LSKAA vs control.

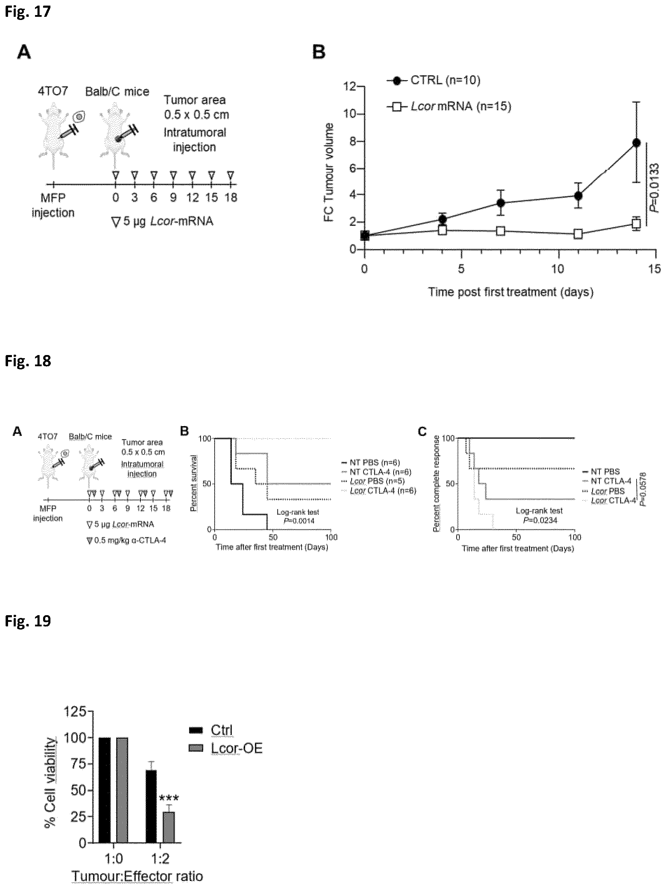

**FIG.17****.** Lcor-mRNA therapy in immunocompetent mice in vivo.
   A. Schematic representation of experimental procedure. Balb/c mice were implanted with 15,000 4TO7 cells in the mammary fat pad (MFP). Once tumors reached 0.5x0.5 cm, intratumoral injections of 5 µg of Lcor-mRNA were administered twice weekly. B. Tumor growth kinetics. Data represents mean±SEM. A total of 10 mice were included in the control group, while 15 mice were treated with Lcor-mRNA. Statical differences were determined using non-parametrical Mann-Whitney U-test.
**FIG.18****. LCOR-OE and anti-CTLA-4 blockade immunotherapy *in vivo.***
   A. Schematic representation of experimental procedure. Balb/c mice were implanted with 20,000 4TO7 cells in the mammary fat pad. Once tumors reached 0.5x0.5 cm, intratumoral injections of 5 µg of Lcor-mRNA were administered twice weekly. A 0.5 mg/kg dose of CTLA-4 immunotherapy were administered once a week. B. Kaplan-Meier survival curve. C. Complete response rates.
**FIG.19****. LCOR-OE and anti-PD1 therapy *in vitro.*** Cytotoxic Lymphocyte (CTL) assays of AT3-OVA cells after 72 h of coculture with OT-1 CD8+ T cells and anti-PD1 treatment at the indicated conditions, and effector (E) and tumor (T) cell ratios. Lcor-OE non-treated versus Lcor-OE treatetd with anti-PD1 (10 ng/ml; n=3). Statical differences were determined using non-parametrical Mann-Whitney U-test.

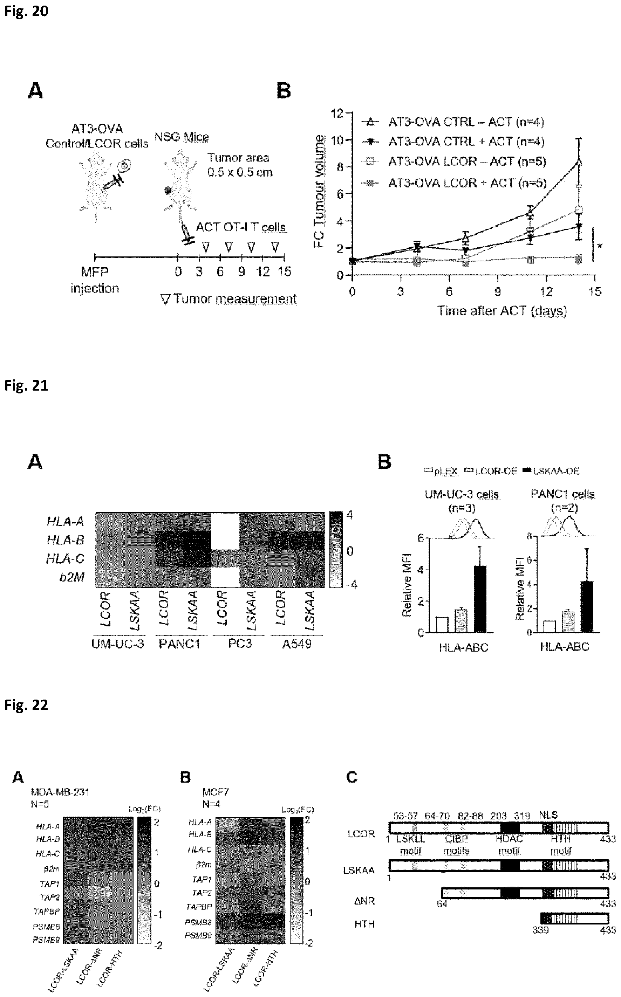

**FIG.20****. Adoptive cell transfer (ACT) therapy in combination with LCOR-OE in NSG mice.** A. Schematic representation of experimental procedure. NSG mice (immunodeficient) were implanted with 500,000 controls (empty vector) or 1,000,000 LCOR-OE AT3-OVA cells in the mammary fat pad. Once tumors reached 0.5x0.5 cm, intravenous injection of 5,000,000 CD8⁺ cells purified from OT-I mice was performed. OT-I mice were genetically engineered to express an OVA-specific TCR, enabling a highly specific recognition for OVA⁺ cells. B. Tumor growth kinetics. Data reflects mean±SEM. Statical differences were determined using non-parametrical Mann-Whitney U-test.
**FIG.21****. LCOR and LSKAA effects in different cancer cells with different NR status.** A. Differential expression levels of key components of the antigen presenting and processing machinery (APM) in overexpressing different fragments of the LCOR protein in bladder (UM-UC-3; ER-/PGR-/AR+; n=3), pancreas (PANC1; ER-/PGR-/AR-; n=2), prostate (PC3; ER+/PGR-/AR-; n=1), and lung (A549; ER-/PGR-/AR+; n=2) cell lines. Normalized to empty vector expression. B. Flow cytometry analysis of pan-HLA levels in UM-UC-3 and PANC1 LCOR-OE and LSKAA-OE cells with respect to their controls. Data represent mean±SEM.
**FIG.22****. LCOR mutants in MDA-MB-231 (HR-) and MCF7 (HR+) cell lines.** A. Differential mRNA expression levels determined by RT-qPCR of key components of the antigen presenting and processing machinery (APM) in MDA-MB-231 (Hormone Receptor; HR-) and B. MCF7 (HR+), overexpressing different fragments of the LCOR protein. Normalized to empty vector expression. Data represent mean. C. Schematic representation of LCOR WT and fragments, and main experimentally described interaction regions.

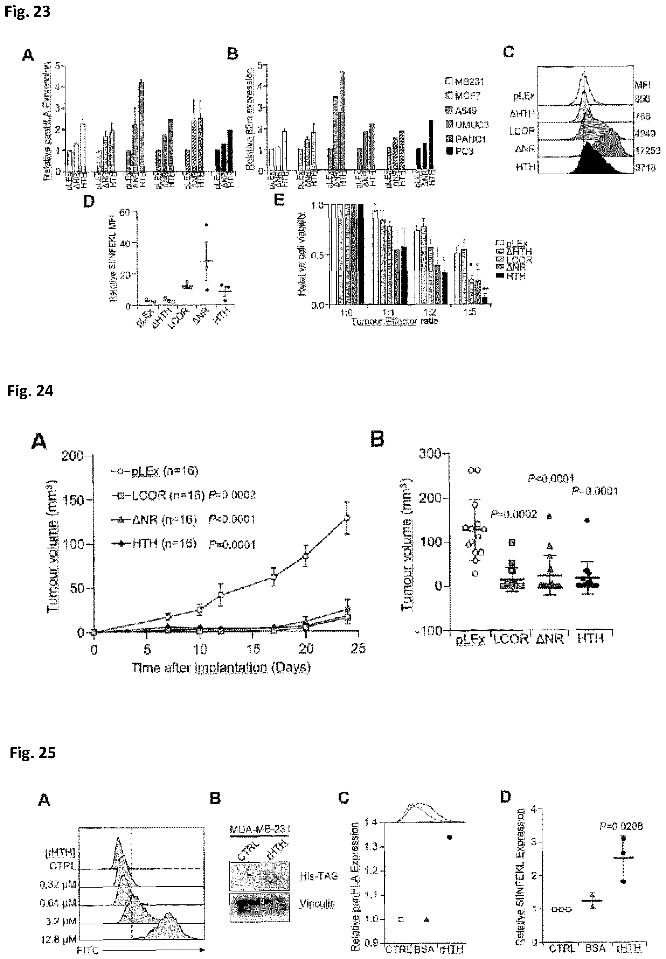

**FIG.23****. LCOR mutants in different types of cancer cells.**
   A-B. Flow cytometry analysis normalized to empty viral vector of pan-HLA (A) and β2M (B) expression levels of triple negative MDA-MB-231; ER-/PGR-/AR-; n=3) and estrogen receptor positive (MCF7; ER+/PGR+/AR+; n=3) breast, lung (A549; ; ER-/PGR-/AR+; n=2), bladder (UM-UC-3; ; ER-/PGR-/AR+ n=1), pancreas (PANC1; ; ER-/PGR-/AR-; n=1-2), and prostate (PC3; ; ER+/PGR-/AR-; n=1) cancer cell lines overexpressing different fragments of the LCOR protein. C. Representative flow cytometry spectra comparing SIINFEKL peptide presentation in AT3-OVA cells overexpressing several LCOR fragments. D. Flow cytometry analysis normalized to isotype of SIINFEKL peptide presentation in AT3-OVA cells overexpressing several LCOR fragments. E. Cytotoxic Lymphocyte (CTL) assays of AT3-OVA cells after 72 h of coculture with OT-1 CD8+ T cells comparing the relative cell viability of tumor cells overexpressing different LCOR fragments at several effector and tumor cell ratios (n=3). Data represents mean±SEM. Statical differences were determined using two-way ANOVA.
**FIG.24****. Tumor growth of LCOR, LCOR-dNR, and LCOR-HTH in immunocompetent mice in vivo.**
   A. Growth curves of orthotopic 4TO7 transplanted tumors with ectopic expression of the indicated LCOR variants. Data represent mean±SEM. B. Tumor volume of orthotopic 4TO7 implanted cells at final timepoint. Data represent mean±SD. Statical differences were determined using non-parametrical Kruskal-Wallis H-test.
**FIG.25****. Recombinant HTH miniprotein (rHTH) cell penetration and induction of tumor immunogenicity.** A. Representative flow cytometry spectra comparing rHTH accumulation inside cells. Cells treated for 15 minutes at 37ºC with different rHTH concentrations. rHTH was labelled previously with Alexa488 fluorophore. B. Representative western blot of His-Tag performed by loading MDA-MB-231 lysate treated with 3.2 µM of rHTH (1:500; Invitrogen, #MA1-135-HRP). C. Flow cytometry analysis of pan-HLA levels in MDA-MB-231 cells treated for 48 hours with 32 µM of rHTH or BSA as a control. D. Measurement of antigen processing and presentation machinery (APM) activity: Flow cytometry analysis of SIINFEKL peptide presentation levels in AT3-OVA cells treated with 32 µM of rHTH or BSA as a control. Data represent mean±SEM. Statical differences were determined using non-parametrical Mann-Whitney U-test.

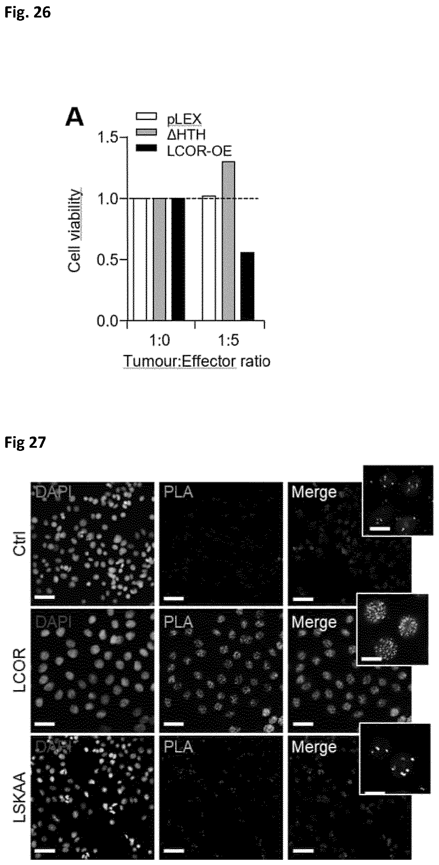

**FIG.26****. Pancreas tumor cells with LCOR-OE coculture with immune cells.** Cytotoxic Lymphocyte (CTL) assays of PANC1 cells after 72 h of coculture with human peripheral blood mononuclear cells (PBMCs) at the indicated conditions, and effector and tumor cell ratios. Delta-HTH refers to LCOR without HTH domain, used here as control. Data represents mean±SEM.
**FIG.27** PLA of LCOR and ERα in MCF7 cells overexpressing LCOR or LSKAA mutant treated with vehicle or 10% charcoal medium for 16 h prior fixation. Representative microscopy images for the PLA green signal and DAPI nuclear staining are shown.

### DESCRIPTION OF EMBODIMENTS

The present invention provides a novel and improved cancer therapy based on an isolated or synthesized Ligand-dependent corepressor (LCOR) or on a mutant or fragment of a Ligand-dependent corepressor (LCOR) or on a polynucleotide sequence coding for the said LCOR or for the said mutant or fragment of a Ligand-dependent corepressor (LCOR), wherein the mutant or fragment of a Ligand-dependent corepressor (LCOR) is characterized by comprising a HTH domain, and wherein said mutant or fragment of a Ligand-dependent corepressor (LCOR) is further characterized by the absence or inactivation of a Nuclear Receptor Binding Domain. In addition, the present invention particularly provides a fragment of the Ligand-dependent corepressor (LCOR) comprising, consisting of or consisting essentially of SEQ ID NO 23

(MPRKKRGRYRQYNSEILEEAISVVMSGKMSVSKAQSIYGIPHSTLEYKVKERLGTLKNPPKKKMKLMRSE GPDVSVKIELDPQGEAAQSANESKNE) or a sequence which is at least 90%, 91%, 92%, 93%, 94%, 95%, 96%, 97%, 98%, or 100% identical to SEQ ID NO 23.

It is noted that as used herein, LCoR can also be referred to as LCOR.

In the context of the present invention, "Ligand-dependent corepressor (LCOR)" shall be understood as the wild-type form of LCOR. Functionally it is a transcription factor that induce the transcriptional expression of antigen presentation machinery (APM) genes, especially when does not interact with nuclear receptors. Its function can be abolished by the interaction with nuclear receptors (such as the oestrogen receptor among others). The full LCOR sequence is represented in figures 12 of the present invention. The full protein and DNA sequences of the LCOR wild type form are respectively represented in sequences 1 and 2 below:
**PROT SEQ (SEQ ID NO 1)** 
MQRMIQQFAAEYTSKNSSTQDPSQPNSTKNQSLPKASPVTTSPTAATTQNPVLSKLLMADQD
SPLDLTVRKSQSEPSEQDGVLDLSTKKSPCAGSTSLSHSPGCSSTQGNGRPGRPSQYRPDGL
RSGDGVPPRSLQDGTREGFGHSTSLKVPLARSLQISEELLSRNQLSTAASLGPSGLQNHGQH
LILSREASWAKPHYEFNLSRMKFRGNGALSNISDLPFLAENSAFPKMALQAKQDGKKDVSHS
SPVDLKIPQVRGMDLSWESRTGDQYSYSSLVMGSQTESALSKKLRAILPKQSRKSMLDAGPD
SWGSDAEQSTSGQPYPTSDQEGDPGSKQPRKKRGRYRQYNSEILEEAISVVMSGKMSVSKAQ
SIYGIPHSTLEYKVKERLGTLKNPPKKKMKLMRSEGPDVSVKIELDPQGEAAQSANESKNE
**cDNA SEQ (SEQ ID NO 2)**

ATGCAGCGAATGATCCAACAATTTGCTGCTGAATATACCTCAAAAAATAGCTCTACTCAGGA
CCCCAGCCAGCCCAATAGCACAAAGAACCAAAGCCTGCCGAAAGCATCTCCAGTCACCACCT
CTCCCACGGCTGCAACTACTCAGAACCCTGTGCTCAGCAAACTTCTCATGGCTGACCAAGAC
TCACCTCTGGACCTTACTGTCAGAAAGTCTCAGTCAGAACCTAGCGAACAAGACGGTGTACT
TGATCTGTCCACTAAGAAAAGTCCATGTGCTGGCAGCACTTCCCTGAGCCACTCTCCAGGCT
GCTCCAGTACTCAAGGAACGGGCGACCTGGGAGACCCAGCCAGTACCGCCCAGACGGACTT
CGGAGTGGTGATGGGGTACCTCCAAGAAGCTTACAGGATGGAACCAGGGAAGGTTTTGGACA
CTCCACATCACTCAAAGTTCCACTGGCTCGATCCCTGCAGATTAGTGAAGAACTACTGAGCA
GAAACCAATTGTCCACAGCTGCCAGCCTTGGGCCATCTGGATTACAGAATCATGGACAACAC
TTAATATTATCCAGGGAAGCCTCTTGGGCAAAACCACATTACGAGTTCAACCTCAGCCGTAT
GAAGTTCAGGGGAAATGGTGCACTCAGCAACATCAGTGACCTTCCTTTTCTTGCAGAAAACT
CTGCCTTTCCAAAAATGGCACTTCAAGCAAAACAAGATGGAAAAAGGATGTGAGCCATTCA
TCTCCTGTAGATTTAAAGATACCACAAGTTCGAGGAATGGATCTTTCTTGGGAGTCTCGCAC
TGGTGATCAGTACAGCTATAGCTCTTTGGTAATGGGTTCACAAACGGAGAGCGCGCTTAGTA
AAAAATTAAGGGCTATTCTTCCAAAACAAAGTAGAAAAAGCATGTTAGATGCTGGACCCGAT
TCTTGGGGCTCAGATGCTGAGCAGTCTACCTCTGGACAGCCATATCCCACATCGGATCAAGA
AGGAGACCCTGGCTCCAAGCAGCCTCGGAAGAAAAGAGGGCGTTACAGACAGTACAACAGTG
AGATACTGGAGGAAGCAATCTCAGTGGTTATGAGTGGAAAAATGAGTGTTTCCAAAGCTCAG
AGTATTTATGGGATTCCCCACAGTACACTGGAGTACAAAGTAAAGGAGAGGCTGGGCACTTT
GAAAAACCCTCCAAAGAAAAAGATGAAATTAATGAGGTCGGAGGGGCCAGATGTTTCTGTAA
AGATTGAATTAGATCCCCAGGGAGAGGCAGCACAAAGTGCAAATGAATCAAAAAACGAG

In the context of the present invention, "a mutant or fragment of a Ligand-dependent corepressor (LCOR)" shall be understood as a further, preferably improved, fragment or form of the Ligand-dependent corepressor (LCOR) that is capable of transcriptionally inducing the expression of antigen presentation machinery (APM) genes as measured by Flow cytometry analysis of MDA-MB-231 cells transduced, transformed or transfected to overexpress the specified LCOR fragment or mutant by using an antibody to measure pan-HLA-ABC (by using, for example, an antibody such as Anti-pan-HLA-ABC-PECyS (clone W6/32) (see Pérez-Núñez et al. Nat Cancer 2022)), expression at the cell surface of these cells by means of fluorescence intensitiy (MFI). Such expression of pan-HLA-ABC at the cell surface of these transduced cells by means of fluorescence intensity (MFI) should be at least 1.5-fold, 2 fold, 3 fold, 4 fold or at least 10 fold change or increase as compared to MDA-MB-231 cells control cells transduced with an empty vector. It is noted that in the present invention all mutants and fragments of the present invention avoid the interaction with nuclear receptors.

In the context of the present invention, "Nuclear Receptor Binding Domain" shall be understood as the domain by which LCOR interacts with nuclear receptors, such as the oestrogen receptor.

In the context of the present invention, "the absence or inactivation of a Nuclear Receptor Binding Domain" shall be understood as significant reduction or absence of interaction of LCOR wildtype and/or mutants with the NRs expressed in the cells analysed. Such as estrogen receptor (ER), progesterone receptor (PGR), or androgen receptor (AR), among others. This can be measure by the amount of signal in proximity ligation assay (PLA), which is a highly sensitive technique used to detect protein-protein interactions (Fig. 27). It relies on the use of different antibodies conjugated to oligonucleotides, which, upon close proximity, enable the amplification and detection of signals through fluorescence, allowing for visualization at the single-molecule level within cells. Another technique used is the or co-immunoprecipitation of LCOR followed by western blot of specific NRs. In example:
Estrogen receptor α sequence (Uniprot ref. P03372)
Androgen receptor sequence (Uniprot ref. P10275 
MEVQLGLGRVYPRPPSKTYRGAFQNLFQSVREVIQNPGPRHPEAASAAPPGASLLLLQQQQQQQQQQQQQQQQ
QQQQQETSPRQQQQQGEDGSPQAHRRGPTGYLVLDEEQQPSQPQSALECHPERGCVPEPGAAVAASKGLPQQLP
APPDEDDSAAPSTLSLLGPTFPGLSSCSADLKDILSEASTMQLLQQQQQEAVSEGSSSGRAREASGAPTSSKDNY
LGGTSTISDNAKELCKAVSVSMGLGVEALEHLSPGEQLRGDCMYAPLLGVPPAVRPTPCAPLAECKGSLLDDSAG
KSTEDTAEYSPFKGGYTKGLEGESLGCSGSAAAGSSGTLELPSTLSLYKSGALDEAAAYQSRDYYNFPLALAGPP
PPPPPPHPHARIKLENPLDYGSAWAAAAAQCRYGDLASLHGAGAAGPGSGSPSAAASSSWHTLFTAEEGQLYGPC
GGGGGGGGGGGGGGGGGGGGGGGEAGAVAPYGYTRPPQGLAGQESDFTAPDVWYPGGMVSRVPYPSPTCVKSEMG
PWMDSYSGPYGDMRLETARDHVLPIDYYFPPQKTCLICGDEASGCHYGALTCGSCKVFFKRAAEGKQKYLCASRN
DCTIDKFRRKNCPSCRLRKCYEAGMTLGARKLKKLGNLKLQEEGEASSTTSPTEETTQKLTVSHIEGYECQPIFL
NVLEAIEPGVVCAGHDNNQPDSFAALLSSLNELGERQLVHVVKWAKALPGFRNLHVDDQMAVIQYSWMGLMVFAM
GWRSFTNVNSRMLYFAPDLVFNEYRMHKSRMYSQCVRMRHLSQEFGWLQITPQEFLCMKALLLFSIIPVDGLKNQ
KFFDELRMNYIKELDRIIACKRKNPTSCSRRFYQLTKLLDSVQPIARELHQFTFDLLIKSHMVSVDFPEMMAEII
SVQVPKILSGKVKPIYFHTQ
Progesterone receptor sequence (Uniprot ref. P06401)

MTELKAKGPRAPHVAGGPPSPEVGSPLLCRPAAGPFPGSQTSDTLPEVSAIPISLDGLLFPRPCQGQDPSDEKTQ
DQQSLSDVEGAYSRAEATRGAGGSSSSPPEKDSGLLDSVLDTLLAPSGPGQSQPSPPACEVTSSWCLFGPELPED
PPAAPATQRVLSPLMSRSGCKVGDSSGTAAAHKVLPRGLSPARQLLLPASESPHWSGAPVKPSPQAAAVEVEEED
GSESEESAGPLLKGKPRALGGAAAGGGAAAVPPGAAAGGVALVPKEDSRFSAPRVALVEQDAPMAPGRSPLATTV
MDFIHVPILPLNHALLAARTRQLLEDESYDGGAGAASAFAPPRSSPCASSTPVAVGDFPDCAYPPDAEPKDDAYP
LYSDFQPPALKIKEEEEGAEASARSPRSYLVAGANPAAFPDFPLGPPPPLPPRATPSRPGEAAVTAAPASASVSS
ASSSGSTLECILYKAEGAPPQQGPFAPPPCKAPGASGCLLPRDGLPSTSASAAAAGAAPALYPALGLNGLPQLGY
QAAVLKEGLPQVYPPYLNYLRPDSEASQSPQYSFESLPQKICLICGDEASGCHYGVLTCGSCKVFFKRAMEGQHN
YLCAGRNDCIVDKIRRKNCPACRLRKCCQAGMVLGGRKFKKFNKVRVVRALDAVALPQPVGVPNESQALSQRFTF
SPGQDIQLIPPLINLLMSIEPDVIYAGHDNTKPDTSSSLLTSLNQLGERQLLSVVKWSKSLPGFRNLHIDDQITL
IQYSWMSLMVFGLGWRSYKHVSGQMLYFAPDLILNEQRMKESSFYSLCLTMWQIPQEFVKLQVSQEEFLCMKVLL
LLNTIPLEGLRSQTQFEEMRSSYIRELIKAIGLRQKGVVSSSQRFYQLTKLLDNLHDLVKQLHLYCLNTFIQSRA
LSVEFPEMMSEVIAAQLPKILAGMVKPLLFHKK

The inventors of the present invention have surprisingly found several mutants or fragments of a Ligand-dependent corepressor (LCOR) that are capable of transcriptionally inducing the expression of antigen presentation machinery (APM) genes, as long as at least the HTH domain is preserved. Moreover, some of these specific mutants or fragments significantly improved the anti-cancer activity of LCOR. In this sense, please refer to figure 8 wherein, remarkably, LSKAA by itself (without the need of combining it with an anti-PDL1 such as atezolizumab) reduces the cell viability not only significantly over the control but even over the combination of LCOR and an anti-PDL1 such as atezolizumab.

In addition, the inventors of the present invention have found that the intratumoral administration of Lcor mRNA therapy using poly(beta aminoesters) (pBAE) nanoparticles significantly reduced tumor growth in 4TO7 tumors, a triple-negative breast cancer cell line.

In addition, we expect a much stronger effect of LCOR mRNA in combination with immunotherapy compared to control groups reaching a complete remission of all tumors.

In addition, the combination of intratumoral administration of Lcor mRNA therapy and intraperitoneal CTLA-4 immune checkpoint blockade (ICB) significantly improved outcomes in mice bearing 4TO7 triple-negative breast tumors. Mice treated with this combination showed a significant extended life expectancy compared to those receiving either treatment alone. The complete response rate increased from 50% with CTLA4 therapy alone to 100% when combined with Lcor mRNA therapy.

On the other hand, cytotoxic lymphocyte (CTL) assays conducted with AT3-OVA cells after 72 hours of coculture with OT-1 CD8+ T cells and anti-PD-1 treatment revealed that ectopic overexpression of Lcor synergizes with PD-1 blockade in vitro, showing a significant enhancement in CTL activity against AT3-OVA cells. This increased effect underscores the potential for combining Lcor overexpression with PD-1 inhibitors to improve therapeutic efficacy.

Furthermore, the present invention demonstrates that Lcor enhances tumor clearance in adoptive cell transfer (ACT) therapy. AT3-OVA Lcor-overexpressing cells were implanted in the mammary fat pad of immunodeficient NSG mice. Once tumor reaches 0.5x0.5 cm, isolated CD8+ T cells from OT-I mice were adoptively administered intravenously as therapy. The results obtained and presented in the instant specification showed a strong increase of CD8 T-cell-mediated anti-tumoral response in Lcor-OE tumors. These results demonstrate that Lcor makes tumor cell much more vulnerable to T cell therapy leading to tumor eradication. Even though ACT therapy alone has a modest effect, the presence of Lcor clearly improves the effectiveness of engineered cell therapy.

Furthermore, the present invention demonstrates that LCOR and LCOR mutants induces APM in different types of cancers. Nuclear receptors are transcription factors normally activated by ligands responding to steroids, thyroid hormones, vitamins and other molecules. These intracellular receptors regulate key functions in reproduction, development, and physiology, In order to explore the effectiveness of LCOR or LCOR mutants in several cancer types, we transduced LCOR and the LSKAA mutant and measured APM gene expression by qPCR in different cancer type cell lines, including NR+ and NR- cell types based on ER/PR/AR levels. We know thar NR can interfere LCOR activity and block the LCOR-mediated APM induction. The results presented herein show how LSKAA is able to induce APM independently to NR+ cell lines, to similar levels as LCOR wild-type in NR- cells. These results clearly show how LSKAA is more efficient increasing APM gene expression by avoiding NR interactions. Moreover, flow cytometry analysis shows how LSKAA can consistently induce HLA-class-I genes in NR-negative and NR-positive cancer cell lines. Importantly, LSKAA can strongly increase MHC-I molecules (HLAs) measured by flow cytometry. Indicating that despite LCOR can induce a strong APM response in several cancer types, LSKAA is even more efficient to do so.

Moreover, considering that both ΔNR and HTH fragments are able to inhibit tumor growth similarly to the original LCOR and given the presence of large unstructured regions in LCOR and ΔNR that could dampens the stability of the protein, we decided to test whether fragment HTH of SEQ ID NO 23, which is more structured due to the HTH domain, performed the function previously observed in ectopic expression assays by its own (naked protein). Synthesized recombinant HTH miniprotein was also tagged it with a fluorescent probe using a maleimide reaction. This approach allowed us to covalently bind the fluorescent probe to a cysteine residue that we added to the protein. The incorporation of this fluorescent tag facilitated testing protein internalization and accumulation within the cells. Strikingly, rHTH treatment in vitro showed efficient uptake and accumulation of the tagged mini-protein through flow cytometry and western blot analysis in the MDA-MB-231 cells, providing clear evidence of its cellular penetration. This is remarkable, since it indicates that the naked rHTH miniprotein has cell penetrating capacity and also nuclear penetration for proper localization and function. Importantly, we found that rHTH significantly increase HLAs in the MDA-MB-231 cell line, as measured by flow cytometry. Additionally, it enhanced the presentation of the OVA peptide by AT3-OVA cells compared to controls treated with bovine serum albumin. These results indicate that the recombinant HTH mini-protein can mediate the expected LCOR functions in APM induction, suggesting its potential to improve tumor immunogenicity and immune recognition in these cellular models. These are remarkable findings that open high potential therapeutic application for the rHTH or SEQ ID NO 23 as a standalone or naked agent, administered parenterally in humans for cancer treatment.

Thus, a first aspect of the present invention refers to an isolated or synthesized Ligand-dependent corepressor (LCOR) or a mutant or fragment thereof, selected from any one of the list consisting of:
a. a fragment of the Ligand-dependent corepressor (LCOR) comprising, consisting of or consisting essentially of SEQ ID NO 23 (MPRKKRGRYRQYNSEILEEAISVVMSGKMSVSKAQSIYGIPHSTLEYKVKERLGTLKNP PKKKMKLMRSEGPDVSVKIELDPQGEAAQSANESKNE) or a sequence which is at least 90%, 91%, 92%, 93%, 94%, 95%, 96%, 97%, 98%, or 100% identical to SEQ ID NO 23;
b. a mutant or fragment of a Ligand-dependent corepressor (LCOR) or a polynucleotide sequence coding for the said mutant or fragment of a Ligand-dependent corepressor (LCOR), wherein the mutant or fragment of a Ligand-dependent corepressor (LCOR) is characterized by comprising amino acid sequence SEQ ID NO 3, or a sequence which is at least 90%, 91%, 92%, 93%, 94%, 95%, 96%, 97%, 98%, or 100% identical to SEQ ID NO 3, and at least one NLS motif (nuclear localization signal) of amino acid sequence SEQ ID NO 4 or a sequence which is at least 90%, 91%, 92%, 93%, 94%, 95%, 96%, 97%, 98%, or 100% identical to SEQ ID NO 4; and wherein the mutant or fragment of a Ligand-dependent corepressor (LCOR) is further characterized by the absence or disruption/inactivation of the Nuclear Receptor Binding Domain; and
c. a Ligand-dependent corepressor (LCOR) or a polynucleotide sequence coding for the said Ligand-dependent corepressor (LCOR), wherein the LCOR is of SEQ ID NO 1 or a sequence which is at least 90%, 91%, 92%, 93%, 94%, 95%, 96%, 97%, 98%, or 100% identical to SEQ ID NO 1.

In a preferred embodiment of the first aspect of the invention, the isolated or synthesized Ligand-dependent corepressor (LCOR) is the fragment of a Ligand-dependent corepressor (LCOR) consisting essentially of or consisting of SEQ ID NO 23 (MPRKKRGRYRQYNSEILEEAISVVMSGKMSVSKAQSIYGIPHSTLEYKVKERLGTLKNPPKKKMKLMRSE GPDVSVKIELDPQGEAAQSANESKNE) or a sequence which is at least 90%, 91%, 92%, 93%, 94%, 95%, 96%, 97%, 98%, or 100% identical to SEQ ID NO 23, wherein preferably said LCOR mutant or fragment is a recombinant protein.

In a preferred embodiment of the first aspect of the invention, the isolated or synthesized Ligand-dependent corepressor (LCOR) is a mutant or fragment of a Ligand-dependent corepressor (LCOR) as defined in paragraph b) above.

It is noted that, as indicated above, the "a mutant or fragment of a Ligand-dependent corepressor (LCOR)" shall be understood as a further, preferably improved, fragment or form of the Ligand-dependent corepressor (LCOR) that is capable of transcriptionally inducing the expression of antigen presentation machinery (APM) genes as measured by Flow cytometry analysis of MDA-MB-231 cells transduced, transformed or transfected to overexpress the specified LCOR fragment or mutant by using an antibody to measure pan-HLA-ABC (by using, for example, an antibody such as Anti-pan-HLA-ABC-PECyS (clone W6/32) (see Pérez-Núñez et al. Nat Cancer 2022)), expression at the cell surface of these cells by means of fluorescence intensitiy (MFI). Such expression of pan-HLA-ABC at the cell surface of these transduced cells by means of fluorescence intensity (MFI) should be at least 1.5-fold, 2 fold, 3 fold, 4 fold or at least 10 fold change or increase as compared to MDA-MB-231 cells control cells transduced with an empty vector. It is noted that in the present invention all mutants and fragments of the present invention avoid the interaction with nuclear receptors as measured by PLA and co-immunoprecipitation.

It is further noted that the term "polynucleotide sequence" as used in the first aspect of the invention above, refers to any oligonucleotide or polynucleotide molecule including DNA and/or RNA oligonucleotide or polynucleotide molecules as well as "oligonucleotide or polynucleotide analogues". "Oligonucleotide or polynucleotide analogues" are the molecules derived from the DNA and/or RNA oligonucleotide or polynucleotide molecules that incorporate some chemical modification in at least one of the nucleotide units that form them, such as in the phosphate group, the pentose or one of the nitrogenous bases. With regard to the possible chemical modifications included in the oligonucleotide or polynucleotide analogues, the term will be applied especially in the case of one or more of the usual modifications known to those skilled in the art of molecular biology, in terms of basic research and, in particular, in the search for therapeutic applications of these molecules.

In a preferred embodiment of the first aspect of the invention, the HTH domain is characterized by comprising or consisting of amino acid sequence VSKAQSIYGIPHSTLEYKVKE (SEQ ID NO. 3). Preferably, the HTH domain is characterized by comprising, consisting of, or consisting essentially of an amino acid sequence VSKAQSIYGIPHSTLEYKVKE, or a sequence which is at least 70%, 75%, 80%, 85%, 90%, 91%, 92%, 93%, 94%, 95%, 96%, 97%, 98%, or 100% identical to VSKAQSIYGIPHSTLEYKVKE (SEQ ID NO 3).

In another preferred embodiment of the first aspect of the invention, the mutant or fragment of a Ligand-dependent corepressor (LCOR) is capable of transcriptionally inducing the expression of antigen presentation machinery (APM) genes and is characterized by comprising amino acid sequence VSKAQSIYGIPHSTLEYKVKE (SEQ ID NO 3), or a sequence which is at least 70%, 75%, 80%, 85%, 90%, 91%, 92%, 93%, 94%, 95%, 96%, 97%, 98%, or 100% identical to VSKAQSIYGIPHSTLEYKVKE (SEQ ID NO 3), and a NLS motif of amino acid sequence PRKKRGR (SEQ ID NO 4) or a sequence which is at least 70%, 75%, 80%, 85%, 90%, 91%, 92%, 93%, 94%, 95%, 96%, 97%, 98%, or 100% identical to SEQ ID NO 4. Preferably, the mutant or fragment of a Ligand-dependent corepressor (LCOR), or a polynucleotide sequence coding for the said mutant or fragment of a Ligand-dependent corepressor (LCOR), is capable of transcriptionally inducing the expression of antigen presentation machinery (APM) genes and the said mutant or fragment of a Ligand-dependent corepressor (LCOR) is characterized by comprising, consisting of, or consisting essentially of amino acid sequence SEQ ID NO 5, or a sequence which is at least 70%, 75%, 80%, 85%, 90%, 91%, 92%, 93%, 94%, 95%, 96%, 97%, 98%, or 100% identical to SEQ ID NO 5. Preferably, the mutant or fragment of a Ligand-dependent corepressor (LCOR), or a polynucleotide sequence coding for the said mutant or fragment of a Ligand-dependent corepressor (LCOR), is capable of transcriptionally inducing the expression of antigen presentation machinery (APM) genes and the said mutant or fragment of a Ligand-dependent corepressor (LCOR) is characterized by comprising, consisting of, or consisting essentially of amino acid sequence SEQ ID NO 7, or a sequence which is at least 70%, 75%, 80%, 85%, 90%, 91%, 92%, 93%, 94%, 95%, 96%, 97%, 98%, or 100% identical to SEQ ID NO 7.

In the context of the present invention, "NLS motif" shall be understood as an amino acid sequence that 'tags' a protein for import into the cell nucleus.
>LCOR_ HTH
PROT SEQ (SEQ ID NO 5) 
PRKKRGRYRQYNSEILEEAISVVMSGKMSVSKAQSIYGIPHSTLEYKVKERLGTLKNPPKKK
MKLMRSEGPDVSVKIELDPQGEAAQSANESKNE
cDNA SEQ (SEQ ID NO 6) 
CCTCGGAAGAAAAGAGGGCGTTACAGACAGTACAACAGTGAGATACTGGAGGAAGCAATCTC
AGTGGTTATGAGTGGAAAAATGAGTGTTTCCAAAGCTCAGAGTATTTATGGGATTCCCCACA
GTACACTGGAGTACAAAGTAAAGGAGAGGCTGGGCACTTTGAAAAACCCTCCAAAGAAAAAG
ATGAAATTAATGAGGTCGGAGGGGCCAGATGTTTCTGTAAAGATTGAATTAGATCCCCAGGG
AGAGGCAGCACAAAGTGCAAATGAATCAAAAAACGAG
>LCOR_ HTH-IDP
PROT SEQ (SEQ ID NO 7) 
QSRKSMLDAGPDSWGSDAEQSTSGQPYPTSDQEGDPGSKQPRKKRGRYRQYNSEILEEAISV
VMSGKMSVSKAQSIYGIPHSTLEYKVKERLGTLKNPPKKKMKLMRSEGPDVSVKIELDPQGE
AAQSANESKNE
cDNA SEQ (SEQ ID NO 8)

CAAAGTAGAAAAAGCATGTTAGATGCTGGACCCGATTCTTGGGGCTCAGATGCTGAGCAGTC
TACCTCTGGACAGCCATATCCCACATCGGATCAAGAAGGAGACCCTGGCTCCAAGCAGCCTC
GGAAGAAAAGAGGGCGTTACAGACAGTACAACAGTGAGATACTGGAGGAAGCAATCTCAGTG
GTTATGAGTGGAAAAATGAGTGTTTCCAAAGCTCAGAGTATTTATGGATTCCCCACAGTAC
ACTGGAGTACAAAGTAAAGGAGAGGCTGGGCACTTTGAAAAACCCTCCAAAGAAAAAGATGA
AATTAATGAGGTCGGAGGGGCCAGATGTTTCTGTAAAGATTGAATTAGATCCCCAGGGAGAG
GCAGCACAAAGTGCAAATGAATCAAAAAACGAG

The term "sequence identity" refers to a percentage value obtained when two sequences are compared using a pairwise sequence alignment tool. In the present case, the sequence identity is obtained using the global alignment tool "EMBOSS Needle" using the default settings (Rice et al., 2000. Trends Genet. 16(6):276-7; Li et al., 2015. Nucleic Acids Res. 43(W1):W580-4). The global alignment tool is available at: https://www.ebi.ac.uk/Tools/psa/ In another preferred embodiment of the first aspect of the invention, the mutant or fragment of a Ligand-dependent corepressor (LCOR) is characterized by an amino acid sequence comprising amino acid sequence VSKAQSIYGIPHSTLEYKVKE (SEQ ID NO 3), or a sequence which is at least 70%, 75%, 80%, 85%, 90%, 91%, 92%, 93%, 94%, 95%, 96%, 97%, 98%, or 100% identical to VSKAQSIYGIPHSTLEYKVKE (SEQ ID NO 3), at least one NLS motif of amino acid sequence PRKKRGR (SEQ ID NO 4) or a sequence which is at least 70%, 75%, 80%, 85%, 90%, 91%, 92%, 93%, 94%, 95%, 96%, 97%, 98%, or 100% identical to SEQ ID NO 4, and a HDAC motif of amino acid sequence NLSRMKFRGNGALSNISDLPFLAENSAFPKMALQAKQDGKKDVSHSSPVDLKIPQVRGMDLSWESRTG DQYSYSSLVMGSQTESALSKKLRAILPKQSRKSMLDAGPDSWGSDAEQS (SEQ ID NO 9) or a sequence which is at least 70%, 75%, 80%, 85%, 90%, 91%, 92%, 93%, 94%, 95%, 96%, 97%, 98%, or 100% identical to SEQ ID NO 9. Preferably, the mutant or fragment of a Ligand-dependent corepressor (LCOR), or a polynucleotide sequence coding for the said mutant or fragment of a Ligand-dependent corepressor (LCOR), is capable of transcriptionally inducing the expression of antigen presentation machinery (APM) genes and the said mutant or fragment of a Ligand-dependent corepressor (LCOR) is characterized by comprising, consisting of, or consisting essentially of amino acid sequence SEQ ID NO 10, or a sequence which is at least 70%, 75%, 80%, 85%, 90%, 91%, 92%, 93%, 94%, 95%, 96%, 97%, 98%, or 100% identical to SEQ ID NO 10. Preferably, the mutant or fragment of a Ligand-dependent corepressor (LCOR), or a polynucleotide sequence coding for the said mutant or fragment of a Ligand-dependent corepressor (LCOR), is capable of transcriptionally inducing the expression of antigen presentation machinery (APM) genes and the said mutant or fragment of a Ligand-dependent corepressor (LCOR) is characterized by comprising, consisting of, or consisting essentially of amino acid sequence SEQ ID NO 12, or a sequence which is at least 70%, 75%, 80%, 85%, 90%, 91%, 92%, 93%, 94%, 95%, 96%, 97%, 98%, or 100% identical to SEQ ID NO 12.
>LCOR_HDAC
PROT SEQ(SEQ ID NO 10) 
NLSRMKFRGNGALSNISDLPFLAENSAFPKMALQAKQDGKKDVSHSSPVDLKIPQVRGMDLS
WESRTGDQYSYSSLVMGSQTESALSKKLRAILPKQSRKSMLDAGPDSWGSDAEQSTSGQPYP
 
TSDQEGDPGSKQPRKKRGRYRQYNSEILEEAISVVMSGKMSVSKAQSIYGIPHSTLEYKVKE
RLGTLKNPPKKKMKLMRSEGPDVSVKIELDPQGEAAQSANESKNE
**cDNA SEQ(SEQ ID NO 11)** 
AACCTCAGCCGTATGAAGTTCAGGGAAATGGTGCACTCAGCAACATCAGTGACCTTCCTTT
TCTTGCAGAAAACTCTGCCTTTCCAAAAATGGCACTTCAAGCAAAACAAGATGGAAAAAAGG
ATGTGAGCCATTCATCTCCTGTAGATTTAAAGATACCACAAGTTCGAGGAATGGATCTTTCT
TGGGAGTCTCGCACTGGTGATCAGTACAGCTATAGCTCTTTGGTAATGGGTTCACAAACGGA
GAGCGCGCTTAGTAAAAAATTAAGGGCTATTCTTCCAAAACAAAGTAGAAAAAGCATGTTAG
ATGCTGGACCCGATTCTTGGGGCTCAGATGCTGAGCAGTCTACCTCTGGACAGCCATATCCC
ACATCGGATCAAGAAGGAGACCCTGGCTCCAAGCAGCCTCGGAAGAAAAGAGGGCGTTACAG
ACAGTACAACAGTGAGATACTGGAGGAAGCAATCTCAGTGGTTATGAGTGGAAAAATGAGTG
TTTCCAAAGCTCAGAGTATTTATGGATTCCCCACAGTACACTGGAGTACAAAGTAAAGGAG
AGGCTGGGCACTTTGAAAAACCCTCCAAAGAAAAAGATGAAATTAATGAGGTCGGAGGGGCC
AGATGTTTCTGTAAAGATTGAATTAGATCCCCAGGGAGAGGCAGCACAAAGTGCAAATGAAT
CAAAAAACGAG
**>LCOR_ΔNR/ΔCTBP**
**PROT SEQ(SEQ ID NO 12)** 
KSPCAGSTSLSHSPGCSSTQGNGRPGRPSQYRPDGLRSGDGVPPRSLQDGTREGFGHSTSLK
VPLARSLQISEELLSRNQLSTAASLGPSGLQNHGQHLILSREASWAKPHYEFNLSRMKFRGN
GALSNISDLPFLAENSAFPKMALQAKQDGKKDVSHSSPVDLKIPQVRGMDLSWESRTGDQYS
YSSLVMGSQTESALSKKLRAILPKQSRKSMLDAGPDSWGSDAEQSTSGQPYPTSDQEGDPGS
KQPRKKRGRYRQYNSEILEEAISVVMSGKMSVSKAQSIYGIPHSTLEYKVKERLGTLKNPPK
KKMKLMRSEGPDVSVKIELDPQGEAAQSANESKNE
**cDNA SEQ(SEQ ID NO 13)**

AAAAGTCCATGTGCTGGCAGCACTTCCCTGAGCCACTCTCCAGGCTGCTCCAGTACTCAAGG
GAACGGGCGACCTGGGAGACCCAGCCAGTACCGCCCAGACGGACTTCGGAGTGGTGATGGGG
TACCTCCAAGAAGCTTACAGGATGGAACCAGGGAAGGTTTTGGACACTCCACATCACTCAAA
GTTCCACTGGCTCGATCCCTGCAGATTAGTGAAGAACTACTGAGCAGAAACCAATTGTCCAC
AGCTGCCAGCCTTGGGCCATCTGGATTACAGAATCATGGACAACACTTAATATTATCCAGGG
AAGCCTCTTGGGCAAAACCACATTACGAGTTCAACCTCAGCCGTATGAAGTTCAGGGGAAAT
GGTGCACTCAGCAACATCAGTGACCTTCCTTTTCTTGCAGAAAACTCTGCCTTTCCAAAAAT
GGCACTTCAAGCAAAACAAGATGGAAAAAGGATGTGAGCCATTCATCTCCTGTAGATTTAA
AGATACCACAAGTTCGAGGAATGGATCTTTCTTGGGAGTCTCGCACTGGTGATCAGTACAGC
TATAGCTCTTTGGTAATGGGTTCACAAACGGAGAGCGCGCTTAGTAAAAAATTAAGGGCTAT
TCTTCCAAAACAAAGTAGAAAAAGCATGTTAGATGCTGGACCCGATTCTTGGGGCTCAGATG
CTGAGCAGTCTACCTCTGGACAGCCATATCCCACATCGGATCAAGAAGGAGACCCTGGCTCC
AAGCAGCCTCGGAAGAAAAGAGGGCGTTACAGACAGTACAACAGTGAGATACTGGAGGAAGC
AATCTCAGTGGTTATGAGTGGAAAAATGAGTGTTTCCAAAGCTCAGAGTATTTATGGATTC
CCCACAGTACACTGGAGTACAAAGTAAAGGAGAGGCTGGGCACTTTGAAAAACCCTCCAAAG

AAAAAGATGAAATTAATGAGGTCGGAGGGCCAGATGTTTCTGTAAAGATTGAATTAGATCC
CCAGGGAGAGGCAGCACAAAGTGCAAATGAATCAAAAAACGAG

In another preferred embodiment of the first aspect of the invention, the mutant or fragment of a Ligand-dependent corepressor (LCOR) is further characterized by comprising any one of a CTBP1 binding site and/or a CTBP2 binding site. Preferably, the mutant or fragment of a Ligand-dependent corepressor (LCOR), or a polynucleotide sequence coding for the said mutant or fragment of a Ligand-dependent corepressor (LCOR), is capable of transcriptionally inducing the expression of antigen presentation machinery (APM) genes and the said mutant or fragment of a Ligand-dependent corepressor (LCOR) is characterized by an amino acid sequence comprising amino acid sequence VSKAQSIYGIPHSTLEYKVKE (SEQ ID NO 3), or a sequence which is at least 70%, 75%, 80%, 85%, 90%, 91%, 92%, 93%, 94%, 95%, 96%, 97%, 98%, or 100% identical to VSKAQSIYGIPHSTLEYKVKE (SEQ ID NO 3), at least one NLS motif of amino acid sequence PRKKRGR (SEQ ID NO 4) or a sequence which is at least 70%, 75%, 80%, 85%, 90%, 91%, 92%, 93%, 94%, 95%, 96%, 97%, 98%, or 100% identical to SEQ ID NO 4, a HDAC motif of amino acid sequence SEQ ID NO 9 or a sequence which is at least 70%, 75%, 80%, 85%, 90%, 91%, 92%, 93%, 94%, 95%, 96%, 97%, 98%, or 100% identical to SEQ ID NO 9, and a CTBP1 binding site and/or a CTBP2 binding site, wherein the CTBP1 binding site is of amino acid sequence PLDLTVR (SEQ ID NO 14) or a sequence which is at least 70%, 75%, 80%, 85%, 90%, 91%, 92%, 93%, 94%, 95%, 96%, 97%, 98%, or 100% identical to SEQ ID NO 14, and wherein the CTBP2 binding site is of amino acid sequence VLDLSTK (SEQ ID NO 15) or a sequence which is at least 70%, 75%, 80%, 85%, 90%, 91%, 92%, 93%, 94%, 95%, 96%, 97%, 98%, or 100% identical to SEQ ID NO 15. Preferably, the mutant or fragment of a Ligand-dependent corepressor (LCOR), or a polynucleotide sequence coding for the said mutant or fragment of a Ligand-dependent corepressor (LCOR), is capable of transcriptionally inducing the expression of antigen presentation machinery (APM) genes and the said mutant or fragment of a Ligand-dependent corepressor (LCOR) is characterized by comprising, consisting of, or consisting essentially of amino acid sequence SEQ ID NO 16, or a sequence which is at least 70%, 75%, 80%, 85%, 90%, 91%, 92%, 93%, 94%, 95%, 96%, 97%, 98%, or 100% identical to SEQ ID NO 16.

In the context of the present invention, "CTBP1 binding site" shall be understood as protein domain interacting with the CTBP1 (C-Terminal Binding Protein 1).

In the context of the present invention, "CTBP2 binding site" shall be understood as protein domain interacting with the CTBP2 (C-Terminal Binding Protein 2).
**>LCOR_ΔNR**
PROT SEQ (SEQ ID NO 16) 
PLDLTVRKSQSEPSEQDGVLDLSTKKSPCAGSTSLSHSPGCSSTQGNGRPGRPSQYRPDGLR
SGDGVPPRSLQDGTREGFGHSTSLKVPLARSLQISEELLSRNQLSTAASLGPSGLQNHGQHL
ILSREASWAKPHYEFNLSRMKFRGNGALSNISDLPFLAENSAFPKMALQAKQDGKKDVSHSS
PVDLKIPQVRGMDLSWESRTGDQYSYSSLVMGSQTESALSKKLRAILPKQSRKSMLDAGPDS
WGSDAEQSTSGQPYPTSDQEGDPGSKQPRKKRGRYRQYNSEILEEAISVVMSGKMSVSKAQS
IYGIPHSTLEYKVKERLGTLKNPPKKKMKLMRSEGPDVSVKIELDPQGEAAQSANESKNE
cDNA SEQ (SEQ ID NO 17)

CCTCTGGACCTTACTGTCAGAAAGTCTCAGTCAGAACCTAGCGAACAAGACGGTGTACTTGA
TCTGTCCACTAAGAAAAGTCCATGTGCTGGCAGCACTTCCCTGAGCCACTCTCCAGGCTGCT
CCAGTACTCAAGGGAACGGGCGACCTGGGAGACCCAGCCAGTACCGCCCAGACGGACTTCGG
AGTGGTGATGGGGTACCTCCAAGAAGCTTACAGGATGGAACCAGGGAAGGTTTTGGACACTC
CACATCACTCAAAGTTCCACTGGCTCGATCCCTGCAGATTAGTGAAGAACTACTGAGCAGAA
ACCAATTGTCCACAGCTGCCAGCCTTGGGCCATCTGGATTACAGAATCATGGACAACACTTA
ATATTATCCAGGGAAGCCTCTTGGGCAAAACCACATTACGAGTTCAACCTCAGCCGTATGAA
GTTCAGGGGAAATGGTGCACTCAGCAACATCAGTGACCTTCCTTTTCTTGCAGAAAACTCTG
CCTTTCCAAAAATGGCACTTCAAGCAAAACAAGATGGAAAAAGGATGTGAGCCATTCATCT
CCTGTAGATTTAAAGATACCACAAGTTCGAGGAATGGATCTTTCTTGGGAGTCTCGCACTGG
TGATCAGTACAGCTATAGCTCTTTGGTAATGGGTTCACAAACGGAGAGCGCGCTTAGTAAAA
AATTAAGGGCTATTCTTCCAAAACAAAGTAGAAAAAGCATGTTAGATGCTGGACCCGATTCT
TGGGGCTCAGATGCTGAGCAGTCTACCTCTGGACAGCCATATCCCACATCGGATCAAGAAGG
AGACCCTGGCTCCAAGCAGCCTCGGAAGAAAAGAGGGCGTTACAGACAGTACAACAGTGAGA
TACTGGAGGAAGCAATCTCAGTGGTTATGAGTGGAAAAATGAGTGTTTCCAAAGCTCAGAGT
ATTTATGGGATTCCCCACAGTACACTGGAGTACAAAGTAAAGGAGAGGCTGGGCACTTTGAA
AAACCCTCCAAAGAAAAAGATGAAATTAATGAGGTCGGAGGGGCCAGATGTTTCTGTAAAGA
TTGAATTAGATCCCCAGGGAGAGGCAGCACAAAGTGCAAATGAATCAAAAAACGAG

In another preferred embodiment of the first aspect of the invention, the mutant or fragment of a Ligand-dependent corepressor (LCOR), or a polynucleotide sequence coding for the said mutant or fragment of a Ligand-dependent corepressor (LCOR), is capable of transcriptionally inducing the expression of antigen presentation machinery (APM) genes and the said mutant or fragment of a Ligand-dependent corepressor (LCOR) is characterized by comprising or consisting of an amino acid sequence which is at least 70%, 75%, 80%, 85%, 90%, 91%, 92%, 93%, 94%, 95%, 96%, 97%, 98%, or 100% identical to SEQ ID NO: 18.
**>LCOR_ LSKAA**
**PROT SEQ (SEQ** ID NO 18) 
MQRMIQQFAAEYTSKNSSTQDPSQPNSTKNQSLPKASPVTTSPTAATTQNPVLSKAAMADQD
SPLDLTVRKSQSEPSEQDGVLDLSTKKSPCAGSTSLSHSPGCSSTQGNGRPGRPSQYRPDGL
RSGDGVPPRSLQDGTREGFGHSTSLKVPLARSLQISEELLSRNQLSTAASLGPSGLQNHGQH
LILSREASWAKPHYEFNLSRMKFRGNGALSNISDLPFLAENSAFPKMALQAKQDGKKDVSHS
SPVDLKIPQVRGMDLSWESRTGDQYSYSSLVMGSQTESALSKKLRAILPKQSRKSMLDAGPD
SWGSDAEQSTSGQPYPTSDQEGDPGSKQPRKKRGRYRQYNSEILEEAISVVMSGKMSVSKAQ
SIYGIPHSTLEYKVKERLGTLKNPPKKKMKLMRSEGPDVSVKIELDPQGEAAQSANESKNE
**cDNA SEQ (SEQ ID NO 19)**

ATGCAGCGAATGATCCAACAATTTGCTGCTGAATATACCTCAAAAATAGCTCTACTCAGGA
CCCCAGCCAGCCCAATAGCACAAAGAACCAAAGCCTGCCGAAAGCATCTCCAGTCACCACCT
CTCCCACGGCTGCAACTACTCAGAACCCTGTGCTCAGCAAAGCCGCCATGGCTGACCAAGAC
TCACCTCTGGACCTTACTGTCAGAAAGTCTCAGTCAGAACCTAGCGAACAAGACGGTGTACT
TGATCTGTCCACTAAGAAAAGTCCATGTGCTGGCAGCACTTCCCTGAGCCACTCTCCAGGCT
GCTCCAGTACTCAAGGAACGGGCGACCTGGGAGACCCAGCCAGTACCGCCCAGACGGACTT
CGGAGTGGTGATGGGGTACCTCCAAGAAGCTTACAGGATGGAACCAGGGAAGGTTTTGGACA
CTCCACATCACTCAAAGTTCCACTGGCTCGATCCCTGCAGATTAGTGAAGAACTACTGAGCA
GAAACCAATTGTCCACAGCTGCCAGCCTTGGGCCATCTGGATTACAGAATCATGGACAACAC
TTAATATTATCCAGGGAAGCCTCTTGGGCAAAACCACATTACGAGTTCAACCTCAGCCGTAT
GAAGTTCAGGGGAAATGGTGCACTCAGCAACATCAGTGACCTTCCTTTTCTTGCAGAAAACT
CTGCCTTTCCAAAAATGGCACTTCAAGCAAAACAAGATGGAAAAAGGATGTGAGCCATTCA
TCTCCTGTAGATTTAAAGATACCACAAGTTCGAGGAATGGATCTTTCTTGGGAGTCTCGCAC
TGGTGATCAGTACAGCTATAGCTCTTTGGTAATGGGTTCACAAACGGAGAGCGCGCTTAGTA
AAAAATTAAGGGCTATTCTTCCAAAACAAAGTAGAAAAAGCATGTTAGATGCTGGACCCGAT
TCTTGGGGCTCAGATGCTGAGCAGTCTACCTCTGGACAGCCATATCCCACATCGGATCAAGA
AGGAGACCCTGGCTCCAAGCAGCCTCGGAAGAAAAGAGGGCGTTACAGACAGTACAACAGTG
AGATACTGGAGGAAGCAATCTCAGTGGTTATGAGTGGAAAAATGAGTGTTTCCAAAGCTCAG
AGTATTTATGGGATTCCCCACAGTACACTGGAGTACAAAGTAAAGGAGAGGCTGGGCACTTT
GAAAAACCCTCCAAAGAAAAAGATGAAATTAATGAGGTCGGAGGGGCCAGATGTTTCTGTAA
AGATTGAATTAGATCCCCAGGGAGAGGCAGCACAAAGTGCAAATGAATCAAAAAACGAG

In another preferred embodiment of the first aspect of the invention, the mutant or fragment of a Ligand-dependent corepressor (LCOR), or a polynucleotide sequence coding for the said mutant or fragment of a Ligand-dependent corepressor (LCOR), is capable of transcriptionally inducing the expression of antigen presentation machinery (APM) genes and the said mutant or fragment of a Ligand-dependent corepressor (LCOR) is characterized by comprising or consisting of amino acid sequence ID NO 18 (the LSKAA mutant) or an amino acid sequence which is at least 70%, 75%, 80%, 85%, 90%, 91%, 92%, 93%, 94%, 95%, 96%, 97%, 98%, or 100% identical to SEQ ID NO: 18.

In another preferred embodiment of the first aspect of the invention, the mutant or fragment of a Ligand-dependent corepressor (LCOR) consist of amino acid sequence ID NO 18 (the LSKAA mutant).

In another preferred embodiment of the first aspect of the invention or of any of its preferred embodiments, the polynucleotide is a DNA sequence, such as a cDNA (complementary DNA) sequence, or a polyribonucleotide sequence comprising a sequence from a stable mRNA or an mRNA, wherein the DNA sequence or the polyribonucleotide sequence can be efficiently translated to the mutant or fragment sequence of any of the first aspect of the invention or of any of its preferred embodiments.

A second aspect of the invention refers to an expression vector, a polynucleotide DNA/RNA (such as naked mRNA) or plasmid in which a polynucleotide of the first aspect of the invention is disposed, inserted or comprised within or in the said expression vector, polynucleotide DNA/RNA (such as naked mRNA) or plasmid, wherein the said expression vector, polynucleotide DNA/RNA (such as naked mRNA) or plasmid is capable of or is selected for its ability to express (transcription and translation) the (LCOR) mutant or fragment thereof of the first aspect of the invention, in a mammalian cell, preferably in a human cell, more preferably in a human cancer.

As used herein, an expression vector is designed to allow expression (transcription and translation) of the inserted section of DNA/RNA (the polynucleotide of the first aspect of the invention). The expression vector usually carries a promoter and a transcription terminator.

A preferred embodiment of the second aspect of the invention refers to a mixture of expression vectors, polynucleotides DNA/RNA (such as naked mRNA) or plasmids or any combination thereof, wherein at least a portion of the said expression vectors, polynucleotides DNA/RNA (such as naked mRNA) or plasmids or any combination thereof, is selected for its ability to express the (LCOR) mutant or fragment of the first aspect of the invention in a mammalian cell, preferably in a human cell, more preferably in a human cancer; and another portion of the said expression vectors, polynucleotides DNA/RNA (such as naked mRNA) or plasmids or any combination thereof, is selected by its ability to induce one or more neoantigen presentation in a mammalian cell, preferably in a human cell, more preferably in a human cancer.

In a third aspect of the invention, the invention provides compositions that allow delivery of the isolated or synthesized mutant or fragment of a Ligand-dependent corepressor (LCOR) or polynucleotide coding for the same of the first aspect of the invention, or the expression vector, polynucleotide DNA/RNA (such as naked mRNA) or plasmid or any combination thereof, of the second aspect of the invention, in a cell or tissue such as a mammalian cell, preferably a human cell, more preferably a human cancer, *in vitro or in vivo.* Such compositions are also referred to herein as "carriers" or "vehicles".

In a preferred embodiment of the third aspect of the invention, the composition that allows delivery of the expression vector, polynucleotide DNA/RNA (such as naked mRNA) or plasmid as described above is a nano or micro-emulsion or system, an exosome, a lipid nanoparticle, a polymeric nanoparticle, a nanoparticle, a natural or artificial lipoprotein particle, a cationic lipid, a protein-nucleic acid complex, a liposome, a virosome, or a polymer. In an embodiment of the invention, the vectors are viral vectors including lentiviral and AAV (Adeno-associated viruses).

A fourth aspect of the invention refers to a pharmaceutical composition comprising any of the compositions described in the first to third aspects of the invention, in particular the LCOR mutant or fragment or the polynucleotide coding for the same according to the first aspect of the invention, the expression vector, polynucleotide DNA/RNA (such as naked mRNA), plasmid or any combination thereof, according to the second aspect of the invention, or the compositions that allows delivery of the said LCOR mutant or fragment or expression vector, polynucleotide DNA/RNA (such as naked mRNA) or plasmid according to the third aspect of the invention.

Preferably, the pharmaceutical composition comprises: a) any of the compositions described in the first to third aspects of the invention, in particular the LCOR mutant or fragment or the polynucleotide coding for the same according to the first aspect of the invention, the expression vector, polynucleotide DNA/RNA (such as naked mRNA), plasmid or any combination thereof, according to the second aspect of the invention, or the compositions that allows delivery of the said LCOR mutant or fragment or expression vector, polynucleotide DNA/RNA (such as naked mRNA) or plasmid according to the third aspect of the invention; and b) at least one immune checkpoint inhibitor (ICI). Preferably, wherein the at least one ICI inhibits CTLA-4, PD-1, PD-L1, Lag-3, Tim-3, TIGIT or a combination thereof, preferably inhibits PD-1 or PD-L1, more preferably inhibits PD-1; preferably wherein the ICI is selected between ipilimumab, tremelimumab, nivolumab, dostarlimab, pembrolizumab, pidilizumab, MEDI0680, atezolizumab, BMS-936559, MEDI4736 (durvalumab), MSB0010718C (avelumab), TSR-022 (cobolimab), BMS-986016, TSR- 033, BMS-986207, and combinations thereof, preferably is selected between pembrolizumab, atezolizumab, dostarlimab and combinations thereof.

Also preferably, the pharmaceutical composition comprises: a) any of the compositions described in the first to third aspects of the invention, in particular the LCOR mutant or fragment or the polynucleotide coding for the same according to the first aspect of the invention, the expression vector, polynucleotide DNA/RNA (such as naked mRNA), plasmid or any combination thereof, according to the second aspect of the invention, or the compositions that allows delivery of the said LCOR mutant or fragment or expression vector, polynucleotide DNA/RNA (such as naked mRNA) or plasmid according to the third aspect of the invention; and b) at least one adoptive cell therapeutic composition. Preferably wherein the at least one adoptive cell therapeutic composition comprises a cell type selected from a group consisting of a tumor infiltrating lymphocyte (TIL), T-cell receptor modified lymphocytes and chimeric antigen receptor modified lymphocytes, preferably wherein the adoptive cell therapeutic composition comprises a cell type selected from a group consisting of engineered cells, T-cells, CD8+ cells, CD4+ cells, NK-cells, delta-gamma T- cells, regulatory T-cells, and peripheral blood mononuclear cells, preferably wherein the adoptive cell therapeutic composition comprises T-cells.

A fifth aspect of the invention refers to any of the compositions described in the first to third aspects of the invention, in particular the LCOR, the LCOR fragment of SEQ ID NO 23, the LCOR mutant or fragment or the polynucleotide coding for the same according to the first aspect of the invention, the expression vector, polynucleotide DNA/RNA (such as naked mRNA), plasmid or any combination thereof, according to the second aspect of the invention, or the compositions that allows delivery of the said LCOR mutant or fragment or expression vector, polynucleotide DNA/RNA (such as naked mRNA) or plasmid according to the third aspect of the invention, or any of the pharmaceutical compositions according to the fourth aspect, for use in therapy or for use as a medicament. Preferably for use in immunotherapy, particularly for cancer immunotherapy, more particularly for use in the treatment of cancer such as breast cancer or Triple Negative Breast Cancer (TNBC).

In a preferred embodiment of the fifth aspect of the invention, the cancer is characterized by comprising or consisting of cancer cells that are androgen or estrogen or progesteron receptor positive (AR+ or ER+ or PGR+), or any other nuclear receptor (NR) positive. Preferably, the cancer is characterized by comprising or consisting of cancer cells that are androgen or estrogen receptor positive (AR+ or ER+). More preferably, the cancer is characterized by comprising or consisting of cancer cells that are estrogen receptor positive (ER+).

As used herein, the term "estrogen receptor positive (ER+) or progesterone receptor (PGR+) positive, shall be understood as hormone receptor (HR+) proteins capable of interacting with the wild type form of LCOR in breast cancer.

As used herein, the term "androgen or estrogen receptor positive (AR+ or ER+) or progesterone receptor (PGR+) positive, or any other nuclear receptor (NR) positive" shall be understood as nuclear receptor proteins capable of interacting with the wild type form of LCOR.

In another preferred embodiment of the fifth aspect of the invention, the cancer is selected from chondrosarcoma, Ewing's sarcoma, malignant fibrous histiocytoma of bone/osteosarcoma, osteosarcoma, rhabdomyosarcoma, heart cancer, astrocytoma, brainstem glioma, pilocytic astrocytoma, ependymoma, primitive neuroectodermal tumor, cerebellar astrocytoma, cerebral astrocytoma, glioma, medulloblastoma, neuroblastoma, oligodendroglioma, pineal astrocytoma, pituitary adenoma, visual pathway and hypothalamic glioma, breast cancer, invasive lobular carcinoma, tubular carcinoma, invasive cribriform carcinoma, medullary carcinoma, male breast cancer, phyllodes tumor, inflammatory breast cancer, adrenocortical carcinoma, islet cell carcinoma (endocrine pancreas), multiple endocrine neoplasia syndrome, parathyroid cancer, pheochromocytoma, thyroid cancer, Merkel cell carcinoma, uveal melanoma, retinoblastoma, anal cancer, appendix cancer, cholangiocarcinoma, carcinoid tumor, colon cancer, extrahepatic bile duct cancer, gallbladder cancer, gastric (stomach) cancer, gastrointestinal carcinoid tumor, gastrointestinal stromal tumor, hepatocellular cancer, pancreatic cancer, islet cell, rectal cancer, bladder cancer, cervical cancer, endometrial cancer, extragonadal germ cell tumor, ovarian cancer, ovarian epithelial cancer (surface epithelial-stromal tumor), ovarian germ cell tumor, penile cancer, renal cell carcinoma, renal pelvis and ureter, transitional cell cancer, prostate cancer, testicular cancer, gestational trophoblastic tumor, urethral cancer, uterine sarcoma, vaginal cancer, vulvar cancer, Wilms tumor, esophageal cancer, head and neck cancer, nasopharyngeal carcinoma, oral cancer, oropharyngeal cancer, paranasal sinus and nasal cavity cancer, pharyngeal cancer, salivary gland cancer, hypopharyngeal cancer, basal cell carcinoma, squamous cell carcinoma, skin adnexal tumors (e.g. sebaceous carcinoma), melanoma, sarcomas of primary cutaneous origin (e.g. dermatofibrosarcoma protuberans), lymphomas of primary cutaneous origin (e.g. mycosis fungoides), bronchial adenomas/carcinoids, small cell lung cancer, mesothelioma, non-small cell lung cancer, pleuropulmonary blastoma, laryngeal cancer, thymoma and thymic carcinoma, Kaposi sarcoma, epithelioid hemangioendothelioma (EHE), desmoplastic small round cell tumor, liposarcoma, preferably the cancer is selected from lung cancer, melanoma, renal carcinoma, bladder carcinoma, prostate cancer, mesothelioma, glioblastoma and breast cancer, more preferably the cancer is selected from melanoma, bladder cancer and breast cancer, preferably ER+ breast cancer or Triple Negative Breast Cancer (TNBC).

In a preferred embodiment of the fifth aspect of the invention, the composition is the pharmaceutical composition according to the fourth aspect of the invention, wherein said composition is for use in a method of treating cancer, preferably for cancer immunotherapy. In particular, wherein the cancer is characterized by comprising or consisting of cancer cells that are androgen, estrogen or progesterone receptor positive, preferably wherein the cancer is an estrogen receptor (ER) positive breast cancer. In particular, wherein the cancer is selected from chondrosarcoma, Ewing's sarcoma, malignant fibrous histiocytoma of bone/osteosarcoma, osteosarcoma, rhabdomyosarcoma, heart cancer, astrocytoma, brainstem glioma, pilocytic astrocytoma, ependymoma, primitive neuroectodermal tumor, cerebellar astrocytoma, cerebral astrocytoma, glioma, medulloblastoma, neuroblastoma, oligodendroglioma, pineal astrocytoma, pituitary adenoma, visual pathway and hypothalamic glioma, breast cancer, invasive lobular carcinoma, tubular carcinoma, invasive cribriform carcinoma, medullary carcinoma, male breast cancer, phyllodes tumor, inflammatory breast cancer, adrenocortical carcinoma, islet cell carcinoma (endocrine pancreas), multiple endocrine neoplasia syndrome, parathyroid cancer, pheochromocytoma, thyroid cancer, Merkel cell carcinoma, uveal melanoma, retinoblastoma, anal cancer, appendix cancer, cholangiocarcinoma, carcinoid tumor, colon cancer, extrahepatic bile duct cancer, gallbladder cancer, gastric (stomach) cancer, gastrointestinal carcinoid tumor, gastrointestinal stromal tumor, hepatocellular cancer, pancreatic cancer, islet cell, rectal cancer, bladder cancer, cervical cancer, endometrial cancer, extragonadal germ cell tumor, ovarian cancer, ovarian epithelial cancer (surface epithelial-stromal tumor), ovarian germ cell tumor, penile cancer, renal cell carcinoma, renal pelvis and ureter, transitional cell cancer, prostate cancer, testicular cancer, gestational trophoblastic tumor, urethral cancer, uterine sarcoma, vaginal cancer, vulvar cancer, Wilms tumor, esophageal cancer, head and neck cancer, nasopharyngeal carcinoma, oral cancer, oropharyngeal cancer, paranasal sinus and nasal cavity cancer, pharyngeal cancer, salivary gland cancer, hypopharyngeal cancer, basal cell carcinoma, squamous cell carcinoma, skin adnexal tumors (e.g. sebaceous carcinoma), melanoma, sarcomas of primary cutaneous origin (e.g. dermatofibrosarcoma protuberans), lymphomas of primary cutaneous origin (e.g. mycosis fungoides), bronchial adenomas/carcinoids, small cell lung cancer, mesothelioma, non-small cell lung cancer, pleuropulmonary blastoma, laryngeal cancer, thymoma and thymic carcinoma, Kaposi sarcoma, epithelioid hemangioendothelioma (EHE), desmoplastic small round cell tumor, liposarcoma, preferably the cancer is selected from lung cancer, melanoma, renal carcinoma, bladder carcinoma, prostate cancer, mesothelioma, glioblastoma and breast cancer, more preferably the cancer is selected from melanoma, bladder cancer and breast cancer, preferably triple-negative breast cancer. More particularly, wherein the administration(s) of adoptive cell therapeutic composition or the ICI and the isolated or synthesized Ligand-dependent corepressor (LCOR) or a mutant or fragment thereof, or the polynucleotide sequence coding for the same, to a subject is(are) conducted simultaneously or consecutively, in any order. More particularly, wherein there is a time period of one minute to four weeks between the consecutive administration of adoptive cell therapeutic composition or ICI and the mutant or fragment thereof, or the polynucleotide sequence coding for the same, and/or there are several administrations of adoptive cell therapeutic composition or ICI and the mutant or fragment thereof, or the polynucleotide sequence coding for the same. More particularly, wherein the administration of the adoptive cell therapeutic composition and/or ICI and/or the mutant or fragment thereof, or the polynucleotide sequence coding for the same, is conducted through an intra- tumoral, intra-arterial, intravenous, intrapleural, intravesicular, intracavitary or peritoneal injection, or an oral administration.

In another preferred embodiment of the fifth aspect of the invention, when the fifth aspect of the invention refers to any of the compositions described in the first to third aspects of the invention, in particular the LCOR, the LCOR fragment of SEQ ID NO 23, the LCOR mutant or fragment or the polynucleotide coding for the same according to the first aspect of the invention, the expression vector, polynucleotide DNA/RNA (such as naked mRNA), plasmid or any combination thereof, according to the second aspect of the invention, or the compositions that allows delivery of the said LCOR mutant or fragment or expression vector, polynucleotide DNA/RNA (such as naked mRNA) or plasmid according to the third aspect of the invention, the method of treatment can be carried out prior to, simultaneously to, or subsequently to a cancer treatment with an anticancer agent. Preferably, the anticancer agent is selected from, but not restricted to, adoptive cell transfer or an immune checkpoint inhibitor (ICI). That is, in this embodiment any of the compositions described in the first to third aspects of the invention may be administered prior to, simultaneously or sequentially to an anticancer agent. Such combination treatments involving any of the compositions described in the first to third aspects of the invention and an anticancer agent such as an adoptive Cell Transfer or an immune checkpoint inhibitor (ICI), can be achieved by administering any of the compositions described in the first to third aspects of the invention and the anticancer agent at the same time. Such combination treatments can be achieved by administering a single pharmaceutical composition that includes both agents, or by administering two distinct pharmaceutical compositions, at the same time, wherein one composition includes any of the compositions described in the first to third aspects of the invention and the other includes the anticancer agent. Alternatively, treatment with any of the compositions described in the first to third aspects of the invention can precede or follow treatment with the anticancer agent by intervals ranging from minutes to weeks.

In a preferred embodiment, the composition for use of the fifth aspect of the invention is administered before or after surgery.

As used herein, "anticancer agent" is understood as a therapeutic agent to block, reduce, eliminate cancer cells.

Immune checkpoint inhibitors include agents that inhibit or block or reduce CTLA-4, PD-1, PD-L1, Lag-3, Tim-3, TIGIT and the like. Suitable anti-CTLA-4 therapy agents for use in the present invention, include, without limitation, anti-CTLA-4 antibodies, human anti-CTLA-4 antibodies, mouse anti-CTLA-4 antibodies, mammalian anti-CTLA-4 antibodies, humanized anti-CTLA-4 antibodies, monoclonal anti-CTLA-4 antibodies, polyclonal anti-CTLA-4 antibodies, chimeric anti-CTLA-4 antibodies, ipilimumab, tremelimumab, anti-CD28 antibodies, anti-CTLA-4 adnectins, anti-CTLA-4 domain antibodies, single chain anti-CTLA-4 fragments, heavy chain anti-CTLA-4 fragments, light chain anti- CTLA-4 fragments, inhibitors of CTLA-4 that agonize the co-stimulatory pathway. Suitable anti-PD-1 and anti-PD-L1 therapy agents for use in the present invention, include, without limitation, anti-PD-1 and anti-PD-L1 antibodies, human anti- PD-1 and anti-PD-L1 and anti-PD-L1 antibodies, mouse anti-PD-1 and anti-PD-L1 antibodies, mammalian anti-PD-1 and anti-PD-L1 antibodies, humanized anti-PD-1 and anti-PD-L1 antibodies, monoclonal anti-PD-1 and anti-PD-L1 antibodies, polyclonal anti-PD-1 and anti-PD-L1 antibodies, chimeric anti-PD-1 and anti-PD-L1 antibodies. In specific embodiments, anti-PD-1 therapy agents include nivolumab, pembrolizumab, pidilizumab, MEDI0680, and combinations thereof. In other specific embodiments, anti- PD-L1 therapy agents include atezolizumab, BMS-936559, MEDI4736, MSB0010718C, and combinations thereof. Suitable anti-Lag-3 therapy agents for use in the present invention include, without limitation, BMS-986016 and TSR-033. Suitable anti-Tim-3 therapy agents for use in the present invention include, without limitation, TSR-022 (cobolimab). Suitable anti-TIGIT therapy agents for use in the present invention include, without limitation, BMS-986207.

It is noted that when the composition or the pharmaceutical composition according to the fifth aspect of the invention comprises a fragment of the Ligand-dependent corepressor (LCOR) comprising, consisting of or consisting essentially of SEQ ID NO 23 (MPRKKRGRYRQYNSEILEEAISVVMSGKMSVSKAQSIYGIPHSTLEYKVKERLGTLKNPPKKKMKLMRSE GPDVSVKIELDPQGEAAQSANESKNE) or a sequence which is at least 90%, 91%, 92%, 93%, 94%, 95%, 96%, 97%, 98%, or 100% identical to SEQ ID NO 23, then such composition is used in immunotherapy, particularly for cancer immunotherapy, as a naked or standalone fragment of the Ligand-dependent corepressor (LCOR) comprising, consisting of or consisting essentially of SEQ ID NO 23 (MPRKKRGRYRQYNSEILEEAISVVMSGKMSVSKAQSIYGIPHSTLEYKVKERLGTLKNPPKKKMKLMRSE GPDVSVKIELDPQGEAAQSANESKNE). That is without the involvement of a carrier or vehicle or a chemical moiety that facilitates the cellular uptake of the polypeptide. That is, the polypeptide of SEQ ID NO 23 has a remarkable intrinsic cell penetrating activity that allows the polypeptide to be administered as such (in naked form) without the use of a carrier or vehicle or a chemical moiety that facilitates the cellular uptake of the polypeptide. This in turn means that this specific fragment can be use in immunotherapy, particularly for cancer immunotherapy, more particularly for use in the treatment of cancer such as breast cancer or Triple Negative Breast Cancer (TNBC), wherein the method does not involved the administration of the nucleic acid encoding the polypeptide of SEQ ID NO 23 or any functionally equivalent variant thereof, or wherein the method does not involved the administration of any carrier or vehicle or a chemical moiety that facilitates the cellular uptake of the polypeptide.

The following examples merely illustrate the present invention but do not limit the same.

### EXAMPLES

### MATERIALS AND METHODS

### Ethical regulations

This study complies with all ethical regulations. Clinical patient samples have approval from the Ethical Committee of Clinical Investigation-Mar Park of Health, the institutional review boards at Vall d'Hebron Hospital and from the INCLIVA ethical committee. All individuals gave their informed consent before inclusion. All animal procedures presented in this study were approved by the Ethical Committee for Animal Research of the Barcelona Biomedical Research Park and by regulation from the Departament de Medi Ambient i Habitatge de la Generalitat de Catalunya (Catalonia Government). For all experimental procedures, euthanasia was applied once tumors reached a volume of 1,500 mm3 or when animal health was compromised.

### Animal studies

For this study, mouse strains Balb/c, C57BL/6J, C57BL/6-Tg (TcraTcrb)1100Mjb/J (OT-1), Ptprca (TcrbTcra)Ln1Bdb H2d/J Just Enhanced GFP (JEDI) and NOD.Cg-Prkdcscid Il2rgtm1Wjl/SzJ (NSG) were used. Tumor cells were orthotopically injected into the MFP using 1:1 PBS:Matrigel (Corning). For TIC evaluation, severely immunocompromised NSG female mice were orthotopically transplanted with series of LDA. After 2 weeks, tumor incidence was evaluated by palpation and TIC frequency calculated using extreme limiting dilution analysis (ELDA) software16. For tumor comparison between immunocompetent and immunodeficient strains, 5,000 AT3 or 4TO7 cells were transplanted into the MFP of ten glands of NSG and Balb/c or CS7BL/6J mice. Tumor volume was measured twice weekly with digital calipers, and calculations were applied (π × length × width2/6). In ICB assays, mice were treated with either anti-human/mouse anti-PD-L1 (atezolizumab, clone SP142, Tecentriq) or anti-mouse anti-PD-L1 (BioXCell, clone 10F.9G2, catalog no. BE0101) versus vehicle. Treatments were initiated when tumor size reached 0.5 × 0.5 cm2. Dose regimes were applied at 10 mg kg-1 every 3 or 7 days (as indicated in the different experiments). Depletion of CD4 and CD8 was achieved using anti-CD4 and anti-CD8 neutralizing antibodies, respectively, at 400 µg per mouse once tumors reached 0.5 × 0.5 cm2, followed by a weekly dose of 250 µg. For lung metastasis experiments, 50,000 transduced control and 150,000 Lcor-overexpressing 4TO7 cells were TV injected in Balb/c mice to obtain similar metastatic growth among conditions. After 1 week, metastases were treated with 10 mg kg-1 anti-PD-L1 once weekly. Metastatic lesions were monitored by photon flux bioluminescence (BLI) imaging once per week, and data were collected using Live Image v.4.3.1 in a Perkin Elmer Living Image system. For the humanized xenograft model, NSG mice were orthotopically transplanted with 15,000 MDA-MB-231 cells and 500,000 MCF7 cells (figure 15). Once tumors reached 0.3 × 0.3 cm2, animals were intraperitoneally injected with 10,000,000 peripheral blood mononuclear cells (PBMCs) from a healthy donnors. When tumor size reached 0.5 × 0.5 cm2, the animals were treated with either vehicle or human/mouse crossreactive anti-PD-L1 at 10 mg kg-1 every 3 days.

### Paraffin-embedded tissue samples and PDOs

Paraffin-embedded tissue samples were obtained from a total of four patients with early-stage TNBC pre and post treatment. Two patients were treated with polychemotherapy (abraxane + carboplatin) in combination with atezolizumab (NeoTRIP study) while the other two were treated with TAK649 in combination with anti-PD1 nivolumab (NCT02834247 study). Samples were obtained from the Hospital Clinic of Valencia. Hormonal receptor status was evaluated by IHC (ER- and PGR- were defined as <1% positive stained nuclei), and HER2 was assessed by IHC and fluorescence in situ hybridization.

Small patient-derived tumor pieces (~1-5 cm3) were obtained from surgical resection of patients with TNBC at Hospital del Mar (Barcelona), with previous informed consent. Small tumor pieces were mechanically and enzymatically digested at 37 °C for 2 h in mammary epithelial cell growth medium16 supplemented with enzymes, followed by a short incubation with 5 mg ml-1 dispase (Merck, catalog no. D4693), 0.1 mg ml-1 DNase I (Merck, catalog no. D5025-150) and 0.25% trypsin (Cultek, catalog no. 25300-062). The digested tissue suspension was strained through a 100-µM filter, and single cells were cultured and expanded in vitro as previously described64.

### Cell lines, culture conditions and treatments

All cell lines used in this study-breast cancer cell lines (human MDA-MB-231, MCF7 and mouse Py8119, 4TO7 and AT3)-were obtained from Y. Kang at Princeton University and cultured according to the American Type Culture Collection (ATCC). HMLE cells were obtained from R. Weinberg at MIT. HEK293T cells were obtained from ATCC. Cells were routinely checked for Mycoplasma and all were negative. For this study, anti-human/mouse anti-PD-L1 (atezolizumab, clone SP142, Tecentriq) was used in vitro at 10 µg ml-1 for 72 h; recombinant mouse or human IFN-γ (R&D systems, human catalog no. 285-IF-100; mouse: catalog no. 485-MI-100) at 10 ng ml-1 for 24 h; the JAK1-JAK2 inhibitor ruxolitinib (LC laboratories, catalog no. R-6688) at 1 µM for 24 h; and BX-795 (TBK1i) (Merck, catalog no. SML0694) at 0.5 nM for 24 h.

### CTL assay

For mouse cell coculture assays, splenocytes from OT-I and JEDI mouse models were obtained. Spleens were mechanically minced, and single cells were incubated with ACK buffer (Fisher, catalog no. A1049201). The single-cell suspension was strained over a 70-µM filter and activated overnight with 2 µg ml-1 SIINFEKL (Merck, catalog no. S7951) or HYLSTQSAL (Proimmune, catalog no. F198-2A-E) peptide for OT-1 and JEDI, respectively, in mouse splenocyte medium. CD8+ T cells were purified using the CD8a+ T cell Isolation kit (Milteny, catalog no. 130-104-075) for negative selection, then cocultured with tumor cells for 72 h with or without anti-human/mouse PD-L1 (ref. 65) (atezolizumab, clone SP142, Tecentriq) at 10 µg ml-1. For the T cell-killing assay with β2m knockdown, cell lines were transfected using lipofectamine 3000 (Life Technologies, catalog no. L3000015) with either 100 ρM siRNA negative control (Life Technologies, catalog no. AM4613) or 100 ρM Silencer Pre-designed siRNA against murine β2m (Life Technologies, catalog no. 160820). For human coculture assays, PBMCs were isolated by gradual centrifugation of blood samples from healthy adult donors from the Tissue Bank of Catalonia, and cocultured with tumor cells in coculture medium and with human patient derived organoids (PDOs) (figure 16). Cell viability or enrichment was determined after 72 h by the crystal violet method or flow cytometry, respectively.

### EV isolation and in vitro/in vivo delivery assays

HEK293T cells were cultured in DMEM with 10% extracelular vesicles (EVs)-reduced fetal bovine serum (FBS) (Hyclone) and transfected with either control pLEX-HA vector (control condition) or mouse pLEX-Lcor-HA overexpression construct using Lipofectamine 3000. Medium was changed 24 h after transfection, and supernatants were collected at 48 and 72 h for EV isolation. Cells, debris and large vesicles were first removed by serial spinning at 500g for 10 min, 2,000g for 15 min and 10,000g for 30 min, followed by ultracentrifugation in a Beckman Coulter L-90K ultracentrifuge at 70,000g for 60 min. The resulting EV pellet was resuspended in PBS. EV RNA content was estimated by RNA isolation (Qiagen) and quantification with Nanodrop; protein content was measured by bicinchoninic acid assay. EVs were treated with 10 µg ml-1 RNAse A (Fisher Scientific, catalog no. 12091021) and 5 µl of proteinase K at 20 mg ml-1 (Fisher Scientific, catalog no. EO0491) for 30 min at 37 °C before assay. 4TO7 cells in culture were treated with 4 ng µl-1 EV protein for 72 h at 37 °C. For experimental metastasis in vivo, 50,000 4TO7 cells were TV injected. Mice were treated with 10 mg kg-1 anti-PD-L1 (atezolizumab, Tecentriq) and 8 µg of EV protein (in a total volume of 100 µl of PBS) from control or Lcor-HA EVs via retro-orbital venous sinus injection every 3 days, when BLI imaging (>2 × 106 photon flux) showed metastatic colonization. Metastatic lesions were monitored by photon flux BLI, then lungs were harvested for anti-HA IHC and hematoxylin and eosin staining.

### IF, LSFM and IHC analysis

For IF analysis of LCOR-OE and LCOR mutants, cells were seeded in coverslips, fixed for 1 h with methanol at -20 °C and washed with acetone. Samples were blocked for 30 min using blocking buffer, incubated for 2 h at room temperature with anti-HA, PBS washed and incubated for 1 h at room temperature with a secondary antibody Alexa Fluor 488 anti-rabbit. For IF analysis of LCOR-GFP knock-in reporter system and pan-HLA expression, cells were seeded, fixed for 15 min at room temperature with 4% paraformaldehyde (PFA), washed and blocked for 1 h at room temperature with blocking buffer. Cells were incubated with anti-pan-HLA-ABC for 2 h at room temperature, followed by incubation with an Alexa Fluor 647 anti-mouse. Images were taken using an upright Nikon Eclipse Ni-E fluorescence microscope (Nikon), and data were collected using Ni SetupTool v.1.2.2. Fiji software was used for further analysis.

For imaging of living organoids transduced with the SORE6+ CSC reporter, we used the LS1 live light-sheet microscope system (Viventis). Individual organoids were incubated with anti-pan-HLA-ABC for 2 h at room temperature, washed five times with PBS and incubated with Alexa Fluor 647 anti-mouse for 1 h at room temperature. Hoechst 33342 (Fisher, catalog no. H3570) was used. 3D rendering of organoids was performed using the Clear Volume plugin in FIJI software.

For IHC analysis of infiltration by CD45+ and CD8+ T cells, paraffin-embedded mouse tumor samples were stained with an Auto stainer Plus (Dako) using the 3,3'-diaminobenzidine (DAB) staining method. Tissue sections (3 µm) were stained for CD8 and CD45 antibodies; necrotic areas were excluded from quantification. For LCOR evaluation in clinical samples, paraffin-embedded sections (3 µm) from tumor tissue blocks were stained with anti-LCOR at room temperature for 1 h, followed by incubation with an anti-rabbit Ig dextran polymer (Flex, Agilent). Sections were visualized with DAB and counterstained with hematoxylin. All incubations were performed on the Agilent Link platform. Nuclear immunoreactive score (nIRS) was used to evaluate LCOR expression in samples. nIRS provides a range between 0 and 12 as the product of positive cell proportion score (0-4) (0, 0%; 1, 1-30%; 2, 31-60%; and 3, >60%) and staining intensity score (0-3) (0, no reaction; 1, weak signal; 2, mild signal; and 3, strong signal). For confirmation of Lcor-HA delivery in metastatic lesions, metastatic lungs treated with control or Lcor-HA EVs were collected and fixed in 4% PFA overnight. Paraffin-embedded sections (3 µm) from tissue blocks were stained with anti-HA at room temperature for 2 h. For all IHC analyses performed in this study, slides were visualized and analyzed using either QuPath-0.2.0 (ref. 66) or CellSens software.

### Time-lapse confocal microscopy imaging

Stained tumor cells (CellTracker Deep Red; Fisher, catalog no. C34565) were cocultured with stained CD8+ T cells (CellTracker CM-Dil Dye; Fisher, catalog no. C7000) at a 1:1 ratio in coverslips µ-Slide 8 Well (lbidi, catalog no. 80826) for 16 h in RPMI medium containing SYTOX green dye (Invitrogen, no. S7020). Forty random zones were selected and analyzed using Zeiss Cell Observer HS (Zeiss), taking images every 5 min in the green, red, deep red and phase contrast channels. Images were processed and visualized using FIJI software.

### Viral production and transduction of cell lines

HEK293T cells were transfected with lentiviral plasmids jointly with pocket plasmid (VSVG) and gag-pol plasmid (pCMV-R8.91), following the standard lentiviral packaging protocol. Cell lines were transduced in six-well plates, with concentrated viruses in the appropriate medium for each cell line containing 8 µg ml-1 Polybrene and selected with the corresponding antibiotic resistance.

### ChIP and ChlP-seq library preparation

For ChIP-qPCR and sequencing, cells were grown on 150-mm2 plates, fixed, lysed and sonicated for seven cycles of 30 min on/30 min off using Bioruptor Pico Tubes (Diagenode) with Sonication Beads (Diagenode) in a Bioruptor Sonicator (Diagenode). Samples were incubated overnight at 4 °C with rotation, with either 5 µg of anti-HA antibody or 5 µg of goat anti-rabbit IgG (R&D, no. SC-2025). Dynabeads Protein A for Immunoprecipitation (Fisher Scientific) was used for chromatin isolation. Immunoprecipitated chromatin was washed, eluted and purified using the Qiagen DNA purification kit (Qiagen). For ChlP-seq, chromatin quality and quantity were checked using the Agilent Bioanalyzer. Libraries were sequenced (50-base pair (bp) single-end) on a HiSeq 2500 platform (Illumina). The quality of fastq files was checked with FastQC software (https://www.bioinformatics.babraham.ac.uk/projects/fastqc/). Reads were aligned with the bowtie2 (ref. 69) mapper to release 27 of the Homo sapiens Gencode version of the genome (GRCh38/hg38 assembly) (https://www.gencodegenes.org/human/release_27.html). Quality of the mapped files (BAM format) was checked with QualiMap. Peaks70 on individual samples were identified using the MACS2 (ref. 71) program (narrow peaks with q < 0.1 were initially selected). Overlap of peaks across biological replicates was retrieved using the Bedtools72 suite of tools.

### Knock-in generation

We generated two different LCOR knock-in types in MDA-MB-231 in the endogenous LCOR of human chromosome 10: LCOR tagged with GFP (LCOR-GFP) and LCOR tagged with HA (×3) (IRES-GFP). We cloned single-guide RNAs targeting the 3' of exon 8 into the pX330-U6 plasmid. Insertion of each guide was checked by PCR and Sanger sequencing. To construct donor vectors for LCOR-GFP and LCOR-3 × HA-IRES-GFP knock-in, homology arms of 1 kb targeting the cutting site were amplified and inserted in a homology direct repair (HDR)-plasmid donor backbone. Plasmid sequences were verified by Sanger sequencing. MDA-MB-231 was cotransfected with lipofectamine 3000 with plasmids HDR-GFP-HDR or HDR-3 × HA-IRES-GFP-HDR and pX330-U6-sgRNA at a 10:1 ratio, respectively. Cells were harvested and single-cell sorted for GFP+. Single clones were tested by flow cytometry and Sanger sequencing.

### Molecular cloning and plasmids

LCOR-overexpression plasmids (controls pLEX-MCS and pLEX-HA; pLEX- mouse Lcor and human LCOR-HA, LCOR-LSKAA-HA and LCOR-ΔHTH-HA) and an OVA-overexpressing plasmid (pLEX-OVA-IRES-mCherry) were obtained from the laboratory of Y. Kang. pLEX-Lcor-HA was generated by PCR amplification of mouse Lcor complementary DNA and the addition of HA at the 3' end, inserted together into pLEX-MCS after digestion with Spel and Agel (NEB). For gene knockdown assays, shRNA were purchased from Sigma-Aldrich for targeting of mouse gene Lcor (no. TRCN0000085107) and human gene LCOR (nos. TRCN0000016306 and TRCN0000436034). Other plasmids used were the SORE6 series31 including minCMV-GFP, minCMV-RFP, SORE6-minCMV-GFP and SORE6-minCMV-RFP, plus the proteasomal-activity pQCXIN/ZsGreen reporter46. The ISRE reporter was generated by the introduction of ISRE sequences (six tandem repeats of the interferon-stimulated response element (ISRE) 5'-CAGTTTCACTTTCCC-'3) in front of the minimal CMV promoter (mCMVp)-RFP after removal of binding sites of the SORE6-minCMV-RFP construct using BstZ17I-HF and Clal (NEB) restriction enzymes. A mutated version of the ISRE reporter was also designed by mutation of two thymines to guanines on two highly conserved thymine triplets. Sequence insertion was assessed by Sanger sequencing.

### Flow cytometry analysis and cell sorting

Flow cytometry analysis data were collected on either a Fortessa Flow Cytometer (BD) or with LSRII Flow cytometry (BD) using FACSDiva v.9.0 software, and analyzed by FlowJo software v.10.8.1 (FlowJo). Cells were strained through a 70-µM filter, counted and diluted in PBS + 10% FBS buffer before proceeding with a general staining protocol using anti-SIINFEKL (OVA), anti-mouse or human PD-L1, intracellular staining anti-TAP1, proteasomal activity using the pQCXIN reporter system46, pan-HLA-ABC, anti-mouse or human β2M, pan-H2-Kd/Dd, CSC SORE6 reporter31 and ISRE reporter in cell lines or organoids; and anti-CD69 in OT-1 CD8+ T cells. For analysis of tumor-infiltrating lymphocytes, tumors were disaggregated as described above. Cells were strained through a 70-µM filter, counted and diluted in PBS + 10% FBS buffer before proceeding with the staining protocol using anti-CD45, anti-CD3, anti-CD8, anti-CD4, anti-CD11c, anti-CD11b and anti-F4/80. For cell cycle analysis, cells were fixed with ice-cold methanol for 2 h at 4 °C, stained with propidium iodide buffer and analyzed for DNA content by flow cytometry.

Either FacsAria (BD) or Influx (BD) cell sorter equipment was used to isolate CD24lo/CD44hi CSC populations from PDOs, which were then incubated with anti-CD24 and anti-CD44. For cell sorting from cell lines, cells were washed with PBS, trypsinized, counted and diluted. Poorly and highly immunogenic cells, and stable OVA-expressing AT3 and Py8119 cells, were incubated with anti-OVA and isolated gating for OVA-/low and OVAhigh populations. For SORE6 system validation in our mouse models, Py8119 and AT3 cells SORE6-pCMV-GFP were separated into GFP+ and GFP- cells. For CSC isolation from MDA-MB-231, cells were stained using anti-CD44 and anti-CD104 and sorted by CD44hi/CD104hi-enriched CSC and CD44hi/CD104lo-nonenriched CSC populations; ALDH- was compared with ALDH+ using the Aldefluor Kit (Stemcell technologies) and gated using DEAB control to delimit the ALDH+ population, following the manufacturer's instructions. HMLE cells were sorted by CD44hi/CD24lo-enriched CSC and CD44lo/CD24hi-nonenriched CSC populations43. For CSC isolation in cell lines 4TO7 and AT3, CD24hi/CD29hi CSC and CD24lo/CD29lo non-CSC populations and CD44hi/CD24lo CSC and CD44lo/CD24hi non-CSC populations were isolated, respectively. Cells were incubated with anti-CD29, anti-CD24 and anti-CD44.

### RT-qPCR analysis

Total mRNA was purified using the RNeasy Mini Kit (Qiagen) and reverse transcribed into cDNA using the High-Capacity cDNA Reverse Transcription Kit (Life Technologies). RT-qPCR was performed using LightCycler 480 SYBR Green I Master (Merck). Data were collected using QuantStudio 12 K Flex software.

### RNA-seq analysis

RNA was isolated from tumors using the Qiagen RNA extraction Kit. Poly-A sequencing was selected for library preparation, and samples were sequenced using the Illumina Hi-Seq 2500 platform with 1 × 50-bp settings at the Centre of Genomic Regulation (CRG). Quality checking of raw data (fastq files) was done with FastQC software (https://www.bioinformatics.babraham.ac.uk/projects/fastqc/). Estimation of ribosomal RNA in the raw data was obtained with riboPicker74. Raw reads were aligned with the STAR mapper75 to the Mus musculus genome (Gencode release M24 of the GRMm38/mm10 assembly: https://www.gencodegenes.org/mouse/release_M24.html). The raw count of reads per gene and per sample was obtained with STAR (quantMode TranscriptomeSAM GeneCounts option). The R/Bioconductor package DESeq2 v.1.30.1 was used to assess differential expression between experimental groups (Wald statistical test plus false discovery rate (FDR) correction). Genes for which the sum of raw counts across all samples was <1 were discarded. Genes are considered differentially expressed if their absolute log2 fold change was >1 and their adjusted P < 0.05. With these parameters, we generated an ICB-resistant signature taking the top 300 genes upregulated in IRT versus control cells.

### Clinical dataset analysis

Clinical assessment of LCOR expression was performed using RNA-seq transcriptomic data from the TONIC trial (no. EGAS0001003535)40, which included 53 metastatic TNBC preinduced patient samples with nine responders (one CR and eight partial responses) and 44 nonresponders (one stable disease and 43 progression disease); RNA-seq transcriptomic data of stage I-III TNBC pretreated samples (durvalumab in combination with olaparib) from the I-SPY2 trial (no. GSE173839)52 with nine responders and 12 nonresponders; and RNA-seq transcriptomic data from 30 on-treatment metastatic melanoma biopsies with either pembrolizumab or nivolumab (no. phs001919)76 including 13 patients with progression disease, three with stable disease, ten with partial response and two with CR. The METABRIC dataset was obtained from the open access cBioportal website (http://www.cbioportal.org/index.do), and mRNA expression data from 186 patients with TNBC were analyzed. METABRIC TNBC and TONIC trial data were also used in GSEA and GSVA.

### GSEA, GO and ChEA

Transcriptomic and ChlP-seq data generated in this study were submitted and analyzed for GO and ChEA. For GO analysis, up- and downregulated genes in IRT cells and promoter/enhancer regions from LCOR ChlP-seq were interrogated for enriched pathways using BioCarta signatures. For ChEA77, upregulated genes in IRT were interrogated for enriched transcription factors.

GSEA was used for correlation of signatures in stratified datasets, performing 1,000 random permutations of their labeled phenotypes and obtaining P and q values and normalized enrichment score (NES). Genes were ranked using the formula r(g) = sign (FCg) (1 - Pg), where r(g) is the enrichment score of each gene, FCg reflects median fold change (FC) between two conditions and Pg represents the P value of the Wilcoxon rank-sum test40. The following datasets were used: METABRIC, in which samples were stratified as higher or lower than LCOR expression levels based on median expression after normality was confirmed; and the TONIC dataset40, stratified by responders and nonresponders. IRT transcriptomic data were also compared to IRT control cells. Datasets were interrogated with different signatures: the public signature of embryonic stem cell-like (BENPORATH_ES_1)29 with 379 genes; the LlU breast ER- CSC signature (generated by median expression of ER- breast CSCs isolated by CD24-/CD44+ from HCC1954, MC1, SUM149 and SUM159 cells)34 with 164 UP genes qualifying as >2FC; the human Kyoto Encyclopedia of Genes and Genomes (KEGG: M16004) signature of APM with 88 APM-related genes; the mouse GO 0048002 signature of APM) with 76 APM-related genes; our generated ICB-resistant signature of 300 UP genes qualifying as >1FC and adjusted P > 0.05; and the previously described immunoresistant signature53 in melanoma patients.

### GSVA

The GSVA package was used to establish an enrichment score representing the enrichment of signatures across patients78. GSVA was applied using the following datasets: IRT transcriptomic RNA-seq data, and METABRIC TNBC clinical data and transcriptomic data from 39 ER- breast cancer cell lines from the CCLE. Datasets were interrogated with the following gene signatures: BENPORATH_ES_1 (ref. 29); NOS (NANOG, OCT4, SOX2) targets29; LlU breast ER- CSC34; APM signature (KEGG: M16004); our IRS; and the melanoma immunotherapy-resistant signature (Jerby-Arnon)53.

### Deconvolution and immunophenotype analysis

For estimation of immune infiltrating cells, the xCell algorithm (https://xcell.ucsf.edu/)50 was used on METABRIC TNBC data. A nonhierarchical k-means cluster analysis of four clusters based on Euclidean distance was applied, and LCOR expression was assessed in the various clusters or immune phenotypes.

### Protein structure prediction

To make the structure prediction for the different LCOR variants, the sequence was obtained from UniProt (http:\\www.uniprot.org) and the modifications were made deleting or changing the corresponding squence parts. The structure prediction for these sequences was performed using AlphaFold v3. For later visualization, we obtained the structure with a higher prediction score for each of the variants.

### Statistical analysis and reproducibility

No statistical method was used to predetermine sample size in animal studies; rather, this was guided by pilot and previous studies. Randomization among litters was performed before injection time, with animals of a similar age and female sex. Researchers were not blinded to allocation during experimentation and outcome since it was necessary to know the treatment groups. Tumor initiation assays in vivo were monitored by a trained technician in a blinded fashion. For in vitro experiments no statistical method was used to predetermine sample size, all samples being analyzed equally, and thus no randomization was required. Tissue staining scores were determined by three independent researchers blinded to sample information. For all in vivo and in vitro experiments, independent biological replicates are indicated in the figure legends. Each result is represented by mean ± s.e.m. For all experiments, normality and variance equality were checked by Kolmogorov-Smirnov and Bartlett's test, respectively. Statistical significance was determined by applying either a one- or two-tailed unpaired t-test or paired Wilcoxon signed-rank test for parametric, and Welsh's t-test for nonparametric. For multiple independent groups, either two-/one-way analysis of variance (ANOVA) or the Mann-Whitney test was applied. Tests used to check for statistical differences are specified in the figure legends. ForTIC, ELDA software was used with Pearson's χ2-test. P value and Rho index were used to assess significance for correlations. For free-survival analysis, P log-rank tests were applied using GraphPad Prism. All other statistics were calculated with the Rand R-studio interface (https://www.r-project.org/). All experiments were reproduced in independent biological experiments at least three times, unless indicated.

### RESULTS

**ICB resistance emerges from MaSC-like states.** To understand breast cancer ICB resistance, we generated a preclinical immuno competent syngeneic model of anti-PD-L1 ICB resistance *in vivo.* Mouse breast cancer 4TO7 cells were orthotopically implanted into the mammary fat pad (MFP). Once tumors had reached a size of 0.5×0.5 cm², mice were treated with anti-PD-L1. After an initial transient response, all tumors developed resistance to anti-PD-L1 treatment (Fig. 1a). Anti-PD-L1 immunotherapy-resistant tumors (IRTs) were harvested in cell culture and their resistance was confirmed again in vivo.

Transcriptomic analysis of IRT cells revealed loss of APM among the most downregulated Gene Ontology (GO) pathways, followed by an expected loss of IFN signaling (Fig. 1b,c). Interestingly, among upregulated pathways we found enrichment of stem cell signatures (Fig. 1b) and, using gene set variation analysis (GSVA), we verified an important enrichment of CSC-like signatures associated with breast cancer aggressiveness, such as ES_1, NOS_Targets29 and breast_CSCs (Fig. 1d). Moreover, chromatin immunoprecipitation (ChIP)-enrichment analysis (CHEA) ranked stem cell transcription factors (SC-TFs) most enriched in IRT. We validated these transcriptomic findings by quantitative PCR with reverse transcription (RT-qPCR), showing that IRT cells are enriched for SC-TF genes Pou5f1 (Oct4), Sox2, Sox9 and Nanog and depleted in mammary differentiation factors- in particular, Lcor (Fig. 1e). Importantly, IRT cells also showed increase in the MaSC markers CD24+/CD29hi, a population that has also been reported in 4TO7 cells and others (Fig. 1f). This cellular selection by ICB treatment was not observed when we disrupted antigen presentation using β2M knockout (KO) cells, consistent with absence of selective immune-mediated killing. Of note, IRT cells maintained high PD-L1 expression, suggesting that they resisted anti-PD-L1 despite expressing the target. We propose that this resistance has emerged through downregulation of APM in CSCs, as suggested by the absence of CSC-like cell enrichment in β2M KO cells after treatment. Further characterization showed that IRT cells possess increased tumor sphere formation ability across sphere generations in vitro, despite no differences in cell cycle and with proliferation rates similar to the control condition, in both systems IRT and CD24hi/CD29hi CSCs. To evaluate their TIC frequency in vivo, a hallmark of CSCs, we performed orthotopic MFP injections in limiting dilution assays (LDAs) in immunodeficient NOD-SCID Gamma (NSG) mice. IRT cells were enriched >tenfold for TIC frequency (Fig. 1g), confirming that CSC properties are selected by ICB therapy.

Next, we performed in vitro cytotoxic T lymphocyte (CTL) assays by coculture of AT3-OVA cells and OT-I CD8 T cells, which specifically recognize the OVA peptide (SIINFEKL). After 3 days of coculture, surviving tumor cells that had evaded immune killing were enriched in CD24lo/CD44hi AT3 CSCs, particularly after anti-PD-L1 treatment (Fig. 1h). Accordingly, sorted AT3 and 4TO7 CSCs were resistant to immune-mediated cell killing compared to non-CSCs when cocultured with OT-I CD8 and JEDI CD8 T cells, respectively (Fig. 1i). AT3 CSCs induced less antitumor T cell activity than non-CSCs. We utilized the SORE6 CSC reporter system, which reports SOX2 and OCT4 activity, in AT3-OVA and Py8119-OVA cells. We first validated SORE6 fidelity in our models, showing that SORE6+ expressed SC-TFs and had >sixfold higher TIC frequency in vivo when injected into the MFP in LDAs. Similar to previous results, the residual surviving cell population in CTL assays was highly enriched in SORE6+ CSCs due to selective elimination of SORE6- cells, an effect further enhanced by anti-PD-L1 treatment. Overall, these results demonstrate the anti-PD-L1 ICB escape ability of CSC populations.

Despite the limitations in studying immune-specific interactions in human models, CD104hi/CD44hi CSCs isolated from MDA-MB-231 cells were more resistant to anti-PD-L1 when cocultured with human peripheral blood mononuclear cells (PBMCs) in an allogenic in vitro setting. Moreover, when immune-humanized mice with PBMCs were orthotopically injected with MDA-MB-231 cells, after 5 weeks tumors treated with anti-PD-L1 were enriched for the CD104hi/CD44hi CSC population.

**LCOR-low breast CSCs have reduced antigen-presenting ability.** Based on the finding that ICB-resistant cells have a stem cell-like phenotype and low APM expression (Fig. 1b,c), we analyzed available MaSC transcriptomic profiles. As expected, fetal mammary stem cells (fMaSCs) showed simultaneous downregulation of APM pathway genes, including immunoproteasome factors (PSMB8 and PSMB9), transporters (TAP1 and TAP2), β2M and MHC-I genes.

To study the APM pathway in human CSCs, we generated patient- derived organoids (PDOs) from clinical samples and isolated CD24lo/CD44hi CSCs37 from four different patients with TNBC (Fig. 2a). Our analysis confirmed that APM genes are downregulated in clinical CD24lo/CD44hi low-Lcor CSCs, by RT-qPCR analysis (Fig. 2a) and by flow cytometry of pan-HLAs-ABC and β2M (Fig. 2b). Additionally, three-dimensional (3D) PDO imaging with advanced light-sheet fluorescence microscopy (LSFM) also demonstrated the absence of pan-HLA staining in SORE6+ CSCs (Fig. 2c). In human mammary cell lines, HMLE CD24lo/CD44hi stem cells and MDA-MB-231 CD104hi/CD44hi CSC populations also showed downregulation of APM genes and Lcor compared to non-CSCs, as well as in ALDH+ populations. Accordingly, mouse 4TO7 CSCs and IRT cells also showed low Lcor and low APM. Next, we generated a fused LCOR- green fluorescent protein (GFP) knock-in in MDA-MB-231 cells reporting the endogenous protein levels and localization of LCOR. As expected, LCORlow MDA-MB-231 cells were enriched in OCT4+/SOX2+ CSCs (SORE6+). We then used fluorescence-activated cell sorting (FACS) to isolate cells based on GFP (LCOR-GFP) levels and measured the expression of APM genes in LCOR-/low and LCORhigh cells by RT-qPCR. APM gene expression progressively increased with increasing levels of LCOR (Fig. 2e). This was further validated by immunofluorescence (IF) of pan-HLA-ABC and LCOR-GFP (Fig. 2f). These results indicate reduced APM activity in human LCORlow CSCs.

To demonstrate impaired APM in CSCs, we isolated breast cancer cells based on APM activity using the OVA antigen-peptide presentation as readout. We ectopically expressed the full-length native chicken egg ovalbumin (OVA) in AT3 and Py8119 cells, which are H2-K1b haplotype cells established from mouse breast cancer C57BL/6J PyMT tumors44. Ectopic OVA is processed by the immunoproteasome generating the OVA257-264 (SIINFEKL) peptide, transported and presented in a MHC-I H2-K1b context. As we hypothesized, isolated OVA-/low cells expressed higher levels of CSC genes (Oct4, Sox2, Sox9 and Nanog) compared to OVAhigh cells as measured by RT-qPCR, and substantially lower levels of Lcor (Fig. 2h). Accordingly, the AT3 CSC population CD24lo/CD44hi is largely segregated in OVA-/low AT3 cells by flow cytometry (Fig. 2i) whereas CSCs are only partially segregated by Pd-l1.

Functional assays in both cell lines demonstrated increased tumor sphere formation and increased TIC capacity of OVA-/low cells in orthotopic MFP LDAs in immunodeficient NSG mice in vivo (Fig. 2j), reflecting an inherent tumor-initiation stem cell potential of OVA-/low cells.

Therefore, within tumor cell heterogeneity, LCOR-low CSCs have a defective APM system as an important immune-evasive property for ICB escape. LCOR regulates APM independently of interferon signaling. LCOR is among the top downregulated mammary differentiation factors in our IRT model, and LCOR-low CSCs are associated with low APM. Here, we show expression correlation of LCOR and APM components in estrogen receptor (ER)-negative breast cancer cell lines of the Cancer Cell Line Encyclopedia (CCLE) (Fig. 3a). Moreover, patient stratification of the TNBC METABRIC dataset based on LCOR levels demonstrates a strong correlation between LCOR expression and both the APM pathway (Kyoto Encyclopedia of Genes and Genomes (KEGG): M16004) (Fig. 3b) and IRS (Fig. 3c). These results suggest that LCOR may play a key role in APM regulation.

To investigate the mechanistic link of LCOR with the APM pathway, we used LCOR gain-and-loss modifications in MDA-MB-231 and HMLE cells. Remarkably, ectopic LCOR expression induced APM pathway genes while LCOR-KD reduced their expression in both cell types (Fig. 3d). Moreover, we measured the expression of different APM components via flow cytometry in these models. LCOR-KD cells phenocopy CSCs and display low APM as measured by the reduced proteasome activity reporter pQCXIN/ZsGreen, and reduced expression of transporters (TAP1) and presenting molecules (β2M and HLA-ABC) (Fig. 3e-g). The inverse results were obtained with LCOR-OE boosting antigen processing and presentation (Fig. 3d-g).

Surprisingly, these effects were not dependent on IFN stimulation. Despite IFN-y treatment enhancing the effects of LCOR on APM components, ruxolitinib-an inhibitor of JAK1/ JAK2-mediated activation of STATs/IRFs-did not affect the ability of LCOR to induce different APM components in vitro (Fig. 3e-g). Importantly, the effects of IFN-y treatment were abrogated in LCOR-KD, suggesting that LCOR levels modulate sensitivity to IFN and its impact on the APM pathway (Fig. 3e-g). To validate global APM activity, murine AT3-OVA and Py8119-OVA Lcor-KD cells showed reduced OVA presentation while Lcor-OE cells had high presentation in all three conditions, thus demonstrating the essential role of LCOR in modulating and priming APM activity (Fig. 3h). Again, IFN-y treatment was unable to increase OVA presentation in Lcor-KD cells. Overall, our data demonstrate that LCOR levels determine APM activity in tumor cells with and without IFN signals, highlighting a dominant role of LCOR in antigen presentation.

**LCOR directly regulates APM factors through ISRE binding.** To understand how LCOR regulates the APM pathway, we transduced MDA-MB-231 cells with ectopic expression of LCOR and LCOR mutant forms, including a double-point mutation in the nuclear receptor (NR) binding domain (LSKLL to LSKAA) preventing binding to NRs and deletion of the HTH DNA-binding domain (ΔHTH), blocking its putative binding to DNA. We verified the correct overexpression and nuclear localization of each variant. Next, we performed chromatin immunoprecipitation sequencing (ChIP-seq) analysis. The highest cluster of peaks genome wide was found on the short arm of chromosome 6 (chr6) for the wild-type and LSKAA forms of LCOR, but not for the ΔHTH mutant (Fig. 4a). Importantly, this genomic region of approximately 8 Mb (chr6: 27,810,120-35,980,577) contains the MHC-I cluster and all APM genes (Fig. 4a,b), except β2M located on chr15. Consistently, gene expression analysis in MDA-MB-231 cells showed that the wild-type and LSKAA forms of LCOR, but not the ΔHTH mutant, induce APM genes (Fig. 4a,b). Of note, LCOR peaks were located in gene regulatory elements (GREs) of these genes (Fig. 4c), including β2M, suggesting direct master regulation of the APM cluster, also validated by endogenous LCOR ChIP-qPCR using an LCOR-HA knock-in system. Accordingly, ChlP-seq peak enrichment analysis ranked the APM pathway as the most highly enriched pathway among BioCarta biological processes (Fig. 4d). Interestingly, by analysis of available assay for transposase-accessible chromatin using sequencing (ATAC-seq) data, we observed that this region is shut down in fMaSCs, reflecting a conserved gene regulatory mechanism of mammary cell immunogenicity. Therefore, we investigated the evolutionary conservation of LCOR in other species and found that it is highly conserved in all vertebrates, especially the NR and HTH domains, the latter being the only domain preserved beyond vertebrates. Therefore, it is an ancient DNA-binding domain originating in prokaryotes as a transcription factor domain. These findings support a conserved role of LCOR in coordination of APM transcriptional regulation through its HTH domain.

Our transcriptomic and conservation analyses suggest that LCOR can act as a transcriptional activator. However, LCOR has previously been described as a transcriptional corepressor and its function as a possible transcriptional activator had not yet been proven. Performing motif discovery analysis of LCOR ChlP-seq peaks using HOMER software revealed ISREs among the top-ranked prediction motifs-in particular, the interferon-regulatory factor 1 (IRF1) binding site (Fig. 4e), typical of interferon-stimulated genes and APM genes in antiviral responses. To demonstrate that LCOR can activate APM genes, we generated a promoter-reporter with six tandem repeats of the ISRE motif (6 × CAGTTTCACTTTCCC) upstream of a minimal CMV promoter, and also a mutated version (6 × CAGTGGCACGGTCCC) to discern specific LCOR binding (Fig. 4f). Only LCOR and LSKAA, but not ΔHTH, increased reporter activity as quantified by red fluorescent protein (RFP) flow cytometry in MDA-MB-231 cells (Fig. 4g). Remarkably, no induction was detected when the ISRE sequence was mutated (Fig. 4g), showing that LCOR binds to ISREs and activates transcription. Next, we combined the ISRE reporter with the LCOR-GFP knock-in, generating a unique flow cytometry system to study ISRE activity dependent on endogenous LCOR regulation. We explored the effects of different conditions, including IFN-y treatment and different IFN inhibitors-ruxolitinib (inhibitor of JAK1/JAK2) and BX-795 (TBK1 inhibitor blocking STING signaling)-to further extricate the interference by IRFs and LCOR on reporter activity. We observed that IFN-y treatment increased ISRE in LCOR+ cells (medium and high), but not in LCOR- cells. Moreover, ruxolitinib inhibited ISRE activity only in LCOR- cells but not in LCOR+ cells, demonstrating that LCOR induction of ISRE is independent of IFN and essential for the transcriptional activation of ISRE-controlled genes (Fig. 4h). Accordingly, these treatments did not suppress ISRE induction in LCOR-OE cells and IFN-y failed to induce ISRE in LCOR-KD cells. This is consistent with the independent induction of APM activity (Fig. 3e-h).

We further confirmed the transcriptional activity of LCOR by performing RNA polymerase II subunit B (POLR2B) ChIP. POLR2B bound MHC-I genes only in the presence of LCOR but not in LCOR-KD cells. Moreover, POLR2B is enriched with either LCOR-OE or LSKKA-OE but not with the ΔHTH mutant, indicating the requirement of LCOR binding at MHC-I gene promoters. Overall, these results reveal that LCOR is a transcriptional activator of the APM pathway.

### LCOR facilitates tumor-immune infiltration and killing

Next, we tested the effects of LCOR-mediated APM induction on tumor immunity. In vitro CTL assays of AT3-OVA and Py8119-OVA cells cocultured with CD8+ OT-I T cells, and of 4TO7-EGFP cocultured with CD8+ JEDI T cells, showed increased immune killing of Lcor-OE cells in both systems (Fig. 5a,b). Knockdown and knockout of β2m in Lcor-OE cells rescued survival and evasion of T cell killing, demonstrating that this effect is dependent on antigen presentation capacity (Fig. 5c). In addition, Lcor-KD cells avoided immune-mediated cell killing (Fig. 5d), consistent with their reduced OVA presentation ability (Fig. 3h). Lcor-OE cells increased T cell activation as measured by CD69, while Lcor-KD reduced it (Fig. 5e). In tumor growth experiments, 4TO7 Lcor-OE cells showed higher Lcor-mediated reduction in immunocompetent mice (Balb/c) than in immunodeficient NSG mice, indicating an elicited immune reaction due to LCOR-mediated immunogenicity. The inverse effect was true for Lcor-KD tumors. 4TO7 Lcor-OE tumors showed a substantial increase in CD4 and CD8 infiltrating lymphocytes compared to control tumors as measured by immunohistochemistry (IHC) and flow cytometry (Fig. 5f,g), and a nonsignificant increase in CD45+ leukocytes (Fig. 5f). The CD45+/CD3- compartment showed no significant changes in dendritic cells (CD45+/CD3-/CD11c+/F4/80-) and macrophages (CD45+/CD3-/CD11b+/F4/80+). These results support a lymphocytic immunoreactive response to Lcor-OE tumors due to their high immunogenicity.

We performed deconvolution xCell50 analysis of the METABRIC dataset to estimate the immune content of 186 samples from patients with TNBC51. We generated immunophenotype clusters representative of the immune landscape: cluster 1, low-immune-infiltrated tumors; cluster 2, immunosuppressive populations; cluster 3, cytotoxic populations; and cluster4, highly cytotoxic populations. Next, we analyzed LCOR levels in these different clusters and found higher expression in the cytotoxic clusters (Fig. 5h,i). LCORhigh tumors were also more enriched for CD4, CD8 and γδ T lymphocyte gene signatures. Overall, these results support the premise that LCOR promotes immunogenicity and adaptive immune infiltration in TNBC, mediating antitumor immunity.

### LCOR levels and ICB responsiveness in patients with TNBC

We performed LCOR IHC analysis on a small set of matched clinical TNBC samples before and after ICB treatment in the neoadjuvant setting. Two patients (P1 and P2) were treated with anti-PD-L1 atezolizumab plus polychemotherapy (abraxane and carboplatin) while two others (P3 and P4) were treated with anti-PD1 nivolumab plus SYK/FLT3 inhibitor. In all cases, LCOR expression was lower in residual disease (Fig. 6a). These data support the supposition that LCOR cells are eliminated by combinatorial neoadjuvant ICB therapy.

We also explored the value of LCOR in ICB response in larger clinical trials. Analysis of the TONIC trial, which included 53 metastatic TNBC patient samples pretreatment40, showed higher levels of LCOR in responders (Fig. 6b). Additionally, data from the phase-II I-SPY2 trial, using durvalumab, olaparib and neoadjuvant paclitaxel in patients with TNBC52, also showed higher levels of LCOR in responders (Fig. 6b). These findings demonstrate that LCOR levels are associated with response to ICB-containing combination therapy in TNBC. To assess whether these observations also apply in other cancer types, we examined a melanoma cohort treated with single-agent anti-PD-1, showing higher levels of LCOR in responders. Moreover, our breast IRS overlaps with a melanoma ICB resistance signature53, and patients with LCORhigh TNBC inversely correlated with the latter signature. Overall, these results support the association of LCOR with ICB clinical benefit.

### LCOR overcomes resistance to ICB leading to tumor eradication

Antigen presentation and PD-L1 are hallmarks of anti-PD-1/PD-L1 therapy responses. Lcor slightly increased Pd-I1 expression in 4TO7 and AT3 cells, and showed positive correlation in patients with TNBC. This may be explained by LCOR priming IFN sensitivity, which can induce PD-L1 (refs. 3,5). Remarkably, the positive levels of PD-L1 in combination with the potent LCOR induction of APM sets an ideal tumor configuration for anti-PD-1/PD-L1 therapy. Therefore, we performed orthotopic MFP transplantation of syngeneic 4TO7 cells in immunocompetent mice, allowed tumors to reach a size of 0.5 × 0.5 cm2, then initiated ICB therapy with anti-PD-L1 once per week. Control-nontreated and control-treated tumors continued growing; however, Lcor-OE tumors treated with anti-PD-L1 ICB totally regressed, with complete response (CR) by 20 days in all mice (Fig. 6d,e). Importantly, depletion of the CD4/CD8 compartment led to no response to ICB, validating that the observed CR to anti-PD-L1 in Lcor-OE tumors was mediated by the adaptive immune system. We performed up to five independent experiments and observed CR in 49 out of 50 Lcor-OE tumors (Fig. 6f). Of note, the only Lcor-OE tumor that did not respond had lost the ectopic expression of Lcor. All 49 mammary glands with CR were tumor free after 2 months of anti-PD-L1 discontinuation. We followed up 15 of these mice for 1 year, and in none had tumors recurred and all had tumor-free glands, suggesting that we had irreversibly eradicated the tumors and cured these mice (Fig. 6f,g). Accordingly, 4TO7 Lcor-KD tumors demonstrated higher resistance to anti-PD-L1 compared to control 4TO7 cells (Fig. 6h). The AT3 syngeneic model in C57BL/6J mice also confirmed the CR of Lcor-OE tumors to ICB therapy, both in vivo and in vitro.

To study the dominant role of LCOR over IFN signaling in vivo, we performed a MFP experiment comparing the effects of Lcor-OE and the IFN type-I inducer Poly (I:C)54. The combination of Poly (I:C) treatment and anti-PD-L1 did not reach the efficiency shown by the Lcor + anti-PD-L1 condition, again demonstrating the dominant role of LCOR in ICB therapy beyond IFN-mediated effects.

Next, we conducted preclinical lung metastasis assays using 4TO7 tail vein (TV) administration and allowed for the establishment and growth of lung metastases before starting anti-PD-L1 therapy (Fig. 6i). To achieve synchronous metastases among the different conditions, we injected threefold more Lcor-OE cells due to their inherent reduced tumorigenicity. 4TO7 Lcor-OE metastasis were cured after 4 weeks of anti-PD-L1 treatment in five out of six mice, while all control metastatic tumors progressed despite anti-PD-L1 exposure (Fig. 6i). Overall, these preclinical assays demonstrate a conclusive curative response of anti-PD-L1 treatment mediated by the effects of LCOR on tumor cell immunogenicity, which represents a promising therapeutic target for early and advanced TNBC.

### mRNA-based LCOR therapy in combination with ICB in vivo

Therapeutic mRNA delivery using nanoparticles has enabled the rapid development of highly effective vaccines against COVID-19 and may similarly revolutionize cancer therapy. Based on current knowledge on extracellular vesicles (EVs) and mRNA delivery, we designed a proof-of-concept approach to restore Lcor expression through the introduction of Lcor mRNA into tumor cells, and to combine this treatment with ICB. We ectopically expressed Lcor tagged with HA in HEK293T cells, which produce large quantities of EVs containing ectopic Lcor-HA mRNA transcripts (Fig. 7a). In vitro treatment of 4TO7 cells with Lcor-HA mRNA EVs showed incorporation and translation of the Lcor protein as detected by immunoblot using anti-HA (Fig. 7b), and corrected nuclear localization (Fig. 7c). Lcor EVs reduced the CSC population and upregulated APM genes in 4TO7 cells, increasing CD8+ T cell-mediated killing. Next, we designed a preclinical lung metastasis assay to test Lcor mRNA therapy. After 5 days of EV administration, lung metastases were already showing incorporation and translation of Lcor-HA protein in most tumor cells (Fig. 7d) as proof of principle in vivo. Next, mice serially treated with the combination of EV-based Lcor mRNA therapy and anti-PD-L1 showed significantly longer survival and complete elimination of lung metastasis compared with the EV-control and anti-PD-L1 therapy (Fig. 7e-g). These results suggest that LCOR mRNA therapy is a potential therapeutic partner of ICB therapy.

### LCOR function in ER+BC

Co-culture assays of MCF7 cells, which are estrogen receptor possitive breast cancer cells (ER+BC) with ectopic expression of LCOR does not show a very efficient killing effect mediated by the immune cells. In this case, using an allogenic system with human peripheral blood mononuclear cells (PBMCs) during 5 days with/without anti-PD-L1 (Atezolizumab) (Fig.8). Instead, the LSKAA ectopic overexpression, which lacks the ability to interact with ER (and other NRs), is more efficient and we observed a strong killing of this MCF7-LSKAA tumor cells mediated by the immune system. This result mimics LCOR in TNBC tumors in which ER is absent and thus LCOR is not interfered by the interaction with ER to mediate APM gene expression. These results show functional evidence of a much better efficiency of LCOR fragments that cannot interact with NR in terms of inducing tumor cell immunogenicity.

### LCOR and mutants/fragments of LCOR inducing AMP in different breast cancer subtypes

Using MCF7 (ER+) transduced with LCOR and the mutant LSKAA we measured APM gene expression by qPCR. APM gene expression induction is only observed when using LSKAA (Fig. 9). Moreover, LSKAA is able to induce APM in MCF7 to similar levels as LCOR in MDA-MB-231 cells (ER-). These results clearly show how LSKAA is more efficient increasing APM gene expression by avoiding NR interactions.

Intriguingly, in order to explore specific LCOR binding in APM gene regulatory elements (promoters, enhancers,...) as shown performed in MDA-MB-231 (Fig. 4), the performed ChIP assays clearly show how LCOR direct binding and regulation of AMP is not occurring in MCF7 (ER+) cells. However, LSKAA by disrupting its interaction with NRs can directly bind and regulate APM genes (Fig. 10). These results further reinforce the specificity of the mechanism and the strong efficiency of LSKAA inducing APM gene expression.

Flow cytometry analysis show how LCOR can consistently induce HLA genes in ER-negative BC such as MDA-MB-231 (Fig. 3), however it cannot really potentiate a strong induction in ER+BC cells. Instead LSKAA can strongly increase MHC-I molecules (HLAs and b2M) measured by flow cytometry (Fig.11). Importantly, LSKAA also substantially increment the presence of HLA molecules in MDA-MB-231 cells when compared to LCOR wild type (Fig. 11). Indicating that despite LCOR can induce a strong APM response in ER⁻BC, LSKAA is even more efficient to do so.

Therefore, additional shorter versions of LCOR, here referred as mutants or fragments, were generated to be tested functionally (Fig. 12). Results clearly show how shorter versions of the protein are still active inducing APM genes, such as HLAs-ABC, measured by flow cytometry indicating the minimal functional element is the HTH domain. The delta-HTH (ΔHTH) fragment is not capable inducing HLAs, however the rest of fragment induce HLAs in MDA-MB-231 cells (Fig. 13). Importantly, the most efficient fragments are the longest fragments without full NR, the LSKAA and the delta-NR fragments (Fig. 12-13). In order to predict the structure and stability of the LCOR fragments, we used AlphaFold v3 to model the three-dimensional shapes. The results show that the LSKAA mutation does not alter the overall structure of LCOR but specifically disrupts the alpha-helix of the nuclear receptor binding domain suggesting binding disruption. On the other hand, the deletion of the first 64 amino acids does not significantly affect the folding of the rest of LCOR protein (Fig. 14), which remains functional.

LCOR and LSKAA efficiency was also tested in vivo using MCF7 cells implanted ortothopically in NSG mice with and without humanized immune cells by administrating human PBMCs. Interestingly, LCOR did not show potent tumor suppressions in either conditions (with/out PBMCs), however LSKAA showed a potent tumor suppression in NSG only with the PBMC administration indicating how LSKAA can induce tumor immunogenicity in MCF7 cells (ER+/PGR+/AR+) (Fig. 15). In addition, patient-derived organoids (PDO) from ER+BC tumor tissue also showed the potent effect of LSKAA promoting immune infiltration in the core of the PDOs when human PBMCs were co-cultured (Fig. 16). These results show how LSKAA can mediate strong tumor immunogenicity in ER+BC, to a similar or greater magnitude of LCOR in TNBC.

### Example 2.

### Materials and methods and results of figures 14 to 27.

### METHODS:

### Viral production and transduction of cell lines

For viral production, HEK293T cells were seeded at 7-8 × 10^6 cells per 10 cm dish 18-24 hours prior to transfection, aiming for 70-90% confluence at the time of transfection. Then, plasmids were mixed in Opti-MEM: 1 µg of VSVG, 2.5 µg of pCMV-dR8.91, and 4 µg of the lentiviral vector (in this case pLEX construct). The DNA and PEI solutions were combined and incubated at room temperature for 30 minutes and then added to the HEK293T cells preconditioned with Opti-MEM. After incubating for 4-6 hours at 37°C, the transfection media were replaced with fresh DMEM. Viral supernatants were collected daily from day 2 to day 3 post-transfection, filtered through a 0.45 µm filter, and stored at 4°C for short-term use or -80°C for long-term storage. For cell infection, 0.5-1 mL of virus-containing medium was mixed with fresh medium and polybrene (final concentration of 8 µg/mL) and added to the target cells. Cells were expanded for further assays, such as antibiotic selection or sorting, two days post-infection to allow sufficient time for viral integration.

### Molecular cloning and plasmids

LCOR-overexpression plasmids (controls pLEX-HA and LCOR-ΔHTH-HA; human LCOR-HA, LCOR-LSKAA-HA; and an OVA-overexpressing plasmid (pLEX-OVA-IRES-mCherry) were obtained from the laboratory of Y. Kang. pLEX- mouse Lcor-HA and pLEX-ΔNR-HA, pLEX-HTH-HA were generated by PCR amplification of mouse Lcor complementary DNA and the addition of HA at 25 the 3' end, inserted together into pLEX family plasmid after digestion with Spel and Agel (NEB). pLEX-OVA-IRES-mCherry is a lentiviral expression plasmid that contains the sequence of the ovoalbunim, followed for an internal ribosome entry site (IRES) followed by the expression of the fluorescent protein mCherry. The SIINFEKL peptide is a prominent antigenic epitope derived from the ovalbumin (OVA) protein, commonly used in immunological studies involving C57BL/6 mice or cells derived from these mice. SIINFEKL (OVA 257-264) peptide is presented by the H-2Kb molecule, a class I major histocompatibility complex (MHC). This presentation enables the activation of engineered cytotoxic CD8+ T cells, which specifically recognize SIINFEKL in the context of H-2Kb. As a result, SIINFEKL is a widely utilized model antigen for studyingT cell responses, antigen processing, and the development of immune-based therapies in C57BL/6 mice, a strain extensively used in immunology research.

### Animal studies

Mouse strains Balb/c, C57BL/6J, C57BL/6-Tg (TcraTcrb)1100Mjb/J (OT-1), and NOD.Cg-Prkdcscid lI2rgtm1Wjl/SzJ (NSG) were used. Mice were orthotopically injected into the mammary fat pad with from 5,000 to 20,000 4TO7 cells resuspended in 1:1 PBS:Matrigel (Corning). Tumor volume was measured twice weekly with digital calipers, and calculations were applied (π × length × width2/6). Mice were intratumorally treated with 5 µg of Lcor-mRNA-polymeric nanoparticles or control mRNA twice a week. Lyophilized mRNA-RH mixture was stored at -20ºC, and were resuspended in nuclease free water at a final concentration of 0.5 mg·mL-1. In immune-checkpoint blockade (ICB) assays, mice were intraperitoneally treated with anti-mouse anti-CTLA-4 (BioXCell, clone 9D9, catalog no. BE0164) at 0.5 mg·kg-1 versus vehicle once a week. Treatments were initiated when tumor size reached 0.5 × 0.5 cm.

### Lcor mRNA-polymeric nanoparticle administration

Tumors located in the mammary fat pad were palpated to identify the injection site and skin were cleaned with an antiseptic solution. 27G needle was injected directly into the tumor mass, ensuring that the needle is positioned properly within the tumor, the mRNA-nanoparticle mixture was slowly injected into the tumor, ensuring minimal backflow or leakage. Finally, the needle was carefully withdrawn and gentle pressure with sterile gauze to the injection site was applied when necessary.

### Cell lines, culture conditions and treatments

All cell lines used in this study-breast cancer cell lines (human MDA-MB-231, MCF7 and mouse 4TO7 and AT3)-were obtained from Y. Kang at Princeton University and cultured according to the American Type Culture Collection (ATCC). UM-UC-3, A549, PANC1, and PC3 cells were obtained from P. Navarro at HMRIB. HEK293T cells were obtained from ATCC. Cells were routinely checked for Mycoplasma and all were negative. For this study, anti-mouse PD-1 (BioXCell, clone RMP1-14, catalog no. BE0146) was used in vitro at 10 ng·ml-1 for 72 h.

### Cytotoxic T Lymphocyte (CTL) assay

For mouse cell coculture assays, splenocytes from OT-I mouse models were obtained. Spleens were mechanically minced, and single cells were incubated with ACK buffer (Fisher, catalog no. A1049201). The single-cell suspension was strained over a 70-µM filter and CD8+ T cells were purified using the CD8a+ T cell Isolation kit (Milteny, catalog no. 130-104-075) for negative selection, then cocultured with AT3-OVA tumor cells for 72 h. Cell viability was determined after 72 h by the crystal violet method. PBMCs were isolated by gradual centrifugation of blood samples from healthy adult donors from the Tissue Bank of Catalonia, and cocultured with tumor cells in coculture medium. Cell viability was determined after 72 h by the crystal violet method.

### Flow cytometry analysis

Flow cytometry analysis data were collected on either a Fortessa Flow Cytometer (BD) or with LSRII Flow cytometry (BD) using FACSDiva v.9.0 software, and analyzed by FlowJo software 10 v.10.8.1 (FlowJo). Cells were strained through a 70-µM filter, counted and diluted in PBS + 10% FBS buffer before proceeding with a general staining protocol using anti-SIINFEKL (OVA), anti-pan-HLA-ABC or anti-human β2M antibodies (diluted at 2 µg·mL-1 and 2.5 µg·mL-1, respectively, in PBS + 10% FBS buffer) for 20 min at 4ºC in dark conditions.

### RT-qPCR analysis

Total mRNA was purified using the RNeasy Mini Kit (Qiagen) and reverse transcribed into cDNA using the High-Capacity cDNA Reverse Transcription Kit (Life Technologies). RT-qPCR was performed using LightCycler 480 SYBR Green I Master (Merck). Data were collected using QuantStudio 12 K Flex software. mRNA expression was normalized by the expression of GAPDH.

### Bioinformatic domains prediction

Comprehensive bioinformatic prediction of various domains, interaction sites, secondary structures, and disordered regions within the LCOR protein were done utilizing http://elm.eu.org/ computational tools, LCOR's structural features have been elucidated, providing insights into its functional properties and regulatory mechanisms.

### Production, purification, and labelling of rHTH

The recombinant HTH (rHTH) peptide with the following sequence MPRKKRGRYRQYNSEILEEAISVVMSGKMSVSKAQSIYGIPHSTLEYKVKERLGTLKNPPKKKMKLMRSE GPDVSVKIELDPQGEAAQSANESKNECHHHHHH was synthetized, codon optimized for expression in E.coli, cloned in a pET30a expression vector (GenScript), and purified from BL21 (DE3) Arabinose-Inducible (Invitrogen) bacterial strain. rHTH were purified from supernatant of cell lysate induced for 16h at 15°C. Identity was confirmed by SDS-PAGE electrophoresis and western blot. Maleimide conjugation with AlexaFluor488 (Thermo Scientific; catalog number 46602) moieties to the C-terminal artificial cysteine residue of rHTH was performed according to the manufacturers' indications. The covalently modified peptides were purified from the free labelling agent using dye removal spin columns (Invitrogen; catalog number 22858).

For flow cytometry analysis, cells were treated with the indicated concentration of rHTH-488 for 15 minutes in serum-free culture medium at 37ºC or 4ºC (4ºC were used in order to inhibit all active uptake pathways), washed twice with PBS, trypsinized with 0.25% trypsin-EDTA (Gibco) for 30 minutes to remove membrane-bound mini-protein, resuspended in PBS pH 7.0, and immediately analyzed.

### Western blot analysis

Protein extracts were run on 10% gels, transferred to PVDF membranes (Millipore), and incubated with antibodies against His-TAG (1:500; Invitrogen, #MA1-135-HRP).

### Proximity ligation assay (PLA)

Cells were seeded in coverslips and fixed with 4% PFS for 20' PLA was followed according to manufacturer's instructions. In brief, cells were blocked and incubated with rabbit and mouse primary antibodies at 4ºC o/n. Samples were then incubated with rabbit and mouse probe followed to enzymatic reactions. Cells were washed with TBE and mounted with DAPI mounting solution. Images were acquired using an upright Nikon Eclipse Ni-E fluorescence microscope (Nikon). Data was processed and quantified using Fiji software.

### RESULTS

### mRNA-based LCOR therapy in vivo with or without ICB

We performed Lcor mRNA therapy using poly(beta aminoesters) (pBAE) nanoparticle. The intratumoral administration of 5ug of LCOR mRNA with nanoparticles significantly reduced tumor growth in 4TO7 tumors, a triple-negative breast cancer cell line. After 2 weeks of treatment, we observed a remarkable decrease in tumor growth (Figure 17). In addition, we expect a much stronger effect of LCOR mRNA in combination with immunotherapy compared to control groups reaching a complete remission of all tumors. These results suggest that Lcor mRNA therapy is a suitable therapeutic strategy as demonstrated in our preclinical models in vivo.

In addition, the combination of intratumoral administration of 5 µg of Lcor mRNA therapy and 0.5 mg·kg-1 of intraperitoneal CTLA-4 immune checkpoint blockade (ICB) significantly improved outcomes in mice bearing 4TO7 triple-negative breast tumors (Figure 18). Mice treated with this combination showed a significant extended life expectancy compared to those receiving either treatment alone (Figure 18B). The complete response rate increased from 50% with CTLA4 therapy alone to 100% when combined with Lcor mRNA therapy (Figure 18C). These findings highlight the enhanced therapeutic potential of the combination of Lcor and anti-CTLA4 ICB in treating aggressive tumors.

On the other hand, cytotoxic lymphocyte (CTL) assays conducted with AT3-OVA cells after 72 hours of coculture with OT-1 CD8+ T cells and anti-PD-1 treatment revealed that ectopic overexpression of Lcor synergizes with PD-1 blockade in vitro, showing a significant enhancement in CTL activity against AT3-OVA cells (Figure 19). This increased effect underscores the potential for combining Lcor overexpression with PD-1 inhibitors to improve therapeutic efficacy.

Lcor enhances tumor clearance in adoptive cell transfer (ACT) therapy AT3-OVA Lcor-overexpressing cells were implanted in the mammary fat pad of immunodeficient NSG mice. Once tumor reaches 0.5x0.5 cm, isolated CD8+ T cells from OT-I mice were adoptively administered intravenously as therapy. The results obtained showed a strong increase of CD8 T-cell-mediated anti-tumoral response in Lcor-OE tumors. These results demonstrate that Lcor makes tumor cell much more vulnerable to T cell therapy leading to tumor eradication (Figure 20). Even though ACT therapy alone has a modest effect, the presence of Lcor clearly improves the effectiveness of engineered cell therapy.

### LCOR and LCOR mutants induces APM in different types of cancers

Nuclear receptors are transcription factors normally activated by ligands responding to steroids, thyroid hormones, vitamins and other molecules. These intracellular receptors regulate key functions in reproduction, development, and physiology. In order to explore the effectiveness of LCOR or LCOR mutants in several cancer types, we transduced LCOR and the LSKAA mutant and measured APM gene expression by qPCR in different cancer type cell lines, including NR+ and NR- cell types. We know thar NR can interfere LCOR activity and block the LCOR-mediated APM induction. These results show how LSKAA is able to induce APM independently to NR+ cell lines, to similar levels as LCOR wild-type in NR- cells (Figure 21A). These results clearly show how LSKAA is more efficient increasing APM gene expression by avoiding NR interactions. Moreover, flow cytometry analysis shows how LSKAA can consistently induce HLA-class-I genes in NR-negative and NR-positive cancer cell lines (Figure 21B). Importantly, LSKAA can strongly increase MHC-I molecules (HLAs) measured by flow cytometry. Indicating that despite LCOR can induce a strong APM response in several cancer types, LSKAA is even more efficient to do so.

Therefore, additional shorter mutant versions (fragments) of LCOR were generated for functional testing. The fragment ΔNR has a deletion of the first 64 amino acids from the N-terminal domain. The fragment HTH contains from the 339 to the 433 amino acids from the original LCOR sequence (Figure 22C). The results demonstrate that these shorter mini-protein variants remain active across various breast cancer subtypes independently of the NR status (such as estrogen and progesterone receptor), as evidenced by the induction of APM genes measured by RT-qPCR (Figure 22A-B). Flow cytometry analysis of several cancer type cell lines, including triple negative (MDA-MB-231) and estrogen receptor (ER) positive (MCF7) breast cancer, lung cancer (A549), bladder cancer (UM-UC-3), pancreatic cancer (PANC1), and prostate cancer (PC3) reveals increased levels of HLAs-ABC and β2M in all the cases, indicating that the minimal functional element is the so called HTH fragment. While the empty vector fails to induce HLAs, the LCOR shorter fragments effectively increased AMP expression in several cell lines (Figure 23A-B), also shown by SIINFEKL flow cytometry analysis (specific OVA peptide presented by the MHCl molecule in the AT3 cell line). Notably, in CTL immune killing co-cultures ΔNR and HTH fragments (figure 23D) increased tumor killing, and it was increased in the shorted fragment version called HTH, only including the NLS domain, the DNA-binding domain (helix-turn-helix domain; HTH) and the c-terminus disordered region. In preclinical settings in vivo, when ΔNR and HTH fragments were ectopically expressed in 4TO7 cells, strongly decreased the tumor growth in vivo in immunocompetent mice (Figure 24A-B). These results suggest that ΔNR and HTH fragments can mediate strong tumor APM and immunogenicity that favour anti-tumor immune responses and immunotherapy with higher efficiency than the rest of the fragments. Therefore, these fragments should be also considered for therapeutic applications.

### Recombinant HTH

Considering that both ΔNR and HTH fragments are able to inhibit tumor growth similarly to the original LCOR and given the presence of large unstructured regions in LCOR and ΔNR that could dampen the stability of the protein, we decided to test whether fragment HTH, which is more structured due to the HTH domain and performed the function previously observed in ectopic expression assays by its own (naked protein). Synthesized recombinant HTH miniportein was also tagged with a fluorescent probe using a maleimide reaction. This approach allowed us to covalently bind the fluorescent probe to a cysteine residue that we added to the protein. The incorporation of this fluorescent tag facilitated testing protein internalization and accumulation within the cells. Strikingly, rHTH treatment in vitro showed efficient uptake and accumulation of the tagged mini-protein through flow cytometry (Figure 25A) and western blot analysis (Figure 25B) in in the MDA-MB-231 cells, providing clear evidence of its cellular penetration. This is remarkable, since it indicates that the naked rHTH miniprotein has cell penetrating capacity and also nuclear penetration for proper localization and function.

Importantly, we found that rHTH significantly increase HLAs in the MDA-MB-231 cell line, as measured by flow cytometry (Figure 25C). Additionally, it enhanced the presentation of the OVA peptide by AT3-OVA cells compared to controls treated with bovine serum albumin (Figure 25D). These results indicate that the recombinant HTH mini-protein can mediate the expected LCOR functions in APM induction, suggesting its potential to improve tumor immunogenicity and immune recognition in these cellular models. These are remarkable findings that open high potential therapeutic application for the rHTH as a standalone or naked agent, administered parenterally in humans for cancer treatment.

### CLAUSES

1. An isolated or synthesized Ligand-dependent corepressor (LCOR) or a mutant or fragment thereof, selected from any one of the list consisting of:
   a. a fragment of the Ligand-dependent corepressor (LCOR) comprising, consisting of or consisting essentially of SEQ ID NO 23 (MPRKKRGRYRQYNSEILEEAISVVMSGKMSVSKAQSIYGIPHSTLEYKVKERLGTLKNP PKKKMKLMRSEGPDVSVKIELDPQGEAAQSANESKNE) or a sequence which is at least 90%, 91%, 92%, 93%, 94%, 95%, 96%, 97%, 98%, or 100% identical to SEQ ID NO 23;
   b. a mutant or fragment of a Ligand-dependent corepressor (LCOR) or a polynucleotide sequence coding for the said mutant or fragment of a Ligand-dependent corepressor (LCOR), wherein the mutant or fragment of a Ligand-dependent corepressor (LCOR) is characterized by comprising amino acid sequence SEQ ID NO 3, or a sequence which is at least 90%, 91%, 92%, 93%, 94%, 95%, 96%, 97%, 98%, or 100% identical to SEQ ID NO 3, and at least one NLS motif (nuclear localization signal) of amino acid sequence SEQ ID NO 4 or a sequence which is at least 90%, 91%, 92%, 93%, 94%, 95%, 96%, 97%, 98%, or 100% identical to SEQ ID NO 4; and wherein the mutant or fragment of a Ligand-dependent corepressor (LCOR) is further characterized by the absence or disruption/inactivation of the Nuclear Receptor Binding Domain; and
   c. a Ligand-dependent corepressor (LCOR) or a polynucleotide sequence coding for the said Ligand-dependent corepressor (LCOR), wherein the LCOR is of SEQ ID NO 1 or a sequence which is at least 90%, 91%, 92%, 93%, 94%, 95%, 96%, 97%, 98%, or 100% identical to SEQ ID NO 1.
2. The isolated or synthesized Ligand-dependent corepressor (LCOR) or a mutant or fragment thereof according to clause 1, wherein said LCOR is the fragment of a Ligand-dependent corepressor (LCOR) consisting essentially of or consisting of SEQ ID NO 23 (MPRKKRGRYRQYNSEILEEAISVVMSGKMSVSKAQSIYGIPHSTLEYKVKERLGTLKNPPKKKM KLMRSEGPDVSVKIELDPQGEAAQSANESKNEC) or a sequence which is at least 90%, 91%, 92%, 93%, 94%, 95%, 96%, 97%, 98%, or 100% identical to SEQ ID NO 23, wherein preferably said LCOR mutant or fragment is a recombinant protein.
3. The isolated or synthesized Ligand-dependent corepressor (LCOR) or a mutant or fragment thereof according to clause 1, wherein said LCOR is a mutant or fragment of a Ligand-dependent corepressor (LCOR) as defined in claim 1 b).
4. The mutant or fragment of a Ligand-dependent corepressor (LCOR) according to clause 3, or a polynucleotide sequence coding for the said mutant or fragment of a Ligand-dependent corepressor (LCOR), wherein the mutant or fragment of a Ligand-dependent corepressor (LCOR) is characterized by consisting of or consisting essentially of amino acid sequence SEQ ID NO 5, or a sequence which is at least 90%, 91%, 92%, 93%, 94%, 95%, 96%, 97%, 98%, or 100% identical to SEQ ID NO 5.
5. The mutant or fragment of a Ligand-dependent corepressor (LCOR) according to clause 3, or a polynucleotide sequence coding for the said mutant or fragment of a Ligand-dependent corepressor (LCOR), wherein the mutant or fragment of a Ligand-dependent corepressor (LCOR) is characterized by consisting of or consisting essentially of amino acid sequence SEQ ID NO 7, or a sequence which is at least 90%, 91%, 92%, 93%, 94%, 95%, 96%, 97%, 98%, or 100% identical to SEQ ID NO 7.
6. The mutant or fragment of a Ligand-dependent corepressor (LCOR) according to clause 3, or a polynucleotide sequence coding for the said mutant or fragment of a Ligand-dependent corepressor (LCOR), wherein the mutant or fragment of a Ligand-dependent corepressor (LCOR) is characterized by further comprising a HDAC motif of amino acid sequence SEQ ID NO 9 or a sequence which is at least 90%, 91%, 92%, 93%, 94%, 95%, 96%, 97%, 98%, or 100% identical to SEQ ID NO 9.
7. The mutant or fragment of a Ligand-dependent corepressor (LCOR) according to clause 6, or a polynucleotide sequence coding for the said mutant or fragment of a Ligand-dependent corepressor (LCOR), wherein the mutant or fragment of a Ligand-dependent corepressor (LCOR) is characterized by comprising, consisting of, or consisting essentially of amino acid sequence SEQ ID NO 10, or a sequence which is at least 90%, 91%, 92%, 93%, 94%, 95%, 96%, 97%, 98%, or 100% identical to SEQ ID NO 10.
8. The mutant or fragment of a Ligand-dependent corepressor (LCOR) according to clause 6, or a polynucleotide sequence coding for the said mutant or fragment of a Ligand-dependent corepressor (LCOR), wherein the mutant or fragment of a Ligand-dependent corepressor (LCOR) is characterized by consisting of or consisting essentially of amino acid sequence SEQ ID NO 12, or a sequence which is at least 90%, 91%, 92%, 93%, 94%, 95%, 96%, 97%, 98%, or 100% identical to SEQ ID NO 12.
9. The mutant or fragment of a Ligand-dependent corepressor (LCOR) according to clause 1 or 6, or a polynucleotide sequence coding for the said mutant or fragment of a Ligand-dependent corepressor (LCOR), wherein the mutant or fragment of a Ligand-dependent corepressor (LCOR) is characterized by further comprising any one of a CTBP1 binding site and/or a CTBP2 binding site.
10. The mutant or fragment of a Ligand-dependent corepressor (LCOR) according to clause 1 or 6, or a polynucleotide sequence coding for the said mutant or fragment of a Ligand-dependent corepressor (LCOR), wherein the mutant or fragment of a Ligand-dependent corepressor (LCOR) is characterized by further comprising a CTBP1 binding site and/or a CTBP2 binding site, wherein the CTBP1 binding site is of amino acid sequence PLDLTVR (SEQ ID NO 14) or a sequence which is at least 90%, 91%, 92%, 93%, 94%, 95%, 96%, 97%, 98%, or 100% identical to SEQ ID NO 14, and wherein the CTBP2 binding site is of amino acid sequence VLDLSTK (SEQ ID NO 15) or a sequence which is at least 90%, 91%, 92%, 93%, 94%, 95%, 96%, 97%, 98%, or 100% identical to SEQ ID NO 15.
11. The mutant or fragment of a Ligand-dependent corepressor (LCOR) according to clause 10, or a polynucleotide sequence coding for the said mutant or fragment of a Ligand-dependent corepressor (LCOR), wherein the mutant or fragment of a Ligand-dependent corepressor (LCOR) is characterized by consisting of, or consisting essentially of amino acid sequence SEQ ID NO 16, or a sequence which is at least 90%, 91%, 92%, 93%, 94%, 95%, 96%, 97%, 98%, or 100% identical to SEQ ID NO 16.
12. The mutant or fragment of a Ligand-dependent corepressor (LCOR) according to clause 10, or a polynucleotide sequence coding for the said mutant or fragment of a Ligand-dependent corepressor (LCOR), wherein the mutant or fragment of a Ligand-dependent corepressor (LCOR) is characterized by consisting of, or consisting essentially of amino acid sequence SEQ ID NO 18, or a sequence which is at least 90%, 91%, 92%, 93%, 94%, 95%, 96%, 97%, 98%, or 100% identical to SEQ ID NO 18.
13. The mutant or fragment of a Ligand-dependent corepressor (LCOR) according to clause 11, or a polynucleotide sequence coding for the said mutant or fragment of a Ligand-dependent corepressor (LCOR), wherein the mutant or fragment of a Ligand-dependent corepressor (LCOR) consists of SEQ ID NO 16.
14. The mutant or fragment of a Ligand-dependent corepressor (LCOR) according to clause 11, or a polynucleotide sequence coding for the said mutant or fragment of a Ligand-dependent corepressor (LCOR), wherein the mutant or fragment of a Ligand-dependent corepressor (LCOR) consists of SEQ ID NO 18.
15. A pharmaceutical composition comprising an isolated or synthesized Ligand-dependent corepressor (LCOR) or a mutant or fragment thereof, or the polynucleotide sequence coding for the same, according to any one of clauses 1 to 14.
16. A pharmaceutical composition comprising: a) an isolated or synthesized Ligand-dependent corepressor (LCOR) or a mutant or fragment thereof, or the polynucleotide sequence coding for the same, according to any one of clauses 1 to 14; and b) at least one immune checkpoint inhibitor (ICI).
17. The pharmaceutical composition according to clause 16, wherein the at least one ICI inhibits CTLA-4, PD-1, PD-L1, Lag-3, Tim-3, TIGIT or a combination thereof, preferably inhibits PD-1 or PD-L1, more preferably inhibits PD-1; preferably wherein the ICI is selected between ipilimumab, tremelimumab, nivolumab, dostarlimab, pembrolizumab, pidilizumab, MEDI0680, atezolizumab, BMS-936559, MEDI4736 (durvalumab), MSB0010718C (avelumab), TSR-022 (cobolimab), BMS-986016, TSR-033, BMS-986207, and combinations thereof, preferably is selected between pembrolizumab, atezolizumab, dostarlimab and combinations thereof.
18. A pharmaceutical composition comprising: a) an isolated or synthesized Ligand-dependent corepressor (LCOR) or a mutant or fragment thereof, or the polynucleotide sequence coding for the same, according to any one of clauses 1 to 14; and b) at least one adoptive cell therapeutic composition.
19. The pharmaceutical composition according to clause 18, wherein the at least one adoptive cell therapeutic composition comprises a cell type selected from a group consisting of a tumor infiltrating lymphocyte (TIL), T-cell receptor modified lymphocytes and chimeric antigen receptor modified lymphocytes, preferably wherein the adoptive cell therapeutic composition comprises a cell type selected from a group consisting of engineered immune cells, T-cells, CD8+ cells, CD4+ cells, NK-cells, delta-gamma T- cells, regulatory T-cells, and peripheral blood mononuclear cells, preferably wherein the adoptive cell therapeutic composition comprises T-cells.
20. An isolated or synthesized Ligand-dependent corepressor (LCOR) or a mutant or fragment thereof, or the polynucleotide sequence coding for the same, according to any one of clauses 1 to 14, or the pharmaceutical composition according to anyone of clauses 15 to 19, for use as a medicament.
21. An isolated or synthesized Ligand-dependent corepressor (LCOR) or a mutant or fragment thereof, or the polynucleotide sequence coding for the same, according to any one of clauses 1 to 14, or the pharmaceutical composition according to anyone of clauses 15 to 19, for use in a method of treating cancer, preferably for cancer immunotherapy.
22. The isolated or synthesized Ligand-dependent corepressor (LCOR) or a mutant or fragment thereof, or the polynucleotide sequence coding for the same, for use according to the previous clause, wherein the cancer is characterized by comprising or consisting of cancer cells that are androgen, estrogen or progesterone receptor positive, preferably wherein the cancer is an estrogen receptor (ER) positive breast cancer.
23. The isolated or synthesized Ligand-dependent corepressor (LCOR) or a mutant or fragment thereof, or the polynucleotide sequence coding for the same, for use according to clause 21, wherein the cancer is s selected from chondrosarcoma, Ewing's sarcoma, malignant fibrous histiocytoma of bone/osteosarcoma, osteosarcoma, rhabdomyosarcoma, heart cancer, astrocytoma, brainstem glioma, pilocytic astrocytoma, ependymoma, primitive neuroectodermal tumor, cerebellar astrocytoma, cerebral astrocytoma, glioma, medulloblastoma, neuroblastoma, oligodendroglioma, pineal astrocytoma, pituitary adenoma, visual pathway and hypothalamic glioma, breast cancer, invasive lobular carcinoma, tubular carcinoma, invasive cribriform carcinoma, medullary carcinoma, male breast cancer, phyllodes tumor, inflammatory breast cancer, adrenocortical carcinoma, islet cell carcinoma (endocrine pancreas), multiple endocrine neoplasia syndrome, parathyroid cancer, pheochromocytoma, thyroid cancer, Merkel cell carcinoma, uveal melanoma, retinoblastoma, anal cancer, appendix cancer, cholangiocarcinoma, carcinoid tumor, colon cancer, extrahepatic bile duct cancer, gallbladder cancer, gastric (stomach) cancer, gastrointestinal carcinoid tumor, gastrointestinal stromal tumor, hepatocellular cancer, pancreatic cancer, islet cell, rectal cancer, bladder cancer, cervical cancer, endometrial cancer, extragonadal germ cell tumor, ovarian cancer, ovarian epithelial cancer (surface epithelial-stromal tumor), ovarian germ cell tumor, penile cancer, renal cell carcinoma, renal pelvis and ureter, transitional cell cancer, prostate cancer, testicular cancer, gestational trophoblastic tumor, urethral cancer, uterine sarcoma, vaginal cancer, vulvar cancer, Wilms tumor, esophageal cancer, head and neck cancer, nasopharyngeal carcinoma, oral cancer, oropharyngeal cancer, paranasal sinus and nasal cavity cancer, pharyngeal cancer, salivary gland cancer, hypopharyngeal cancer, basal cell carcinoma, squamous cell carcinoma, skin adnexal tumors (e.g. sebaceous carcinoma), melanoma, sarcomas of primary cutaneous origin (e.g. dermatofibrosarcoma protuberans), lymphomas of primary cutaneous origin (e.g. mycosis fungoides), bronchial adenomas/carcinoids, small cell lung cancer, mesothelioma, non-small cell lung cancer, pleuropulmonary blastoma, laryngeal cancer, thymoma and thymic carcinoma, Kaposi sarcoma, epithelioid hemangioendothelioma (EHE), desmoplastic small round cell tumor, liposarcoma, preferably the cancer is selected from lung cancer, melanoma, renal carcinoma, bladder carcinoma, prostate cancer, mesothelioma, glioblastoma and breast cancer, more preferably the cancer is selected from melanoma, bladder cancer and breast cancer, preferably triple-negative breast cancer.
24. A pharmaceutical composition according to anyone of clauses 15 to 19, for use in a method of treating cancer, preferably for cancer immunotherapy.
25. The pharmaceutical composition for use according to the previous clause, wherein the cancer is characterized by comprising or consisting of cancer cells that are androgen, estrogen or progesterone receptor positive, preferably wherein the cancer is an estrogen receptor (ER) positive breast cancer.
26. The pharmaceutical composition for use according to clause 24, wherein the cancer is selected from chondrosarcoma, Ewing's sarcoma, malignant fibrous histiocytoma of bone/osteosarcoma, osteosarcoma, rhabdomyosarcoma, heart cancer, astrocytoma, brainstem glioma, pilocytic astrocytoma, ependymoma, primitive neuroectodermal tumor, cerebellar astrocytoma, cerebral astrocytoma, glioma, medulloblastoma, neuroblastoma, oligodendroglioma, pineal astrocytoma, pituitary adenoma, visual pathway and hypothalamic glioma, breast cancer, invasive lobular carcinoma, tubular carcinoma, invasive cribriform carcinoma, medullary carcinoma, male breast cancer, phyllodes tumor, inflammatory breast cancer, adrenocortical carcinoma, islet cell carcinoma (endocrine pancreas), multiple endocrine neoplasia syndrome, parathyroid cancer, pheochromocytoma, thyroid cancer, Merkel cell carcinoma, uveal melanoma, retinoblastoma, anal cancer, appendix cancer, cholangiocarcinoma, carcinoid tumor, colon cancer, extrahepatic bile duct cancer, gallbladder cancer, gastric (stomach) cancer, gastrointestinal carcinoid tumor, gastrointestinal stromal tumor, hepatocellular cancer, pancreatic cancer, islet cell, rectal cancer, bladder cancer, cervical cancer, endometrial cancer, extragonadal germ cell tumor, ovarian cancer, ovarian epithelial cancer (surface epithelial-stromal tumor), ovarian germ cell tumor, penile cancer, renal cell carcinoma, renal pelvis and ureter, transitional cell cancer, prostate cancer, testicular cancer, gestational trophoblastic tumor, urethral cancer, uterine sarcoma, vaginal cancer, vulvar cancer, Wilms tumor, esophageal cancer, head and neck cancer, nasopharyngeal carcinoma, oral cancer, oropharyngeal cancer, paranasal sinus and nasal cavity cancer, pharyngeal cancer, salivary gland cancer, hypopharyngeal cancer, basal cell carcinoma, squamous cell carcinoma, skin adnexal tumors (e.g. sebaceous carcinoma), melanoma, sarcomas of primary cutaneous origin (e.g. dermatofibrosarcoma protuberans), lymphomas of primary cutaneous origin (e.g. mycosis fungoides), bronchial adenomas/carcinoids, small cell lung cancer, mesothelioma, non-small cell lung cancer, pleuropulmonary blastoma, laryngeal cancer, thymoma and thymic carcinoma, Kaposi sarcoma, epithelioid hemangioendothelioma (EHE), desmoplastic small round cell tumor, liposarcoma, preferably the cancer is selected from lung cancer, melanoma, renal carcinoma, bladder carcinoma, prostate cancer, mesothelioma, glioblastoma and breast cancer, more preferably the cancer is selected from melanoma, bladder cancer and breast cancer, preferably triple-negative breast cancer.
27. The composition for use according to any one of clauses 24 to 26, wherein the administration(s) of adoptive cell therapeutic composition or the ICI and the isolated or synthesized Ligand-dependent corepressor (LCOR) or a mutant or fragment thereof, or the polynucleotide sequence coding for the same, to a subject is(are) conducted simultaneously or consecutively, in any order.
28. The composition for use according to clause 27, wherein there is a time period of one minute to four weeks between the consecutive administration of adoptive cell therapeutic composition or ICI and the mutant or fragment thereof, or the polynucleotide sequence coding for the same, and/or there are several administrations of adoptive cell therapeutic composition or ICI and the mutant or fragment thereof, or the polynucleotide sequence coding for the same.
29. The composition for use according to clause 27, wherein the administration of the adoptive cell therapeutic composition and/or ICI and/or the mutant or fragment thereof, or the polynucleotide sequence coding for the same, is conducted through an intra-tumoral, intra-arterial, intravenous, intrapleural, intravesicular, intracavitary or peritoneal injection, or an oral administration.
30. An isolated or synthesized Ligand-dependent corepressor (LCOR) or a mutant or fragment thereof, or the polynucleotide sequence coding for the same, according to any one of clauses 1 to 14, or the pharmaceutical composition according to anyone of clauses 15 to 19, for use according to any one of clauses 21 to 23, wherein the Ligand-dependent corepressor (LCOR) is the polypeptide fragment of LCOR according to any one of clauses 1a) or claim 2, wherein the method or use does not involved the administration of the nucleic acid encoding the polypeptide of SEQ ID NO 23 or any functionally equivalent variant thereof, and wherein the method does not involved the administration of any carrier or vehicle or a chemical moiety that facilitates the cellular uptake of the polypeptide.
31. An pharmaceutical composition for use according to any one of clauses 24 to 29, wherein the Ligand-dependent corepressor (LCOR) is the polypeptide fragment of LCOR according to any one of clauses 1a) or claim 2, wherein the method or use does not involved the administration of the nucleic acid encoding the polypeptide of SEQ ID NO 23 or any functionally equivalent variant thereof, and wherein the method does not involved the administration of any carrier or vehicle or a chemical moiety that facilitates the cellular uptake of the polypeptide.
32. The composition for use according to any one of clauses 20 to 31, wherein the composition is for use in a human subject in need thereof.

## Claims

1. An isolated or synthesized Ligand-dependent corepressor (LCOR) or a mutant or fragment thereof, selected from any one of the list consisting of:
a. a fragment of the Ligand-dependent corepressor (LCOR) comprising, consisting of or consisting essentially of SEQ ID NO 23 (MPRKKRGRYRQYNSEILEEAISVVMSGKMSVSKAQSIYGIPHSTLEYKVKERLGTLKNP PKKKMKLMRSEGPDVSVKIELDPQGEAAQSANESKNE) or a sequence which is at least 90%, 91%, 92%, 93%, 94%, 95%, 96%, 97%, 98%, or 100% identical to SEQ ID NO 23;
b. a mutant or fragment of a Ligand-dependent corepressor (LCOR) or a polynucleotide sequence coding for the said mutant or fragment of a Ligand-dependent corepressor (LCOR), wherein the mutant or fragment of a Ligand-dependent corepressor (LCOR) is **characterized by** comprising amino acid sequence SEQ ID NO 3, or a sequence which is at least 90%, 91%, 92%, 93%, 94%, 95%, 96%, 97%, 98%, or 100% identical to SEQ ID NO 3, and at least one NLS motif (nuclear localization signal) of amino acid sequence SEQ ID NO 4 or a sequence which is at least 90%, 91%, 92%, 93%, 94%, 95%, 96%, 97%, 98%, or 100% identical to SEQ ID NO 4; and wherein the mutant or fragment of a Ligand-dependent corepressor (LCOR) is further **characterized by** the absence or disruption/inactivation of the Nuclear Receptor Binding Domain; and
c. a Ligand-dependent corepressor (LCOR) or a polynucleotide sequence coding for the said Ligand-dependent corepressor (LCOR), wherein the LCOR is of SEQ ID NO 1 or a sequence which is at least 90%, 91%, 92%, 93%, 94%, 95%, 96%, 97%, 98%, or 100% identical to SEQ ID NO 1.

2. The isolated or synthesized Ligand-dependent corepressor (LCOR) or a mutant or fragment thereof according to claim 1, wherein said LCOR is the fragment of a Ligand-dependent corepressor (LCOR) consisting essentially of or consisting of SEQ ID NO 23 (MPRKKRGRYRQYNSEILEEAISVVMSGKMSVSKAQSIYGIPHSTLEYKVKERLGTLKNPPKKKM KLMRSEGPDVSVKIELDPQGEAAQSANESKNEC) or a sequence which is at least 90%, 91%, 92%, 93%, 94%, 95%, 96%, 97%, 98%, or 100% identical to SEQ ID NO 23, wherein preferably said LCOR mutant or fragment is a recombinant protein.

3. The LCOR fragment according to claim 2, wherein said LCOR is the fragment of a Ligand-dependent corepressor (LCOR) consisting essentially of or consisting of SEQ ID NO 23

(MPRKKRGRYRQYNSEILEEAISVVMSGKMSVSKAQSIYGIPHSTLEYKVKERLGTLKNPPKKKM
KLMRSEGPDVSVKIELDPQGEAAQSANESKNEC).

4. A pharmaceutical composition comprising the LCOR fragment according to any one of claims 2 to 3.

5. An LCOR fragment according to any one of claims 2 to 3, or the pharmaceutical composition according to claim 4, for use in therapy.

6. An LCOR fragment according to any one of claims 2 to 3, or the pharmaceutical composition according to claim 4, for use in a method of treating cancer, preferably for cancer immunotherapy.

7. The LCOR fragment for use according to the previous claim, wherein the cancer is **characterized by** comprising or consisting of cancer cells that are androgen, estrogen or progesterone receptor positive, preferably wherein the cancer is an estrogen receptor (ER) positive breast cancer.

8. The LCOR fragment for use according to claim 6, wherein the cancer is selected from chondrosarcoma, Ewing's sarcoma, malignant fibrous histiocytoma of bone/osteosarcoma, osteosarcoma, rhabdomyosarcoma, heart cancer, astrocytoma, brainstem glioma, pilocytic astrocytoma, ependymoma, primitive neuroectodermal tumor, cerebellar astrocytoma, cerebral astrocytoma, glioma, medulloblastoma, neuroblastoma, oligodendroglioma, pineal astrocytoma, pituitary adenoma, visual pathway and hypothalamic glioma, breast cancer, invasive lobular carcinoma, tubular carcinoma, invasive cribriform carcinoma, medullary carcinoma, male breast cancer, phyllodes tumor, inflammatory breast cancer, adrenocortical carcinoma, islet cell carcinoma (endocrine pancreas), multiple endocrine neoplasia syndrome, parathyroid cancer, pheochromocytoma, thyroid cancer, Merkel cell carcinoma, uveal melanoma, retinoblastoma, anal cancer, appendix cancer, cholangiocarcinoma, carcinoid tumor, colon cancer, extrahepatic bile duct cancer, gallbladder cancer, gastric (stomach) cancer, gastrointestinal carcinoid tumor, gastrointestinal stromal tumor, hepatocellular cancer, pancreatic cancer, islet cell, rectal cancer, bladder cancer, cervical cancer, endometrial cancer, extragonadal germ cell tumor, ovarian cancer, ovarian epithelial cancer (surface epithelial-stromal tumor), ovarian germ cell tumor, penile cancer, renal cell carcinoma, renal pelvis and ureter, transitional cell cancer, prostate cancer, testicular cancer, gestational trophoblastic tumor, urethral cancer, uterine sarcoma, vaginal cancer, vulvar cancer, Wilms tumor, esophageal cancer, head and neck cancer, nasopharyngeal carcinoma, oral cancer, oropharyngeal cancer, paranasal sinus and nasal cavity cancer, pharyngeal cancer, salivary gland cancer, hypopharyngeal cancer, basal cell carcinoma, squamous cell carcinoma, skin adnexal tumors (e.g. sebaceous carcinoma), melanoma, sarcomas of primary cutaneous origin (e.g. dermatofibrosarcoma protuberans), lymphomas of primary cutaneous origin (e.g. mycosis fungoides), bronchial adenomas/carcinoids, small cell lung cancer, mesothelioma, non-small cell lung cancer, pleuropulmonary blastoma, laryngeal cancer, thymoma and thymic carcinoma, Kaposi sarcoma, epithelioid hemangioendothelioma (EHE), desmoplastic small round cell tumor, liposarcoma, preferably the cancer is selected from lung cancer, melanoma, renal carcinoma, bladder carcinoma, prostate cancer, mesothelioma, glioblastoma and breast cancer, more preferably the cancer is selected from melanoma, bladder cancer and breast cancer, preferably triple-negative breast cancer.

9. The LCOR fragment for use according to any one of claims 6 to 8, wherein the method of treatment does not involved the administration of the nucleic acid encoding the polypeptide of SEQ ID NO 23 or any functionally equivalent variant thereof, and wherein the method does not involved the administration of any carrier or vehicle or a chemical moiety that facilitates the cellular uptake of the polypeptide.

10. A pharmaceutical composition comprising: a) an isolated or synthesized Ligand-dependent corepressor (LCOR) or a mutant or fragment thereof, or the polynucleotide sequence coding for the same, according to any one of claims 1 to 3; and b) at least one immune checkpoint inhibitor (ICI).

11. The pharmaceutical composition according to claim 10, wherein the at least one ICI inhibits CTLA-4, PD-1, PD-L1, Lag-3, Tim-3, TIGIT or a combination thereof, preferably inhibits PD-1 or PD-L1, more preferably inhibits PD-1; preferably wherein the ICI is selected between ipilimumab, tremelimumab, nivolumab, dostarlimab, pembrolizumab, pidilizumab, MEDI0680, atezolizumab, BMS-936559, MEDI4736 (durvalumab), MSB0010718C (avelumab), TSR-022 (cobolimab), BMS-986016, TSR-033, BMS-986207, and combinations thereof, preferably is selected between pembrolizumab, atezolizumab, dostarlimab and combinations thereof.

12. A pharmaceutical composition comprising: a) an isolated or synthesized Ligand-dependent corepressor (LCOR) or a mutant or fragment thereof, or the polynucleotide sequence coding for the same, according to any one of claims 1 to 3; and b) at least one adoptive cell therapeutic composition.

13. The pharmaceutical composition according to claim 13, wherein the at least one adoptive cell therapeutic composition comprises a cell type selected from a group consisting of a tumor infiltrating lymphocyte (TIL), T-cell receptor modified lymphocytes and chimeric antigen receptor modified lymphocytes, preferably wherein the adoptive cell therapeutic composition comprises a cell type selected from a group consisting of engineered immune cells, T-cells, CD8+ cells, CD4+ cells, NK-cells, delta-gamma T- cells, regulatory T-cells, and peripheral blood mononuclear cells, preferably wherein the adoptive cell therapeutic composition comprises T-cells.

14. The pharmaceutical composition according to any one of claims 10 to 13, wherein said composition is for use in a method of treating cancer, preferably for cancer immunotherapy, as defined in any one of claims 6 to 8, and wherein the administration(s) of the adoptive cell therapeutic composition or the ICI and the isolated or synthesized Ligand-dependent corepressor (LCOR) or a mutant or fragment thereof, or the polynucleotide sequence coding for the same, to a subject is(are) conducted simultaneously or consecutively, in any order.

15. The composition for use according to claim 14, wherein there is a time period of one minute to four weeks between the consecutive administration of adoptive cell therapeutic composition or ICI and the mutant or fragment thereof, or the polynucleotide sequence coding for the same, and/or there are several administrations of adoptive cell therapeutic composition or ICI and the mutant or fragment thereof, or the polynucleotide sequence coding for the same.
